# EUROPÄISCHE PATENTSCHRIFT

(11) **EP 1 905 415 B1**
(45) Veröffentlichungstag und Bekanntmachung des Hinweises auf die Patenterteilung: **01.07.2009**
(21) Anmeldenummer: 07112299.8
(22) Anmeldetag: 11.07.2007
(51) Int. Cl.: A61K 6/083, C08F 4/72

(54) **Polymerisierbare Zusammensetzungen mit Acylgermanium-Verbindungen als Initiatoren**
Polymerisable compositions comprising acyl-germanium as initiators
Compositions polymérisable avec acyl-germanium comme amorceurs

(30) Priorität: 27.09.2006 EP 06121333
(43) Veröffentlichungstag der Anmeldung: 02.04.2008
(73) Patentinhaber: Ivoclar Vivadent AG, 9494 Schaan (LI)
(72) Erfinder: Moszner, Norbert, Prof. Dr., 9495, Triesen (LI); Zeuner, Frank, Dr., 9488, Schellenberg (LI); Liska, Robert, Prof. Dr., 1200, Wien (AT); Fischer, Urs-Karl, 9320, Arbon (CH); Burtscher, Peter, Dr., A-6830, Rankweil (AT); Salz, Ulrich, Dr., 88131, Lindau (DE); Rheinberger, Volker, Dr., 9490, Vaduz (LI); Gruber, Prof. Dr. Heinrich, AT-Wien 1190 (AT); Ganster, Beate, AT-Wien 1190 (AT)
(74) Vertreter: UEXKÜLL & STOLBERG

(56) Entgegenhaltungen:
- EP-A1- 1 413 569
- EP-A2- 0 405 786
- US-A- 4 457 818

## Beschreibung

Die vorliegende Erfindung betrifft polymerisierbare Zusammensetzungen, die eine Acylgermanium-Verbindung als Polymerisationsinitiator enthalten. Die Zusammensetzungen eignen sich besonders zur Herstellung von Adhäsiven, Beschichtungen, Zementen, Kompositen, Formteilen und insbesondere von Dentalwerkstoffen.

Für die Aushärtung von polymerisationsfähigen Harzen spielt der eingesetzte Initiator eine entscheidende Rolle. Photoinitiatoren absorbieren bei Bestrahlung UV- oder sichtbares Licht und bilden die polymerisationsauslösenden Spezies. Im Fall der radikalischen Polymerisation handelt es sich dabei um freie Radikale. Basierend auf dem chemischen Mechanismus der Radikalbildung werden die Photoinitiatoren in zwei Klassen eingeteilt.

Norrish-Typ-I-Photoinitiatoren bilden beim Bestrahlen durch eine unimolekulare Bindungsspaltung freie Radikale. Norrish-Typ-II-Photoinitiatoren durchlaufen bei der Bestrahlung eine bimolekulare Reaktion wobei der Photoinitiator im angeregten Zustand mit einem zweiten Molekül, dem Coinitiator, reagiert und durch Elektronen- und Protonentransfer oder direkte Wasserstoffabstraktion die polymerisationsauslösenden Radikale bildet. Für die UV-Lichthärtung werden Typ-I- und Typ-II-Photoinitiatoren eingesetzt, für den sichtbaren Lichtbereich kommen bisher nahezu ausschliesslich Typ-II-Photoinitiatoren zum Einsatz.

Die UV-Härtung zeichnet sich durch eine hohe Reaktionsgeschwindigkeit aus und wird häufig für die Beschichtungen von unterschiedlichen Substraten wie z.B. Holz, Metall oder Glas eingesetzt. So wird zum Beispiel in EP 1 247 843 ein UV-härtendes Beschichtungsmaterial beschrieben, bei dem Typ-I-Photoinitiatoren wie Diethoxyphenylacetophenon oder Acylphosphinoxide zum Einsatz kommen.

WO 01/51533 beschreibt eine UV-härtendes Holzbeschichtungsmaterial bei dem ebenfalls Acylphosphinoxide, α-Hydroxyalkylphenone oder α-Dialkoxyacetophenone als Photoinitiator Anwendung finden. Bedingt durch die geringe Wellenlänge des UV-Lichts lassen sich mit der UV-Härtung vor allem transparente Beschichtungen mit geringer Schichtstärke härten, bei starker Einfärbung oder Pigmentierung und größeren Schichtstärken stößt man jedoch an das Limit der UV-Härtung, derartige photopolyreaktionsfähigen Harze härten mit UV-Licht nur unvollständig aus. Bei pigmentierten Zusammensetzungen muß außerdem ein Absorptionsbereich für den Photoinitiator gefunden werden, in dem das Pigment nur schwach absorbiert.

Werden größere Durchhärtungstiefen benötigt, wie zum Beispiel bei der Aushärtung von lichthärtenden Dentalfüllungsmaterialien, so wird in der Regel mit sichtbarem Licht bestrahlt. Das dafür am häufigsten eingesetzte Photoinitiatorsystem ist eine Kombination aus einem α-Diketon mit einem Amincoinitiator wie sie in GB 1 408 265 beschrieben ist.

Dentalzusammensetzungen, in denen dieses Photoinitiatorsystem eingesetzt wird, werden z.B. in der US 4,457,818 oder US 4,525,256 offenbart, wobei vorzugsweise Campherchinon als α-Diketon eingesetzt wird. Campherchinon hat ein Absorptionsmaximum bei einer Wellenlänge von 468 nm. Dadurch zeigt Campherchinon eine starke Gelbfärbung, mit dem Nachteil, daß mit Campherchinon/Amin initiierte Materialien nach der Aushärtung einen deutlichen Gelbstich aufweisen. Dies ist vor allem bei hellen Weißtönen des auspolymerisierten Materials sehr nachteilig.

Ein weiterer Nachteil von Typ-II-Photoinitiatoren ist, daß sie bei der Polymerisation zur Bildung einer klebrigen Oberflächenschicht führen. Diese sogenannte Inhibierungschicht ist auf die Inhibierung der radikalischen Polymerisation durch den Sauerstoff der Luft zurückzuführen.

Die EP 0 405 786 A2 offenbart Initiatoren auf der Basis von Silicium, Germanium oder Zinn, die sich für die Massepolymerisation von Acrylmonomeren in einem Extruder eignen sollen. Die Initiatoren werden zusammen mit CoKatalysatoren wie Tetrabutylammoniumfluorid eingesetzt. Initiatoren auf der Basis von Silicium sind bevorzugt wie beispielsweise 9-Trimethylsilylcarbazol.

Der Erfindung liegt die Aufgabe zugrunde, Polymerisationsinitiatoren zur Verfügung zu stellen, die mit sichtbarem Licht aktiviert werden können, und die eine hohe Durchhärtungstiefe des zu härtenden Materials ergeben. Die Initiatoren sollen in geringer Konzentration wirksam sein und eine schnelle Aushärtung des zu härtenden Materials ermöglichen. Außerdem sollen sie nicht zu Verfärbungen des Materials führen.

Erfindungsgemäß wird diese Aufgabe durch Zusammensetzungen mit mindestens einem polymerisierbaren Bindemittel und mindestens einem Polymerisationsinitiator gelöst, die mindestens eine Acylgermanium-Verbindung gemäß der allgemeinen Formel (I) enthalten, 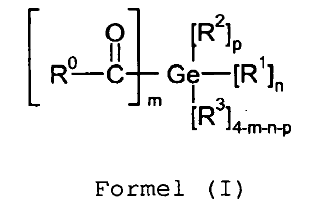 in der
- R⁰: C₁₋₁₈-Alkyl, C₂₋₁₈-Alkenyl, wobei diese Reste unsubstituiert oder einfach oder mehrfach durch Halogen substituiert sein können, -OR¹⁰, -OCO-R¹⁰, -OCO-Hal, -COO-R¹⁰, -CH=CH-CO-OR¹⁰, -N(R¹¹)-CO-R¹⁰, -N(R¹¹)-CO-Hal, -C(C₁₋₄-Alkyl)=C(C₁₋₄-Alkyl)-CO-OR¹⁰, -CO-NR¹¹R¹², -CH=CH-Phenyl, -C (C₁₋₄-Alkyl) =C (C₁₋₄-Alkyl)-Phenyl, C₃₋₁₂-Cycloalkyl, C₂₋₁₈-Alkenyl, Phenyl-C₁₋₄-Alkyl, Phenyl, Naphthyl, Antryl, Biphenyl, ein gesättigter oder ungesättigter 5- oder 6-gliedriger O-, S- oder N-haltiger heterocyclischer Ring, wobei alle genannten Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder 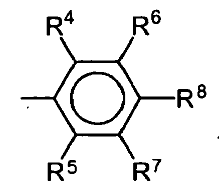 ist, wobei
R¹⁰ H, C₁₋₁₈-Alkyl, C₁₋₁₈-Alkyl, das durch ein oder mehrere Sauerstoffatome unterbrochen ist, C₂₋₁₈-Alkenyl, C₂₋₁₈-Alkenyl, das durch ein oder mehrere Sauerstoffatome unterbrochen ist, C₃₋₁₂-Cycloalkyl, Tetrahydropyran-2-yl, Phenyl-C₁₋₂₀-Alkylen, Phenyl-C₁₋₂₀-Alkenylen, C₁₋₆-Alkyl, das unsubstituiert oder durch Halogen, Cyclohexyl, Cyclopentyl, Tetrahydrofuranyl, Furanyl oder Isopropyl-4-methyl-cyclohexyl substituiert sein kann, Phenyl, Naphthyl oder Biphenyl ist, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können,
R¹¹, R¹² unabhängig voneinander H, C₁₋₁₈-Alkyl, C₁₋₁₈-Alkyl, das durch ein oder mehrere Sauerstoffatome unterbrochen ist, C₂₋₁₈-Alkenyl, C₂₋₁₈-Alkenyl, das durch ein oder mehrere Sauerstoffatome unterbrochen ist, C₃₋₁₂-Cycloalkyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder R¹¹ und R¹² bilden zusammen einen 5- oder 6-gliedrigen O-, S- oder N-haltigen heterocyclischen Ring, der seinerseits mit einem aliphatischen oder aromatischen Ring anelliert sein kann,
- R¹, R²: unabhängig voneinander 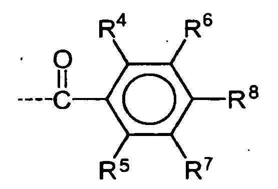 oder H
sind, oder eine der für R³ angegebenen Bedeutungen haben; wobei
R⁴, R⁵ unabhängig voneinander jeweils H, Halogen, ein verzweigter oder linearer C₁₋₆-Alkyl- oder -O-C₁₋₆-Alkylrest sind, der durch ein oder mehrere Sauerstoffatome unterbrochen sein kann;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy-Rest sind, der durch ein oder mehrere O, S oder -NR²⁰- unterbrochen und durch eine oder mehrere polymerisationsfähige Gruppen und/oder Reste R⁹ substituiert sein kann, wobei R²⁰ H, Halogen, ein verzweigter, cyclischer oder vorzugsweise linearer C₁₋₂₀-Alkyl-, -Alkenyl-, -Alkyloxy- oder -Alkenoxy-Rest ist;
- R³: ein verzweigter oder vorzugsweise linearer C₁₋₁₈-Alkyl-Rest oder C₂₋₁₈-Alkenyl-Rest ist, wobei diese Reste unsubstituiert oder einfach oder mehrfach durch einen Rest substituiert sein können, der aus der folgenden Gruppe ausgewählt ist:
Halogen, CN, 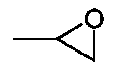 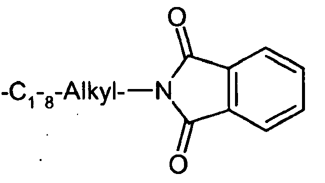 -OR¹⁰, -SR¹⁰, -OCO-R¹⁰, -COO-R¹⁰, -CH=CH-CO-OR¹⁰, -C(C₁₋₄-Alkyl)=C(C₁₋₄-Alkyl)-CO-OR¹⁰, -CO-R¹³, -CO-CH=CH-CO-C₁₋₆-Alkyl, -CO-CH=CH-CO-Phenyl, -CO-CH=CH-COO-C₁₋₁₈-Alkyl, -NR¹¹R¹², -N(R¹¹)-CO-R¹⁰, -N(R¹¹)-COO-R¹⁰, -N(R¹¹)-CO-NR¹¹R¹²,-N(R¹¹)-CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, -CH=CH-Phenyl, -C(C₁₋₄-Alkyl)=C(C₁₋₄-Alkyl)-Phenyl, Phenyl-C₁₋₄-Alkyl, Phenyl, Naphtyl, Biphenyl, C₅₋₁₂-Cycloalkyl, ein gesättigter oder ungesättigter 5- oder 6-gliedriger O-, S- oder N-haltiger heterocyclischer Ring, Benzophenonyl, Thisanthonyl, wobei
R¹³ C₁₋₁₈-Alkyl, C₂₋₁₈-Alkenyl, das durch ein oder mehrere O-Atome unterbrochen ist, C₃₋₁₂-Cycloalkyl, Phenyl-C₁₋₄-Alkyl, Phenyl, Naphtyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈-Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei
R¹⁷, R¹⁸, R¹⁹ unabhängig voneinander jeweils H, C₁₋₈-Alkyl, C₂₋₈-Alkenyl, C₇₋₉-Phenylalkyl, -O-C₁₋₈-Alkyl oder Phenyl sind, und wobei R¹⁰, R¹¹ und R¹² wie oben definiert sind;
oder
- R³: ist ein verzweigter oder vorzugsweise linearer C₂₋₁₈-Alkyl-Rest oder ein C₂₋₁₈-Alkylen-Rest, der einfach oder mehrfach durch -O-, -NH-, -NR¹¹-, -S- unterbrochen ist, wobei die Reste unsubstituiert oder einfach oder mehrfach durch einen Rest substituiert sein können, der aus der folgenden Gruppe gewählt ist:
Halogen, CN, -OR¹⁰, -SR¹⁰, -OCO-R¹⁰, -COO-R¹⁰, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹²,-PO(OC₁₋₈-Alkyl)₂, -SiR¹⁴R¹⁵R¹⁶, Phenyl-C₁₋₄-Alkyl, Phenyl, C₅₋₁₂-Cycloalkyl;
wobei R¹⁰, R¹¹, R¹², R¹⁴, R¹⁵ und R¹⁶ wie oben definiert sind;
oder
- R³: ist ein verzweigter oder vorzugsweise linearer C₂₋₁₈-Alkyl-Rest oder ein C₂₋₁₈-Alkylen-Rest, der einfach oder mehrfach durch -CO-, -COO-, -OCO-, -OCOO-, -CO-N(R¹¹)-, -N(R¹¹)-CO-, -N(R¹¹)-CO-N(R¹¹)-, -N(R¹¹)-COO-, -COO-C₁₋₆-Alkylen, -COS-C₁₋₁₈-Alkylen, -SO₂-, -SO₂-O-, -SO₂-N(R¹¹)-, -(CH₃)₂Si[OSi(CH₃)₂]_{q}-, mit q = 1 bis 6; Phenyl-C₁₋₄-Alkylen, Phenylen, Naphthylen, Biphenylen, C₅₋₁₂-Cycloakylen oder einen 5- oder 6-gliedrigen O-, S- oder N-haltigen heterocyclischen Ring unterbrochen ist;
wobei R¹¹ wie oben definiert ist;
oder
- R³: ist Trimethylsilyl, Hal-(CH₃)₂Si-[OSi(CH₃)₂]ᵣ-, (CH₃)₃Si-[OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
- R³: ist Phenyl-C₁₋₂₀-Alkyl, Phenyl, Naphthyl oder Biphenyl, C₅₋₁₂-Cycloalkyl oder ein gesättigter oder ungesättigter 5-oder 6-gliedriger O-, S- oder N-haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈-Alkylthioreste und/oder -NR¹¹R¹² substituiert sein können,
wobei R¹¹ und R¹² wie oben definiert sind und
- m: 1, 2 oder 3 ist,
- n: 0 oder 1 ist,
- p: 0 oder 1 ist;
oder vorzugsweise
- R³: ist Halogen, OH, ein aromatischer C₆₋₃₀-Rest, der durch einen verzweigten, cyclischen oder vorzugsweise linearen C₁₋₂₀-Alkyl-, -Alkenyl-, -Alkoxy- oder -Alkenoxy-Rest substituiert sein kann, wobei die genannten Reste durch ein oder mehrere O, S oder N-Atome unterbrochen und/oder durch eine oder mehrere polymerisationsfähige Gruppen und/oder Reste R⁹ substituiert sein können, oder ein verzweigter, cyclischer oder vorzugsweise linearer C₁₋₂₀-Alkyl-, -Alkenyl-, -Alkoxy- oder -Alkenoxy-Rest ist, der ein- oder mehrfach durch O, S oder -NR²⁰- unterbrochen und durch eine oder mehrere polymerisationsfähige Gruppen und/oder Reste R⁹ substituiert sein kann, wobei
R⁹ -OH, -CₓF₂ₓ₊₁ mit x = 1 bis 20, -[Si(CH₃)₂]_{y}-CH₃ mit y = 1 bis 20 ist, und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist.

Darüber hinaus können jeweils zwei der Reste R⁰, R¹, R² oder R³ unter Ausbildung eines 5- bis 8-gliedrigen Rings, der seinerseits mit einem oder mehreren, vorzugsweise 1 oder 2 aliphatischen oder aromatischen Ringen anelliert sein kann, miteinander verbunden sein. Diese Ringe können neben dem Germaniumatom weitere Heteroatome enthalten, vorzugsweise O-, S- oder N-Atome. Die Anzahl zusätzlicher Hereroatome beträgt vorzugsweise 1 oder 2. Es können unterschiedliche Reste oder im Fall von m, n, p oder 4-m-n-p > 1 auch gleiche Reste unter Ausbildung eines oder mehrerer Ringe miteinander verbunden sein. Beispielsweise können im Fall p = 2 die beiden Reste R² miteinander verbunden sein. Durch die Verbindung zwischen den Resten werden cyclische Germanium-Verbindungen gebildet, d.h. Verbindungen, bei denen das Germaniumatom in einen Ring integriert ist. Sind zwei mal zwei Gruppen miteinander verknüpft, so handelt es sich um Spiroverbindungen mit Germanium als Zentralatom (Spiroatom). Die gebildeten Ringe können unsubstituiert oder ein- oder mehrfach, vorzugsweise ein- oder zweifach substituiert sein. Bevorzugte Substituenten sind C₁₋₄-Alkylgruppen oder =O

Vorzugsweise haben die Variablen die folgenden Bedeutungen:
- R⁰: C₁₋₁₈-Alkyl, C₂₋₁₈-Alkenyl, wobei diese Reste unsubstituiert oder einfach oder mehrfach durch Halogen substituiert sein können, -OR¹⁰, -OCO-R¹⁰, -OCO-Hal, -COO-R¹⁰, -CH=CH-CO-OR¹⁰, -N(R¹¹)-CO-R¹⁰, -N(R¹¹)-CO-Hal, -C(C₁₋₄-Alkyl)=C(C₁₋₄-Alkyl)-CO-OR¹⁰, -CO-NR¹¹R¹², -CH=CH-Phenyl, -C (C₁₋₄-Alkyl) =C (C₁₋₄-Alkyl)-Phenyl, C₃₋₁₂-Cycloalkyl, C₂₋₁₈-Alkenyl, Phenyl-C₁₋₄-Alkyl, Phenyl, Naphthyl, Antryl, Biphenyl, ein 5- oder 6-gliedriger O-, S- oder N-haltiger heterocyclischer Ring, wobei alle genannten Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder 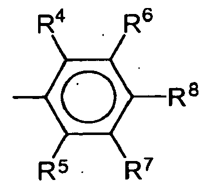 ist, wobei
- R¹⁰: H, C₁₋₁₈-Alkyl, C₂₋₁₈-Alkenyl, C₂₋₁₈-Alkenyl, das durch ein oder mehrere Sauerstoffatome unterbrochen ist, C₃₋₁₂-Cycloalkyl, Tetrahydropyran-2-yl, Phenyl-C₁₋₄-Alkylen, Phenyl-C₁₋₄-Alkenylen, C₁₋₆-Alkyl, das unsubstituiert oder durch Halogen, Cyclohexyl, Cyclopentyl, Tetrahydrofuranyl, Furanyl oder Isopropyl-4-methyl-cyclohexyl substituiert sein kann, Phenyl, Naphthyl oder Biphenyl ist, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können,
- R¹¹, R¹²: unabhängig voneinander H, C₁₋₁₈-Alkyl, C₂₋₁₈-Alkenyl, C₂₋₁₈-Alkenyl, das durch ein oder mehrere Sauerstoffatome unterbrochen ist, C₃₋₁₂-Cycloalkyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder R¹¹ und R¹² bilden zusammen einen 5- oder 6-gliedrigen O-, S- oder N-haltigen heterocyclischen Ring, der seinerseits mit einem aliphatischen oder aromatischen Ring anelliert sein kann,
- R¹, R²: unabhängig voneinander 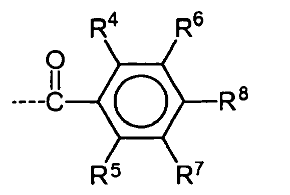 oder H sind, oder eine der für R³ angegebenen Bedeutungen haben; wobei
R⁴, R⁵ unabhängig voneinander jeweils H, Halogen, ein verzweigter oder linearer C₁₋₆-Alkyl- oder -O-C₁₋₆-Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy-Rest sind, der durch ein oder mehrere O, S oder -NR'- unterbrochen und durch eine oder mehrere polymerisationsfähige Gruppen und/oder Reste R⁹ substituiert sein kann, wobei R' H, Halogen, ein verzweigter, cyclischer oder vorzugsweise linearer C₁₋₂₀-Alkyl-, -Alkenyl-, -Alkyloxy- oder -Alkenoxy-Rest ist;
- R³: ein verzweigter oder vorzugsweise linearer C₁₋₁₈-Alkyl-Rest oder C₂₋₁₈-Alkenyl-Rest ist, wobei diese Reste unsubstituiert oder einfach oder mehrfach durch einen Rest substituiert sein können, der aus der folgenden Gruppe ausgewählt ist:
Halogen, CN, 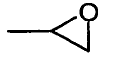 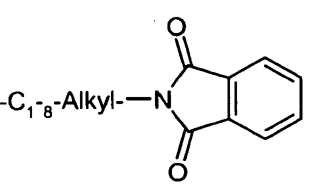 -OR¹⁰, -SR¹⁰, -OCO-R¹⁰, -COO-R¹⁰, -CH=CH-CO-OR¹⁰, -C(C₁₋₄-Alkyl)=C(C₁₋₄-Alkyl)-CO-OR¹⁰, -CO-R¹³, -CO-CH=CH-CO-C₁₋₆-Alkyl, -CO-CH=CH-CO-Phenyl, -CO-CH=CH-COO-C₁₋₁₈-Alkyl, -NR¹¹R¹², -N(R¹¹)-CO-R¹⁰, -N(R¹¹)-COO-R¹⁰, -N(R¹¹)-CO-NR¹¹R¹²,-N(R¹¹)-CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, -CH=CH-Phenyl, -C(C₁₋₄-Alkyl)=C(C₁₋₄-Alkyl)-Phenyl, Phenyl-C₁₋₄-Alkyl, Phenyl, Naphtyl, Biphenyl, C₅₋₁₂-Cycloalkyl, ein 5- oder 6-gliedriger O-, S- oder N-haltiger heterocyclischer Ring, Benzophenonyl, Thisanthonyl, wobei
R¹³ C₁₋₁₈-Alkyl, C₂₋₁₈-Alkenyl, das durch ein oder mehrere O-Atome unterbrochen ist, C₃₋₁₂-Cycloalkyl, Phenyl-C₁₋₄-Alkyl, Phenyl, Naphtyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈-Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei
R¹⁷, R¹⁸, R¹⁹ unabhängig voneinander jeweils H, C₁₋₈-Alkyl, C₂₋₈-Alkenyl, C₇₋₉-Phenylalkyl, -O-C₁₋₈-Alkyl oder Phenyl sind, und wobei R¹⁰, R¹¹ und R¹² wie oben definiert sind;
oder
- R³: ist ein verzweigter oder vorzugsweise linearer C₂₋₁₈-Alkyl-Rest oder ein C₂₋₁₈-Alkylen-Rest, der einfach oder mehrfach durch -O-, -NH-, -NR¹¹-, -S- unterbrochen ist, wobei die Reste unsubstituiert oder einfach oder mehrfach durch einen Rest substituiert sein können, der aus der folgenden Gruppe gewählt ist:
Halogen, CN, -OR¹⁰, -SR¹⁰, -OCO-R¹⁰, -COO-R¹⁰, -NR¹¹R¹², -N(R¹¹)-CO-R¹⁰, -N(R¹¹)-COO-R¹⁰, -N(R¹¹)-CO-NR¹¹R¹², -N(R¹¹)- CO-Hal, -CO-NR¹¹R¹², -SO₂-R¹⁰, -SO₂-OR¹⁰, -SO₂-NR¹¹R¹²,-PO(OC₁₋₈-Alkyl)₂, -SiR¹⁴R¹⁵R¹⁶, Phenyl-C₁₋₄-Alkyl, Phenyl, C₅₋₁₂-Cycloalkyl;
wobei R¹⁰, R¹¹, R¹², R¹⁴, R¹⁵ und R¹⁶ wie oben definiert sind;
oder
- R³: ist ein verzweigter oder vorzugsweise linearer C₂₋₁₈-Alkyl-Rest oder ein C₂₋₁₈-Alkylen-Rest, der einfach oder mehrfach durch -CO-, -COO-, -OCO-, -OCOO-, -CO-N(R¹²)-, -N(R¹²)-CO-, -N(R¹²)-CO-N(R¹²)-, -N(R¹²)-COO-, -COO-C₁₋₆-Alkylen, -COS-C₁₋₁₈-Alkylen, -SO₂-, -SO₂-O-, -SO₂-N(R¹²)-, -(CH₃)₂Si[OSi(CH₃)₂]_{q}-, mit q = 1 bis 6; Phenyl-C₁₋₄-Alkylen, Phenylen, Naphthylen, Biphenylen, C₅₋₁₂-Cycloalkylen oder einen 5- oder 6-gliedrigen O-, S- oder N-haltigen heterocyclischen Ring unterbrochen ist;
wobei R¹² wie oben definiert ist;
oder
- R³: ist Trimethylsilyl, Hal- (CH₃)₂Si-[OSi(CH₃)₂]ᵣ-, (CH₃)₃Si-[OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
- R³: ist Phenyl-C₁₋₄-Alkyl, Phenyl, Naphthyl oder Biphenyl, C₅₋₁₂-Cycloalkyl oder ein 5- oder 6-gliedriger O-, S- oder N-haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈-Alkylthioreste und/oder -NR¹¹R¹² substituiert sein können,
wobei R¹¹ und R¹² wie oben definiert sind und
- m: 1, 2 oder 3 ist,
- n: 0 oder 1 ist,
- p: 0 oder 1 ist;
oder vorzugsweise
- R³: ist Halogen, OH, ein aromatischer C₆₋₃₀-Rest, der durch einen verzweigten, cyclischen oder vorzugsweise linearen C₁₋₂₀-Alkyl-, -Alkenyl-, -Alkoxy- oder -Alkenoxy-Rest substituiert sein kann, wobei die genannten Reste durch ein oder mehrere O, S oder N-Atome unterbrochen und/oder durch eine oder mehrere polymerisationsfähige Gruppen und/oder Reste R⁹ substituiert sein können, oder ein verzweigter, cyclischer oder vorzugsweise linearer C₁₋₂₀-Alkyl-, -Alkenyl-, -Alkoxy- oder -Alkenoxy-Rest ist, der ein- oder mehrfach durch O, S oder -NR²⁰- unterbrochen und durch eine oder mehrere polymerisationsfähige Gruppen und/oder Reste R⁹ substituiert sein kann, wobei
R⁹ -OH, -CₓF₂ₓ₊₁ mit x = 1 bis 20, -[Si(CH₃)₂]_{y}-CH₃ mit y = 1 bis 20 ist, und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist.

Bei den Acylgermanium-Verbindungen der Formel (I) handelt es sich um Mono-, Bis-, oder Triacylgermanium-Verbindungen, wobei Mono- und Bisacylgermanium-Verbindungen bevorzugt sind.

Erfindungsgemäß besonders bevorzugt sind Verbindungen gemäß der folgenden Formel (II) 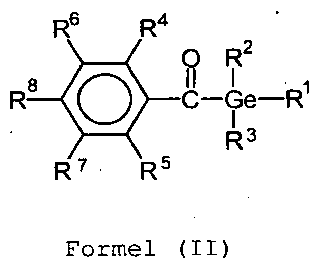 in der
- R¹, R²: unabhängig voneinander 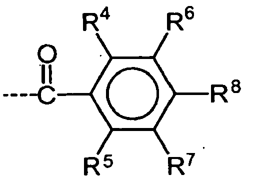 oder H sind, oder eine der für R³ angegebenen Bedeutungen haben;
- R³: Halogen, OH, ein aromatischer C₆₋₃₀-Rest, der durch einen verzweigten, cyclischen oder vorzugsweise linearen C₁₋₂₀-Alkyl-, -Alkenyl-, -Alkoxy- oder -Alkenoxy-Rest substituiert sein kann, wobei die genannten Reste durch ein oder mehrere O, S oder N-Atome unterbrochen und/oder durch eine oder mehrere polymerisationsfähige Gruppen und/oder Reste R⁹ substituiert sein können, oder ein verzweigter, cyclischer oder vorzugsweise linearer C₁₋₂₀-Alkyl-, -Alkenyl-, -Alkoxy- oder -Alkenoxy-Rest ist, der ein- oder mehrfach durch O, S oder -NR²⁰- unterbrochen und durch eine oder mehrere polymerisationsfähige Gruppen und/oder Reste R⁹ substituiert sein kann;
- R⁴, R⁵: unabhängig voneinander jeweils H, Halogen, ein verzweigter oder linearer C₁₋₆-Alkyl- oder -O-C₁₋₆-Alkylrest sind;
- R⁶, R⁷, R⁸: unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy-Rest sind, der ein- oder mehrfach durch O, S oder -NR²⁰- unterbrochen und durch eine oder mehrere polymerisationsfähige Gruppen und/oder Reste R⁹ substituiert sein kann;
- R⁹: -OH, -CₓF₂ₓ₊₁ mit x = 1 bis 20, -[Si(CH₃)₂]_{y}-CH₃ mit y = 1 bis 20 ist; und
- R²⁰: H, Halogen, ein verzweigter, cyclischer oder vorzugsweise linearer C₁₋₂₀-Alkyl-, -Alkenyl-, -Alkyloxy- oder -Alkenoxy-Rest ist.

Weiter bevorzugt sind Germanium-Verbindungen gemäß der folgenden Formel (III) 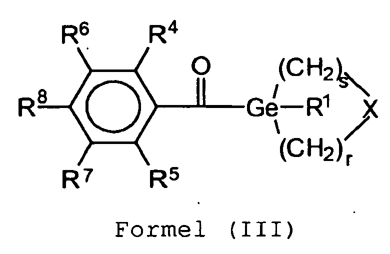 in der
- R¹: 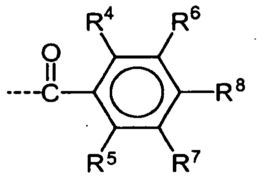 oder H ist, oder eine der für R³ angegebenen Bedeutungen hat;
- r, s: unabhängig voneinander eine ganze Zahl von 0 bis 6, vorzugsweise 1 bis 3 sind, wobei r und s so gewählt sind, daß die Summe der Ringatome einschließlich des Germaniumatoms 5 bis 8 beträgt, und
- X: N-R²¹, O, S oder entfällt bedeutet, wobei R²¹ H oder C₁₋₁₀-Alkyl, vorzugsweise H oder C₁₋₄-Alkyl ist.

Der Germanium enthaltende Ring kann mit einem oder mehreren, vorzugsweise 1 oder 2 aliphatischen oder aromatischen Ringen anelliert und unsubstituiert oder ein- oder mehrfach, vorzugsweise ein- oder zweifach substituiert sein, wodurch die Anzahl der Wasserstoffatome des Rings entsprechend reduziert wird. Bevorzugte Substituenten sind C₁₋₄-Alkylgruppen oder =O. Bevorzugt sind Verbindungen der Formel (III) in denen der Germanium enthaltende Ring nicht mit weiteren Ringen anelliert und abgersehen von R²¹ unsubstituiert ist.

Durch die Formel (I) und die übrigen hierin gezeigten Formeln werden sämtliche stereoisomere Formen sowie Gemische verschiedener stereoisomerer Formen, wie z.B. Racemate, erfaßt. Die Formeln erfassen nur solche Verbindungen, die mit der chemischen Valenzlehre vereinbar sind.

Der Hinweis, daß ein Rest durch ein Heteroatom wie O unterbrochen sein kann, ist so zu verstehen, daß die O-Atome in die Kohlenstoffkette des Restes eingeschoben werden, d.h. beidseitig von Kohlenstoffatomen begrenzt werden. Die Anzahl der O-Atome ist daher um mindestens 1 kleiner als die Zahl der Kohlenstoffatome, und die O-Atome können nicht endständig sein. Erfindungsgemäß sind Reste, die nicht durch O-Atome unterbrochen sind, bevorzugt.

Halogen (abgekürzt Hal) steht vorzugsweise für F, Cl, Br oder I, insbesondere für F, Cl, ganz besonders bevorzugt für Cl.

Bevorzugte polymerisationsfähige Gruppen, die bei den obigen Resten als Substituenten vorhanden sein können, sind Vinyl, Styryl, (Meth)acrylat, (Meth)acrylamid und/oder N-Alkylacrylamid, besonders bevorzugt (Meth)acrylat, (Meth)acrylamid und/oder N-Alkylacrylamid. Vorzugsweise sind die Reste R², R³, R⁶, R⁷ und R⁸ mit 0 bis 3, insbesondere 0 bis 1 polymerisierbaren Gruppen substituiert. Die polymerisationsfähigen Gruppen sind vorzugsweise endständig angeordnet.

Erfindungsgemäß sind solche Verbindungen der allgemeinen Formeln (I) und (II) bevorzugt, bei denen die Variablen die folgenden Bedeutungen haben, die unabhängig voneinander gewählt werden können:
- R¹: 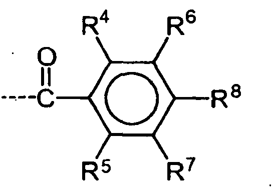 oder H, oder eine der für R² und R³ angegebenen Bedeutungen;
- R², R³: unabhängig voneinander ein linearer C₁₋₄-Alkyl-, oder -Alkenyl-Rest, der durch eine oder mehrere polymerisationsfähige Gruppen substituiert sein kann;
- R⁴, R⁵: unabhängig voneinander jeweils H, Halogen, ein verzweigter oder linearer C₁₋₄-Alkyl- oder -O-C₁₋₄-Alkylrest;
- R⁶, R⁷, R⁸: unabhängig voneinander jeweils H, Halogen, ein linearer C₁₋₂₀-Alkyl-, -Alkenyl-, -Alkoxy- oder -Alkenoxy-Rest, der durch ein oder mehrere -O-, -S- oder -NR²⁰-Reste unterbrochen und durch ein oder mehrere polymerisationsfähige Gruppen substituiert sein kann.

Besonders bevorzugte Definitionen der Variablen, die ebenfalls unabhängig voneinander gewählt werden können, sind:
- R¹: 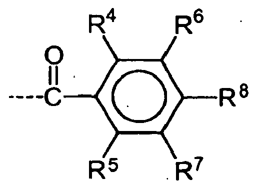 oder eine der für R² und R³ angegebenen Bedeutungen;
- R², R³: C₁-C₄-Alkyl;
- R⁴, R⁵, R⁸: H, Cl, CH₃, OCH₃;
- R⁶,R⁷: H, C₁-C₄-Alkyl, das durch ein oder mehrere O-Atome unterbrochen sein kann.

Ganz besonders bevorzugt sind Verbindungen der Formel (II) in denen R²=R³, R⁴=R⁵ und/oder R⁶=R⁷ ist.

Besonders bevorzugt sind naturgemäß solche Verbindungen, bei denen alle Variablen eine der bevorzugten und insbesondere der besonders bevorzugten Bedeutungen haben.

Es sind solche Acylgermanium-Verbindungen gemäß der Formel (I) und insbesondere der Formel (II) bevorzugt, die 0 bis 2, vorzugsweise 0 oder 1 polymerisationsfähige Gruppe enthalten. Die einzelnen Reste der Formel (I) enthalten vorzugsweise 0 bis 4, besonders bevorzugt 0 bis 2 polymerisierbare Gruppen.

Konkrete Beispiele für besonders bevorzugte Verbindungen sind:

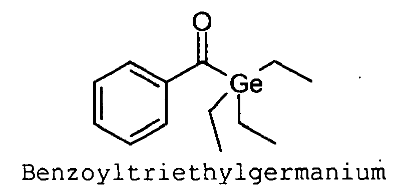

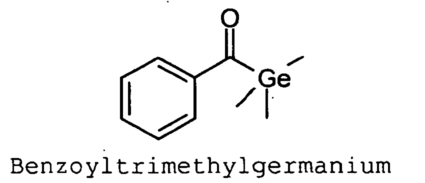

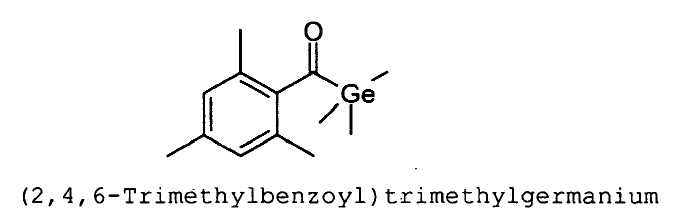

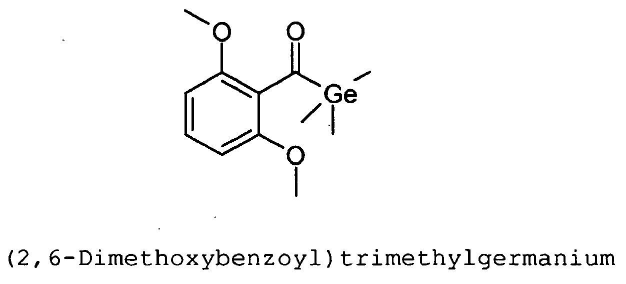

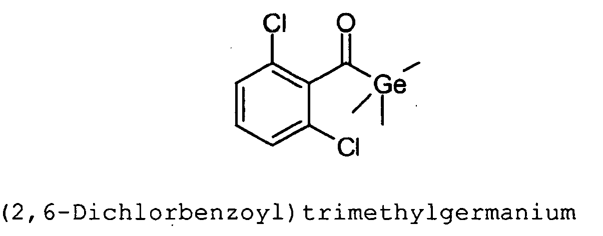

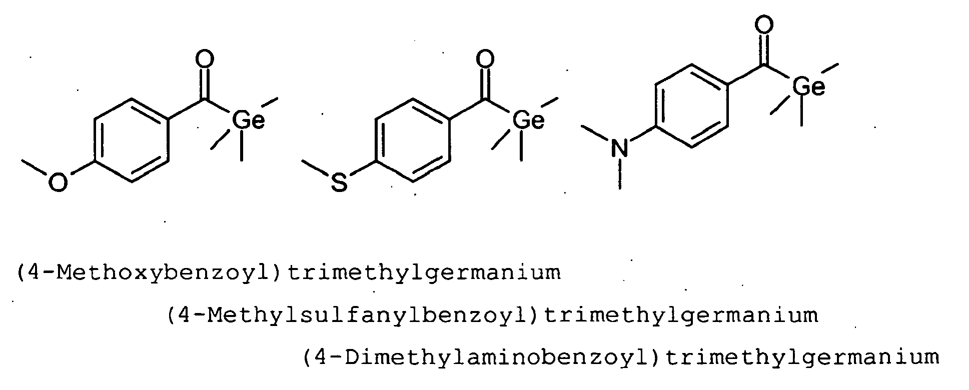

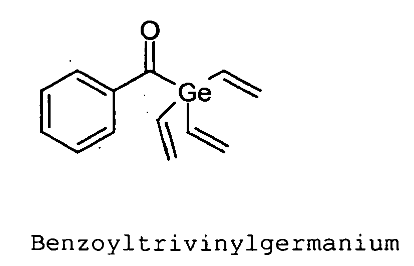

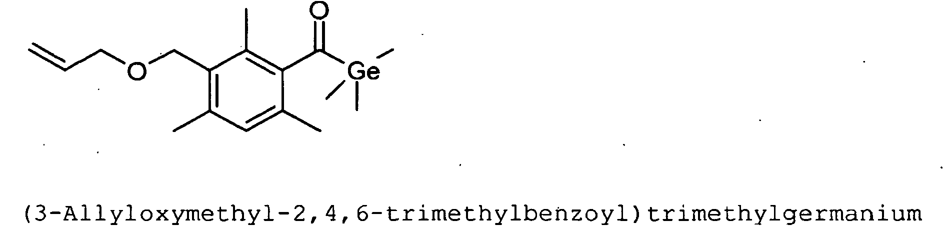

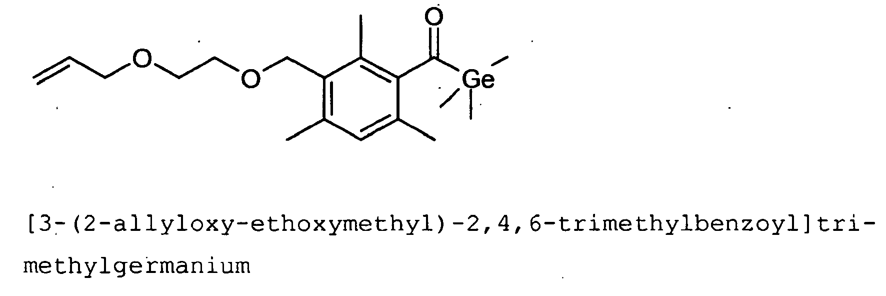

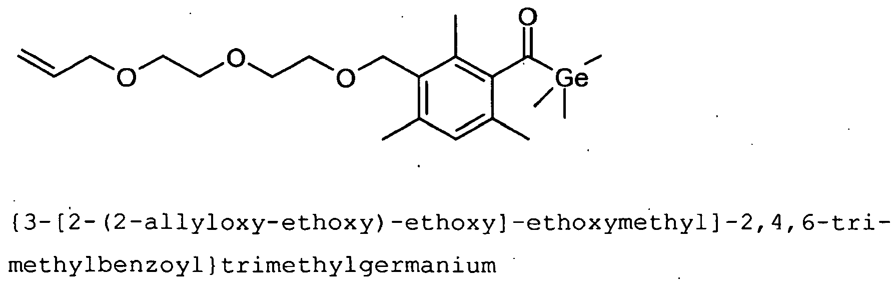

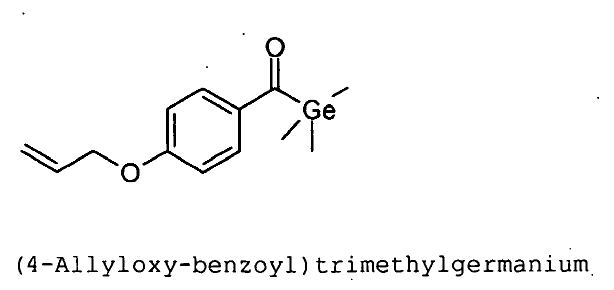

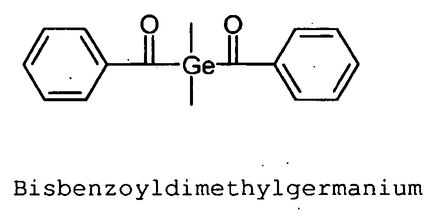

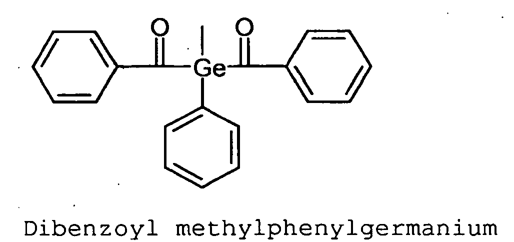

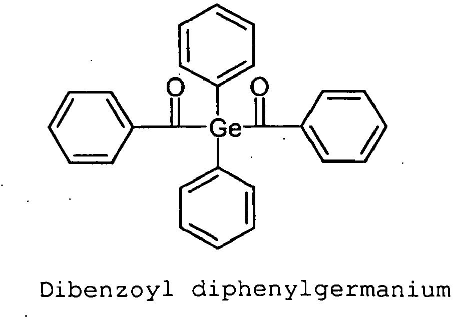

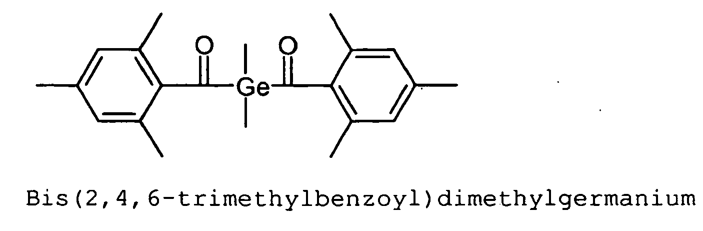

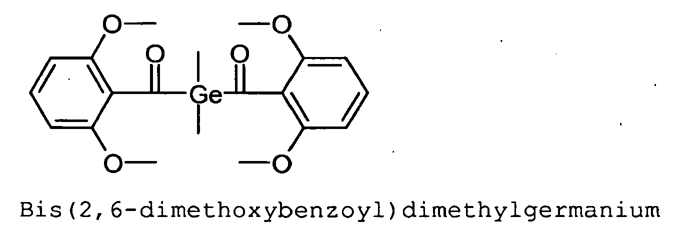

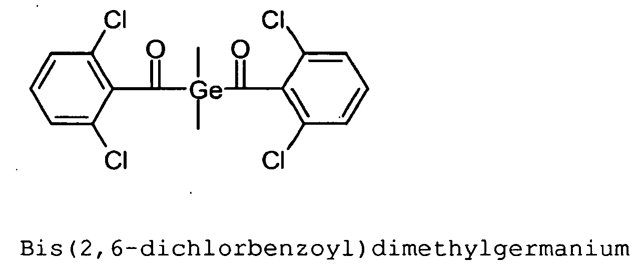

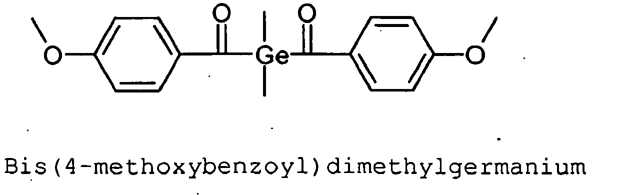

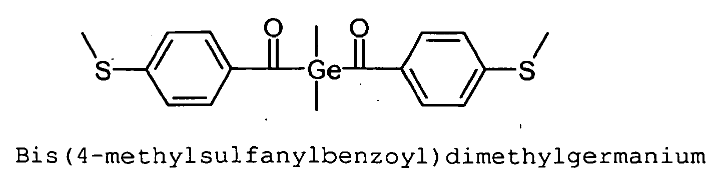

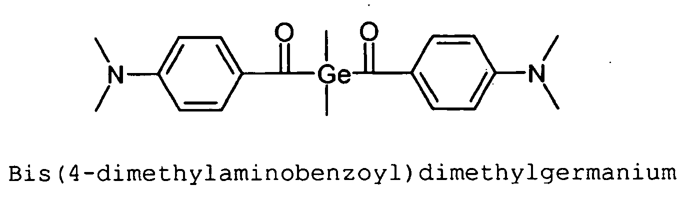

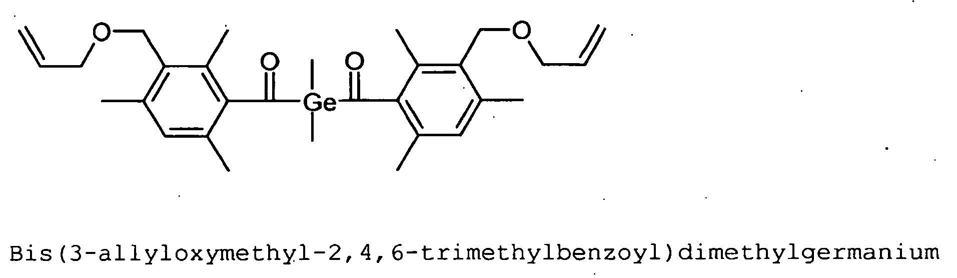

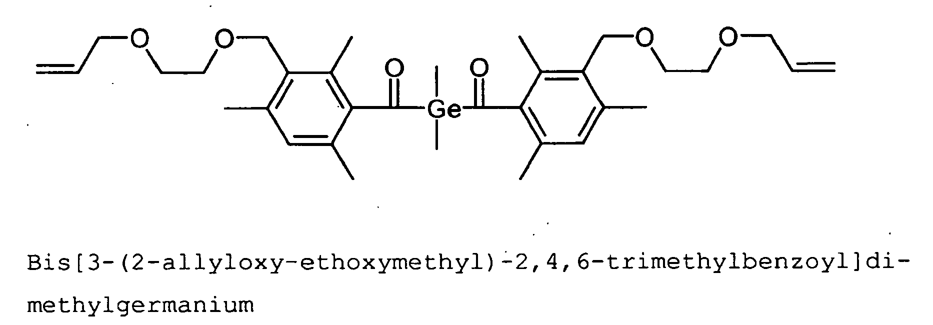

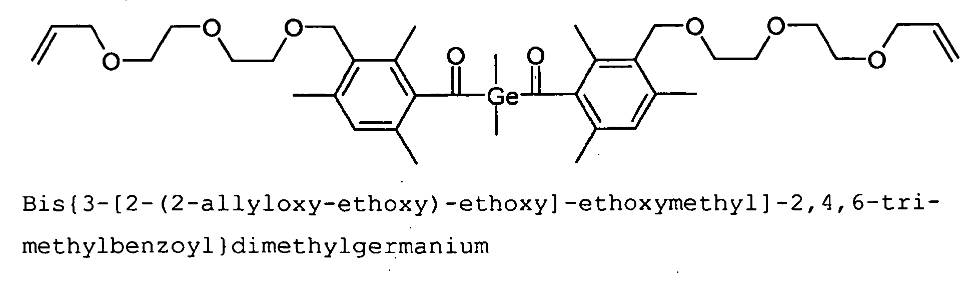

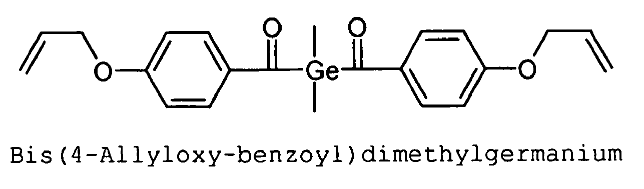

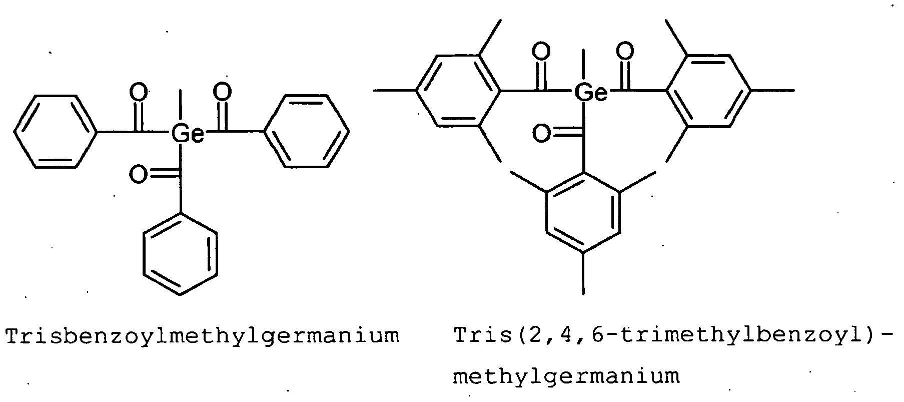

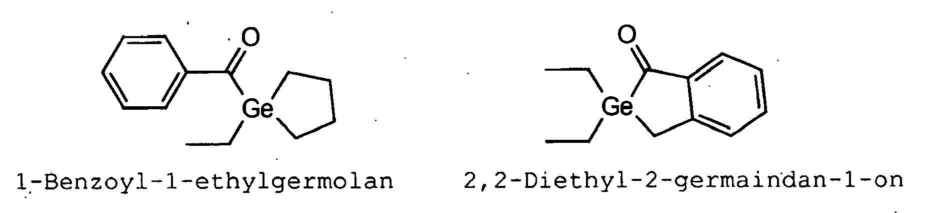

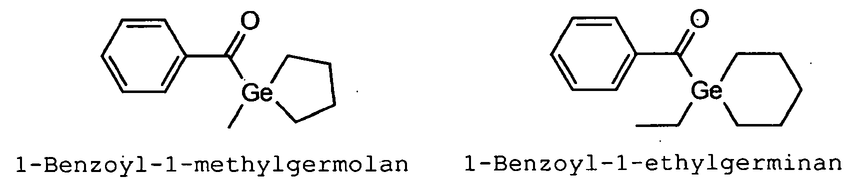

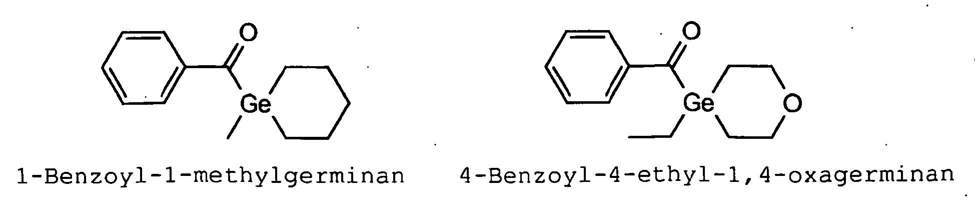

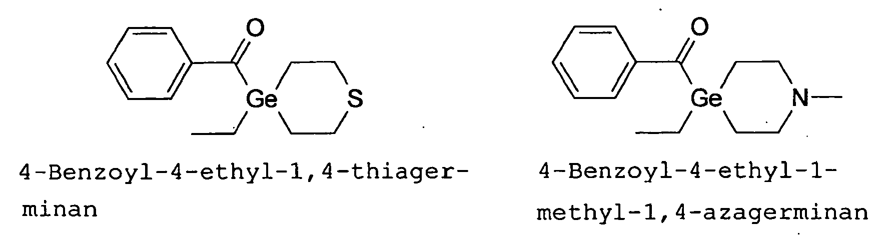

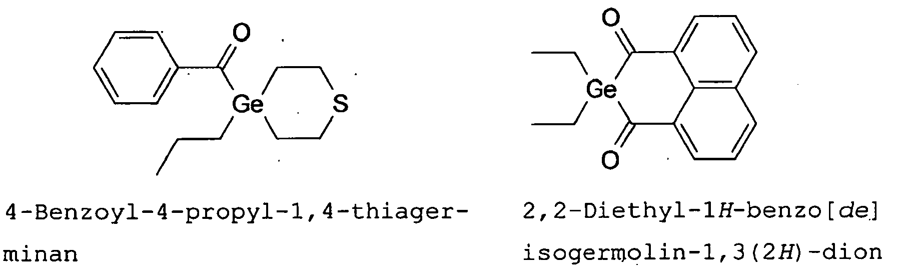

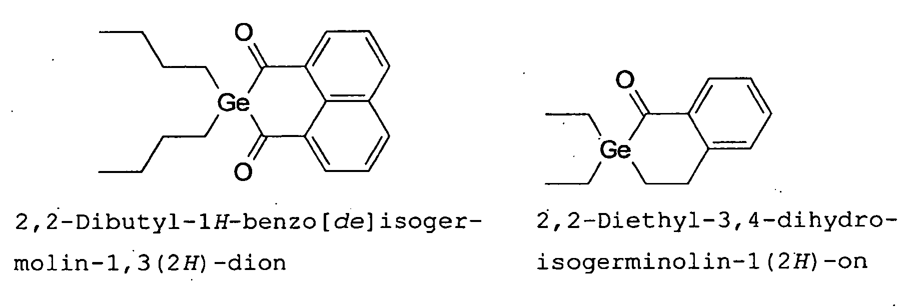

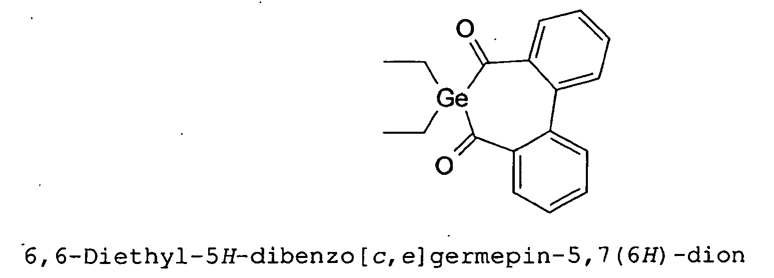

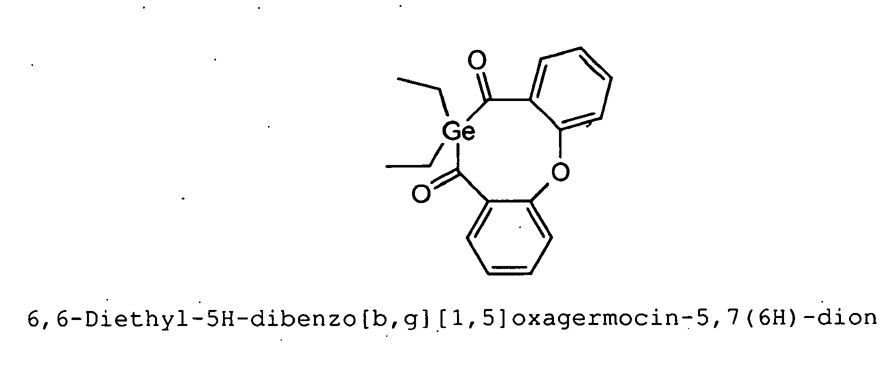

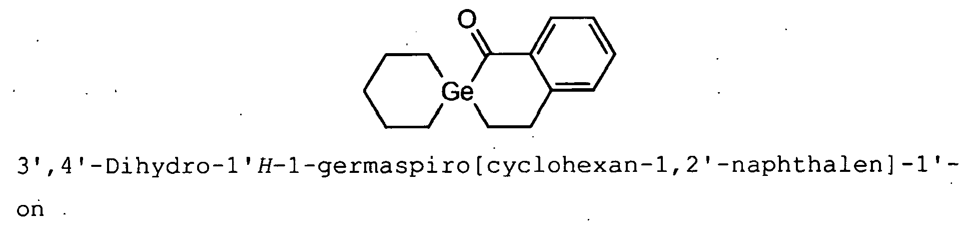

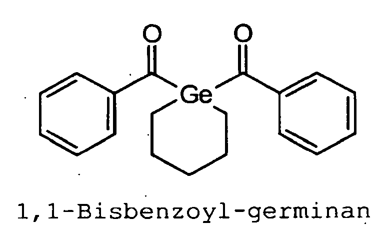

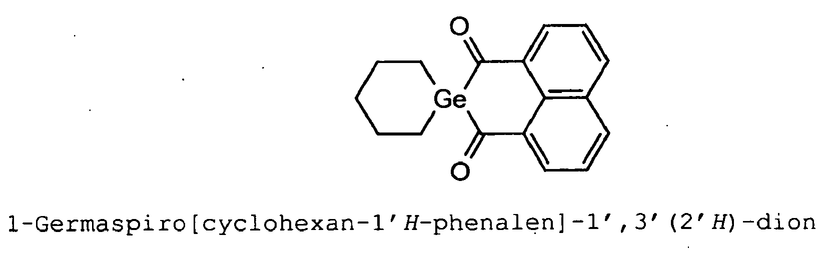

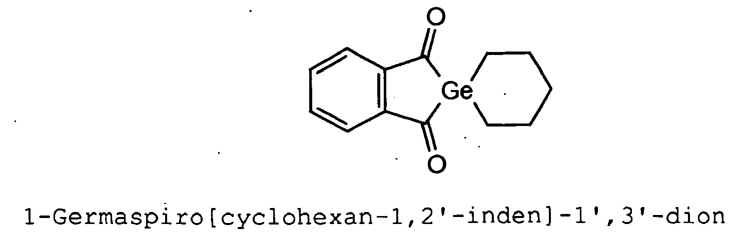

Ebenso bevorzugt sind die zu den oben gezeigten Verbindungen strukturähnlichen Verbindungen (2,4,6-Trimethylbenzoyl)-triethylgermanium, (2,4,6-Trimethylbenzoyl)tripropylgermanium, (2,4,6-Trimethylbenzoyl)tributylgermanium, (2,6-Dimethoxybenzoyl)triethylgermanium, (2,6-Dimethoxybenzoyl)tripropylgermanium, (2,6-Dimethoxybenzoyl)tributylgermanium, (2,6-Dichlorbenzoyl)triethylgermanium, (2,6-Dichlorbenzoyl)-tripropylgermanium, (2,6-Dichlorbenzoyl)tributylgermanium, Bisbenzoyldiethylgermanium, Bisbenzoyldipropylgermanium, Bisbenzoyldibutylgermanium, Bis(2,4,6-trimethylbenzoyl)diethylgermanium, Bis(2,4,6-trimethylbenzoyl)dipropylgermanium, Bis(2,4,6-trimethylbenzoyl)dibutylgermanium, Bis(2,6-dimethoxybenzoyl)diethylgermanium, Bis(2,6-dimethoxybenzoyl)dipropylgermanium, Bis(2,6-dimethoxybenzoyl)dibutylgermanium, Bis(2,6-dichlorbenzoyl)diethylgermanium, Bis(2,6-dichlorbenzoyl)dipropylgermanium, Bis(2,6-dichlorbenzoyl)-dibutylgermanium, Trisbenzoylethylgermanium und Tris(2,4,6-trimethylbenzoyl)ethylgermanium.

Die erfindungsgemäß verwendeten Acylgermanium-Verbindungen der allgemeinen Formel (I) sind zum Teil bereits aus dem Stand der Technik bekannt. Die Synthese der Monoacylgermane kann z.B. nach einer Methode von Yamamoto et. al. (Yamamoto, K.; Hayashi, A.; Suzuki, S.; Tsuji J.; Organometallics; 6 (1987) 974) durch Umsetzung von Hexaalkyldigermanium mit Säurechlorid erfolgen:

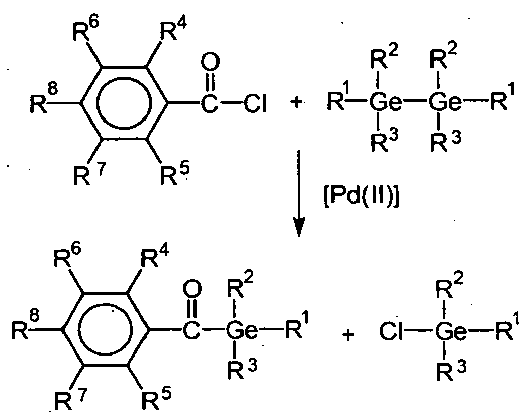

### Konkretes Beispiel:

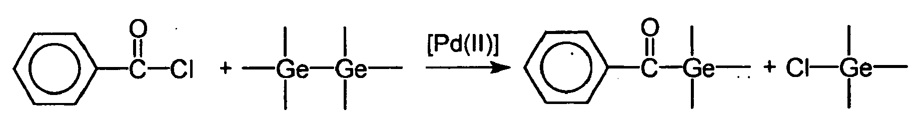

Eine weitere Möglichkeit Bisacylgermane herzustellen, ist die Umsetzung der entsprechenden lithierten Germaniumverbindungen mit Säurechloriden nach Castel et. al. (Castel, A.; Riviere, P.; Satgé, J.; Ko, H.Y.; Organometallics; 9 (1990) 205):

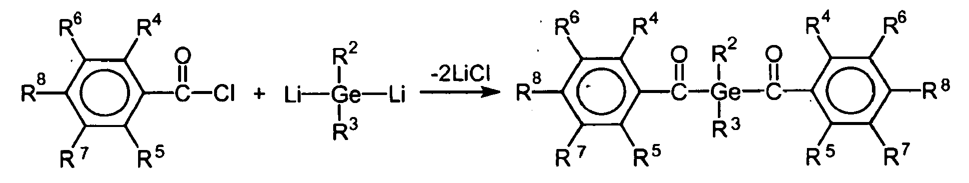

### Konkretes Beispiel:

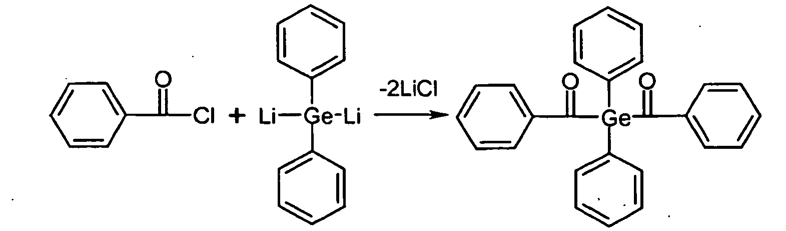

Die Herstellung von lithierten aromatischen Germaniumverbindungen kann z.B. durch Reaktion des entsprechenden Germaniumhalids (X = Halogen) mit Lithium (Li) (Nishimura, T.; Inoue-Ando, S.; Sato, Y., J. Chem. Soc., Perkin Trans.1; (1994) 1589) oder des Hydrogermaniums mit n-Butyllithium (BuLi) erfolgen (Castel, A.; Riviere, P.; Satgé, J.; Ko, H.Y.; Organometallics; 9 (1990) 205):

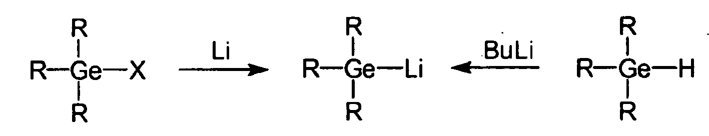

Des weiteren lassen sich Mono- und Bisacylgermane synthetisieren, indem ein Carbanion, welches aus 1,3-Dithianen erhalten wird, mit Germaniumchloriden nach Brook et. al. umgesetzt wird (Brook, A. G.; Duff, J. M.; Jones, P. F.; Davis, N. R.; "Synthesis of Silyl and Germyl Ketones" J. Am Chem. Soc. 89(2), 431 - 434 (1967)). Dieser Syntheseweg eignet sich besonders zur Herstellung von Bisalkylbisacylgermanen:

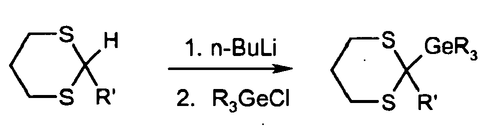

Die erhaltenen Dithiane können nach Methoden, die dem Fachmann allgemein bekannt sind, zu den entsprechenden Ketonen hydrolysiert werden (nach Brook, A. G.; Duff, J. M.; Jones, P. F.; Davis, N. R.; "Synthesis of Silyl and Germyl Ketones" J. Am Chem. Soc. 89(2), 431-434 (1967) oder z.B. auch nach Sharma, H.K.; Cervanes-Lee, F.; Pannel, K. H.; "Organometalloidal derivatives of the transition metals, XXVII. Chemical and structural investigations on (ferrocenylacyl)germanes)", J. Organomet. Chem. 409, 321-330 (1991):

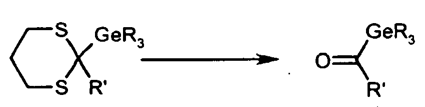

Die Acylgermanium-Verbindungen der allgemeinen Formel (I) eignen sich besonders als Photoinitiatoren für die Polymerisation, insbesondere als Initiatoren für die radikalische Polymerisation, Photoaddition und für die Thiol-En-Reaktion (Polyaddition). Es wurde gefunden, daß mit diesen Initiatoren bei Bestrahlung mit Licht, vorzugsweise im sichtbaren Bereich, insbesondere mit einer Wellenlänge von 400 bis 500 nm, im Vergleich zu herkömmlichen Photoinitiatoren eine hohe Durchhärtungstiefe erzielt werden kann, ohne daß die Initiatoren zu gefärbten Materialien führen. Dies ist bei vielen technischen und besonders medizinischen Werkstoffen, wie z.B. Dentalwerkstoffen und Knochenzementen von großem Vorteil.

Darüber hinaus zeichnen sich die erfindungsgemäß verwendeten Acylgermanium-Verbindungen der Formel (I) im Vergleich zu herkömmlichen Initiatoren durch eine geringere Zytotoxizität aus, was für medizinische Anwendungen ebenfalls ein besonderer Vorteil ist. Die Acylgermanium-Verbindungen eignen sich daher beispielweise auch als Initiatoren für Materialien zur Herstellung von Kontaktlinsen aber auch von gewöhnlichen optischen Linsen, die von der geringen Verfärbungsneigung der Initiatoren profitieren.

Die Verwendung der Initiatoren der Formel (I) ist nicht auf medizinische Anwendungen beschränkt. Die große Durchhärtungstiefe bei der Härtung mit Licht im sichtbaren Wellenlängenbereich ist auch bei technischen Anwendungen ein erheblicher Vorteil. Die erfindungsgemäßen Zusammensetzungen eignen sich für eine Vielzahl von Anwendungszwecken, wie zum Beispiel als Druck- oder Anstrichfarben, Lacke, Adhäsive, zur Herstellung von Druckplatten, integrierten Schaltkreisen, Photoresists, Lötmasken, Tinten für Farbdrucker, als Materialien für die holographische Datenspeicherung, zur Herstellung von nano-dimensionierten mikroelektromechanischen Elementen, Lichtwellenleitern, Formteilen und zur optischen Herstellung von Informationsträgern.

Zur Initiierung der Polymerisation werden die Acylgermanium-Verbindungen der Formel (I) vorzugsweise mit Licht im Wellenlängenbereich von 200 bis 750 nm, besonders bevorzugt 200 bis 550 nm, weiter bevorzugt 300 bis 550 nm und ganz besonders bevorzugt 350 bis 500 nm bestrahlt. Sie sind damit als Initiatoren für die Laser-Härtung und die Laser induzierte 3D Härtung sowie für die 2-Photonen Polymerisation brauchbar. Sie eignen sich insbesondere als Initiatoren für pigmentierte Systeme, da sie die Nutzung von Absorptionslücken des Pigments ermöglichen.

Besonders vorteilhaft ist, daß die Initiatoren auch mit LED Lichtquellen aktiviert werden können. Die Wellenlänge von LEDs ist abhängig von der Gitterkonstante des Substrates. Die Qualität (thermische Festigkeit, Wärmeausdehnung, Konstanz der Atomabstände etc.) des Substrats bestimmt die Höhe der möglichen Leistung der LEDs. In der intra-oralen Anwendung sind Wellenlängen erst ab etwa 380 nm erlaubt, so daß Initiatoren der Formel (I), die sich mit einer Wellenlänge im Bereich von 380 nm und mehr aktivieren lassen, besonders bevorzugt sind.

Gegenstand der Erfindung sind auch Kombinationen von LED-Lichtquellen mit Initiatoren gemäß der Formel (I) oder mit Zusammensetzungen, die einen solchen Initiator enthalten. Zur dentalen Anwendung sind Systeme von LED-Lichtquellen mit einer Wellenlänge im Bereich von 400 bis 550 nm, vorzugsweise 400 bis 480 nm und insbesondere 450 ± 20 nm, und darauf abgestimmten Initiatoren bzw. Zusammensetzungen bevorzugt, d.h. Initiatoren mit einer Aktivierungswellenlänge im Bereich von 400 bis 550 nm, vorzugsweise 400 bis 480 nm und ganz besonders bevorzugt ca. 450 ± 20 nm und diese enthaltende Zusammensetzungen. Daneben sind LED-Lichtquellen mit einer Wellenlänge von etwa 650 ± 30 nm oder etwa 360 ± 30 nm zusammen mit darauf abgestimmten Initiatoren oder Zusammensetzungen erfindungsgemäß bevorzugt.

Die erfindungsgemäßen Zusammensetzungen enthalten neben mindestens einer Acylgermanium-Verbindung der Formel (I) vorzugsweise auch ein polymerisierbares Bindemittel. Bevorzugt sind Bindemittel auf der Basis radikalisch und/oder kationisch polymerisierbarer Monomere und/oder Prepolymere.

Als radikalisch polymerisierbare Bindemittel eignen sich besonders mono- oder multifunktionelle (Meth)acrylate oder deren Mischung. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 polymerisierbaren Gruppen verstanden.

Diesbezügliche Beispiele sind Methyl-, Ethyl-, Hydroxyethyl-, Butyl-, Benzyl-, Tetrahydrofurfuryl- oder Isobornyl-(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)-acrylat, Trimethylolpropantri(meth)acrylat, Pentaerythrittetra(meth)acrylat, sowie Glycerindimethacrylat, 1,4-Butandioldi(meth)acrylat, 1,10-Decandioldi(meth)acrylat oder 1,12-Dodecandioldi(meth)acrylat. Zusammensetzungen, die mindestens ein radikalisch polymerisierbares Monomer mit 2 oder mehr, vorzugsweise 2 bis 3 radikalisch polymerisierbaren Gruppen enthalten, sind besonders bevorzugt. Mehrfach funktionelle Monomere haben vernetzende Eigenschaften.

Als radikalisch polymerisierbare Bindemittel lassen sich auch hydrolysestabile Monomere, wie hydrolysestabile Mono(meth)acrylate, z.B. Mesitylmethacrylat oder 2-(Alkoxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethylacrylamid, N-(2-Hydroxyethyl)acrylamid, N-Methyl-N-(2-hydroxyethyl)-acrylamid oder N-monosubstituierte Methacrylamide, wie z.B. N-Ethylmethacrylamid oder N-(2-Hydroxyethyl)methacrylamid sowie außerdem N-Vinylpyrrolidon oder Allylether einsetzen. Bevorzugte Beispiele für hydrolysestabile Vernetzermonomere sind Urethane aus 2-(Hydroxymethyl)acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechorid synthetisiert werden können.

Bevorzugt sind bei Raumtemperatur flüssige Monomere, die als Verdünnermonomere eingesetzt werden können.

Weiterhin lassen sich als radikalisch polymerisierbare Bindemittel auch schrumpfungsarme radikalisch ringöffnend polymerisierbare Monomere wie z.B. mono- oder multifunktionelle Vinylcyclopropane oder bicyclische Cyclopropanderivate, vorzugsweise die in DE 196 16 183 C2 oder EP 1 413 569 beschriebenen, oder cyclische Allylsulfide, vorzugsweise die in US 6,043,361 und US 6,344,556 beschriebenen, einsetzen. Diese können auch in Kombination mit den voranstehend aufgeführten Di(meth)acrylat-Vernetzern verwendet werden. Bevorzugte ringöffnend polymerisierbare Monomere sind Vinylcyclopropane, wie 1,1-Di(ethoxycarbonyl)- oder 1,1-Di(methoxycarbonyl)-2-vinylcyclopropan, die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl- oder -ethylester, deren Disubstitutionsprodukte in 3-Stellung wie (3,3-Bis(ethoxycarbonyl)bicyclo[3.1.0]hex-1-yl)acrylsäuremethyl- oder -ethylester. Bevorzugte cyclische Allylsulfide sind die Additionprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder dem asymmetrischen Hexamethylendiisocyanat-Trimeren (Desmodur^{®} VP LS 2294 der Bayer AG).

Außerdem können als radikalisch polymerisierbare Bindemittel auch Styrol, Styrolderivate oder Divinylbenzol, ungesättigte Polyester-, Polyurethan- und Epoxy-Harze sowie Allylverbindungen oder radikalisch polymerisierbare Polysiloxane, die aus geeigneten Methacrylsilanen, wie z.B. 3-(Methacryloyloxy)propyltrimethoxysilan, hergestellt werden können und z.B. in der DE 199 03 177 C2 beschrieben sind, eingesetzt werden.

Weiterhin lassen sich als radikalisch polymerisierbare Bindemittel auch Mischungen der voranstehend genannten Monomeren mit radikalisch polymerisierbaren, säuregruppenhaltigen Monomeren verwenden, die auch als Haftmonomere bezeichnet werden. Bevorzugte säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin oder 4-Vinylbenzoesäure.

Als Haftmonomere eignen sich auch radikalisch polymerisierbare Phosphonsäuremonomere, insbesondere Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[2-Dihydroxyphosphoryl)-ethoxymethyl]-acrylsäure-ethyl- oder 2,4,6-trimethylphenylester.

Zudem eignen sich als Haftmonomere acide polymerisationsfähige Phosphorsäureester, insbesondere 2-Methacryloyloxypropylmono- oder -dihydrogenphosphat, 2-Methacryloyloxyethylmono- oder - dihydrogenphosphat, 2-Methacryloyloxyethylphenyl-hydrogenphosphat, Dipentaerythritol-pentamethacryloyloxyphosphat, 10-Methacryloyloxydecyl-dihydrogenphosphat, Dipentaerythritol-pentamethacryloyloxyphosphat, Phosphorsäuremono-(1-acryloylpiperidin-4-yl)-ester, 6-(Methacrylamido)hexyldihydrogenphosphat und 1,3-Bis-(N-acryloyl-N-propyl-amino)-propan-2-yl-dihydrogenphosphat.

Darüber hinaus eignen sich polymerisationsfähige Sulfonsäuren als Haftmonomere, insbesondere Vinylsulfonsäure, 4-Vinylphenylsulfonsäure oder 3-(Methacrylamido)propylsulfonsäure.

Als durch Polyaddition härtbare Bindemittel eignen sich besonders Thiol-En-Harze, die Mischungen von mono- oder multifunktionellen Mercaptoverbindungen und di- oder multifunktionellen ungesättigten Monomeren, vor allem Allyl- oder Norbonenverbindungen enthalten.

Beispiele für mono- oder multifunktionelle Mecaptoverbindungen sind o-, m- oder p-Dimercaptobenzol und Ester der Thioglykol- oder der 3-Mercaptopropionsäure von Ethylen-, Propylen- oder Butylenglykol, Hexandiol, Glycerin, Trimethylolpropan oder Pentaerythrit.

Beispiele für di- oder multifunktionelle Allylverbindungen sind Ester von Allylalkohol mit Di- oder Tricarbonsäuren, wie Malon- Malein- Glutar-, Bernstein-, Adipin-, Sebacin-, Phthal-, Terephthal- oder Gallussäure sowie mono- oder trifunktionelle Allylether, wie z.B. Diallylether, α,ω-Bis[allyloxy]alkane, Resorcin- oder Hydrochinondiallylether sowie Pyrogalloltriallylether, oder andere Verbindungen wie z.B. 1,3,5-Triallyl-1,3,5-triazin-2,4,6-(1H,3H,5H)-trion, Tetraallylsilan oder Tetraallylorthosilikat.

Beispiele für di- oder multifunktionelle Norbonenverbindungen sind Diels-Alder-Additionsprodukte von Cyclopentadien oder Furan mit di- oder multifunktionellen (Meth)acrylaten, sowie Ester und Urethane von 5-Norbornen-2-methanol oder 5-Norbornen-2-ol mit Di- oder Polycarbonsäuren, wie z.B. Malon-Malein- Glutar-, Bernstein-, Adipin-, Sebacin-, Phthal-, Terephthal- oder Gallussäure, mit Di- oder Polyisocyanaten, wie Hexamethylendiisocyanat oder dessen cyclischem Trimer, 2,2,4-Trimethylhexamethylendiisocyanat, Toluylendiisocyanat oder Isophorondiisocyanat.

Weiterhin lassen sich Acylgermanium-Verbindungen der Formel (I) als Co-Initiatoren bzw. Photosensibilisatoren für die kationische Polymerisation von kationisch polymerisierbaren Monomeren einsetzen. Bevorzugte kationisch polymerisierbare Verdünner- oder Vernetzermonomere sind z.B. Glycidylether oder cycloaliphatische Epoxide, cyclische Ketenacetale, Vinylether, Spiroorthocarbonate, Oxetane oder bicyclische Orthoester. Besonders bevorzugte Beispiele sind: Triethylenglycoldivinylether, Cyclohexandimethanoldivinylether, 2-Methylen-1,4,6-trioxaspiro[2.2]nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxy-cyclohexylmethyl-3,4-epoxycyclohexancarboxylat, Bis(-(3,4-epoxycyclohexylmethyl)adipat, Vinylcyclohexendioxid, 3-Ethyl-3-hydroxymethyloxetan, 1,10,-Decandiylbis(oxymethylen)bis(3-ethyloxetan) oder 3,3-(4-Xylylendioxy)-bis-(methyl-3-ethyloxetan).

Als kationisch polymerisierbare Bindemittel eignen sich auch Kieselsäurepolykondensate, die beispielsweise durch hydrolytische Kondensation von Silanen zugänglich sind, die kationisch polymerisierbare Gruppen, bevorzugt z.B. Epoxid-, Oxetan-, Spiroorthoester und/oder Vinylethergruppen tragen. Solche Kieselsäurepolykondensate sind beispielsweise in der DE 41 33 494 C2 oder US 6,096,903 beschrieben.

Neben Acylgermanium-Verbindungen der allgemeinen Formel (I) können die erfindungsgemäßen Zusammensetzungen zusätzlich auch bekannte Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z.B: Benzoinether, Dialkylbenzilketale, Dialkoxyacetophenone, Acyl- oder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon sowie ggf. Coinitiatoren wie tertiäre Amine z.B Dimethylaminobenzoesäureethylester oder N-Methyldiethanolamin enthalten.

Außderdem können die erfindungsgemäßen Zusammensetzungen neben den Acylgermanium-Verbindungen der allgemeinen Formel (I) zusätzlich einen oder mehrere kationische Photoinitiatoren enthalten, vorzugsweise Diaryliodonium- oder Triarylsulfoniumsalze. Bevorzugte Diaryliodoniumsalze sind die kommerziell erhältlichen Photoinitiatoren 4-Octyloxy-phenyl-phenyliodoniumhexafluoroantimonat, Isopropylphenyl-methylphenyliodoniumtetrakis(pentafluorophenyl)borat und 4-Phenylsulfanylphenyl diphenylsulfonium hexafluorphosphat. Diese kationischen Photoinitiatoren können zur Beschleunigung der Aushärtung von erfindungsgemäßen Zusammensetzungen auf der Basis von Acylgermanium-Verbindungen der allgemeinen Formel (I) eingesetzt werden. Umgekehrt läßt sich die Aushärtung von Zusammensetzungen mit kationischen Initiatoren durch die Zugabe von Acylgermanium-Verbindungen der Formel (I) beschleunigen.

Weiterhin können die erfindungsgemäßen Zusammensetzungen neben den Acylgermanium-Verbindungen der allgemeinen Formel (I) zur dualen Härtung auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, *tert*-Butylperoctoat, *tert*-Butylperbenzoat oder Di-(*tert*-butyl)-peroxid enthalten. Zur Beschleunigung der Initiierung mittels Peroxiden lassen sich Kombinationen mit aromatischen Aminen einsetzen. Bevorzugte Redoxsysteme sind Kombinationen aus Benzoylperoxid mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und Reduktionsmitteln, wie z.B. Ascorbinsäure, Barbiturate oder Sulfinsäuren, für die duale Aushärtung geeignet. Die Menge an zusätzlichen Initiatoren liegt vorzugsweise im Bereich von 0 bis 3 Gew.-%.

Erfindungsgemäß sind Zusammensetzungen bevorzugt, die einen oder mehrere Füllstoffe, vorzugsweise organische oder anorganische partikuläre Füllstoffe enthalten. Bevorzugte anorganische partikulären Füllstoffe sind amorphe kugelförmige nanopartikuläre Füllstoffe auf der Basis von Oxiden, wie pyrogener Kieselsäure oder Fällungskieselsäure, ZrO₂ und TiO₂ oder Mischoxiden von SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengröße von 0,2 bis 5 µm sowie röntgenopake Füllstoffe, wie Ytterbiumtrifluorid, nanopartikuläres Tantal(V)-oxid oder Bariumsulfat. Darüber hinaus können auch faserförmige Füllstoffe wie Nanofasern, Glasfasern, Polyamid- oder Kohlenstoff-Fasern eingesetzt werden.

Als weite Komponente können die erfindungsgemäßen Zusammensetzungen Farbmittel wie Farbstoffe und/oder Pigmente enthalten.

Außerdem können die erfindungsgemäßen Zusammensetzungen im Bedarfsfalle weitere Additive sowie Lösungsmittel, wie z.B. Wasser, Ethanol, Aceton und/oder Ethylacetat, enthalten.

Die Additive sind vorzugsweise aus Stabilisatoren, UV-Absorbern, Gleitmitteln, Netzmitteln, Dispergiermitteln, Haftvermittlern, Mattierungs- und Glanzmitteln, Verlaufs- und Filmbildehilfsmitteln, Hautverhinderungsmitteln, Lichtschutzmitteln, Korrosionsschutzmitteln, flammhemmenden Mitteln, Antioxidantien, optischen Aufheller, Fließverbesserern, Verdickern und Antischaummitteln ausgewählt.

Die Initiatoren gemäß den Formeln (I) und (II) zeichnen sich durch eine hohe Reaktivität aus und können daher in geringen Konzentrationen eingesetzt werden (vgl. Beispiel 7). Darüber hinaus gestatten sie die Aushärtung auch von dünnen Schichten oder Filmen ohne die Ausbildung einer Inhibierungsschicht. Die erfindungsgemäßen Initiatoren können daher zur Verhinderung der Ausbildung einer Inhibierungsschicht eingesetzt werden.

Die erfindungsgemäßen Zusammensetzungen enthalten, bezogen auf die Gesamtmasse der Zusammensetzung, vorzugsweise 0,001 bis 5 Gew.-%, besonders bevorzugt 0,01 bis 4 Gew.-% und insbesondere 0,1 bis 3 Gew.-% Acylgermanium-Verbindung der Formel (I).

Die erfindungsgemäßen Materialien enthalten somit vorzugsweise:
(a) 0,001 bis 5 Gew.-% Acylgermanium-Verbindung der allgemeinen Formel (I), vorzugsweise 0,01 bis 4 Gew.-%, besonders bevorzugt 0,1 bis 3 Gew.-%,
(b) 5 bis 99,9 Gew.-% polymerisierbares Bindemittel, vorzugsweise 10 bis 95 Gew.-%, besonders bevorzugt 15 bis 90 Gew.-%, und
(c) 0 bis 90 Gew.-% Füllstoff, vorzugsweise 5 bis 87 Gew.-%, besonders bevorzugt 10 bis 85 Gew.-%.

Die Zusammensetzungen können vorteilhaft außerdem enthalten:
(d) 0 bis 50 Gew.-% Additiv, vorzugsweise 0,01 bis 4 Gew.-%, besonders bevorzugt 0,1 bis 3 Gew.-%, wobei sich diese Mengenangaben auf die Gesamtmasse aller Additive beziehen, und
(e) 0 bis 10 Gew.-%, vorzugsweise 0.01 bis 5 Gew.-% Pigmente und/oder Farbstoffe.

Alle Prozentangaben beziehen sich, wenn nicht anders angegeben, auf die Gesamtmasse der Zusammensetzung.

Die erfindungsgemäßen Zusammensetzungen eignen sich besonders als Adhäsive, Beschichtungen, Lacke, Tinten, Zemente, Komposite, zur Herstellung von Formteilen bzw. Formkörpern, wie Stäben, Platten, Scheiben, optischen Linsen, Kontaktlinsen, und insbesondere als Dentalwerkstoffe, ganz besonders als Füllungskomposite.

Zusammensetzungen zur Anwendung als dentale Zemente enthalten bevorzugt:
(a) 0,001 bis 3 Gew.-% Acylgermanium-Verbindung der allgemeinen Formel (I),
(b) 20 bis 70 Gew.-% polymerisierbares Bindemittel,
(c) 30 bis 75 Gew.-% Füllstoff und
(d) 0,01 bis 5 Gew.-% Additiv.

Zusammensetzungen zur Anwendung als dentale Komposite enthalten bevorzugt:
(a) 0,001 bis 2 Gew.-% Acylgermanium-Verbindung der allgemeinen Formel (I),
(b) 10 bis 60 Gew.-% polymerisierbares Bindemittel,
(c) 40 bis 85 Gew.-% Füllstoff und
(d) 0,01 bis 5 Gew.-% Additiv.

Zusammensetzungen zur Anwendung als dentale Beschichtungsmaterialien enthalten bevorzugt:
(a) 0,001 bis 5 Gew.-% Acylgermanium-Verbindung der allgemeinen Formel (I),
(b) 20 bis 99,9 Gew.-% polymerisierbares Bindemittel,
(c) 0 bis 20 Gew.-% nanopartikuläre Füllstoffe und
(d) 0,01 bis 2 Gew.-% Additiv,
(e) 0 bis 50 Gew.-% Lösungsmittel.

Zusammensetzungen zur Anwendung als Druckertinte enthalten bevorzugt:
(a) 0,001 bis 5 Gew.-% Acylgermanium-Verbindung der allgemeinen Formel (I),
(b) 30 bis 60 Gew.-% polymerisierbares Bindemittel,
(c) 1 bis 45 Gew.-% Farbmittel und
(d) 0,01 bis 30 Gew.-% Additiv.

Zusammensetzungen zur Anwendung als Lack, beispielsweise als Weißlack oder als Lack für optische Fasern, enthalten bevorzugt:
(a) 0,001 bis 5 Gew.-% Acylgermanium-Verbindung der allgemeinen Formel (I),
(b) 55 bis 99,5 Gew.-% polymerisierbares Bindemittel,
(c) 0,1 bis 50 Gew.-% Pigment und ggf.
(d) 0,01 bis 30 Gew.-% Additiv.

Ein bevorzugtes Pigment zur Herstellung von Lacken ist TiO₂.

Dentalwerkstoffe, die sich durch Thiol-en-Reaktion härten lassen, enthalten vorzugsweise eine Mischung aus einer oder mehreren Polythiolverbindungen und einer oder mehreren Polyvinylverbindungen, wobei eine oder mehrere dieser Verbindungen in oligomerer Form vorliegen können. Vorzugsweise sind 45 bis 55 % der funktionellen Gruppen dieser Mischungen Thiolgruppen, die übrigen Gruppen können Vinylgruppen sein. Die Mischungen können weiterhin einen oder mehrere Füllstoffe enthalten, wobei der Menge an polymerisierbaren Harzen vorzugsweise im Bereich von 10 bis 40 Gew.-% und die Füllstoffmenge vorzugsweise im Bereich von 60 bis 90 Gew.-% liegt. Geeignete Mischungen aus Polythiol- und Polyvinylverbindungen sowie geeignete füllstoffhaltige Mischungen werden in der WO 2005/086911 beschrieben. Die Menge an Initiator gemäß Formel (I) beträgt vorzugsweise 0,05 bis 0,5 Gew.-%.

Gegenstand der Erfindung ist auch die Verwendung von Acylgermanen der Formel (I) zur Herstellung von Adhäsiven, Beschichtungen, Lacken, Tinten, Zementen, Kompositen, Formteilen oder Dentalwerkstoffen sowie deren Verwendung als Initiator für die radikalische Polymerisation.

Außerdem betrifft die Erfindung ein Verfahren zur Herstellung von Formkörpern, insbesondere dentalen Kronen, Brücken, Inlays und künstlichen Zähnen, bei dem man eine erfindungsgemäße Zusammensetzung auf an sich bekannte Weise zu dem Formkörper formt und dann zumindest teilweise, vorzugsweise vollständig, härtet. Die Härtung erfolgt vorzugsweise durch radikalische Polymerisation.

Die erfindungsgemäßen Photoinitiatoren zeichnen sich besonders durch eine hohe Reaktivität und eine hohe Aktivität schon bei geringer Einsatzkonzentration aus. Dadurch kann einer äußerst rasche Aushärtung des Photopolymers im Vergleich zu bekannten Photoinitiatoren, die im sichtbaren Bereich absorbieren erreicht werden. Beispielsweise ergaben Messungen von Bisacyldiethylgermanium in einer Harzmischung aus Decandioldimethacrylat (D₃MA):UDMA:Bis-GMA = 1:1:1 eine beinahe doppelt so hohe Polymerisationsrate (Rp) wie Campherchinon in Kombination mit einem Aminbeschleuniger in der gleichen Formulierung. Die Aushärtungszeit konnte im Vergleich zu Campherchinon/Amin ebenfalls halbiert werden. Selbst bei 15-facher Verdünnung an Bisacyldiethylgermanium kann noch immer eine mit Campherchinon/Amin als Photoinitiator vergleichbare Reaktivität erreicht werden (siehe Ausführungsbeispiele, Tabellen 7, 8, 9, Summe aus Initiator und Beschleuniger).

Außerdem weisen die naturgemäß gelblich gefärbten Photoinitiatoren gemäß Formel (I) einen ausgezeichneten Photobleachingeffekt auf, d.h. die Verbindungen der Formel (I) werden bei der Härtung entfärbt und dadurch Verfärbungen des Materials nach dem Aushärten vermieden (siehe Ausführungsbeispiele, Tabelle 2).

Die Erfindung wird im Folgenden anhand von Beispielen näher erläutert.

### Ausführungsbeispiele

### Beispiel 1: Synthese von Benzoyltrimethylgermanium

In einem trockenen 50 ml Dreihalskolben mit Rückflusskühler und Septum wurden unter Argon 1.64 g (4.49 mmol) Allylpalladium(II)chlorid Dimer, 1.49 g (8.97 mmol) Triethylphosphit und 23.24 g (98.7 mmol) Hexamethyldigermanium vorgelegt, und 5 min bei Raumtemperatur gerührt. Anschließend wurde 12.62 g (89.7 mmol) frisch destilliertes Benzoylchlorid zugetropft. Nach 4h Rühren bei 110°C, wurde der Pd-Katalysator vom Reaktionsgemisch abgetrennt und flüchtige Reaktionsprodukte sowie der Überschuß an Hexamethyldigermanium am Rotationsverdampfer abgezogen. Das Reaktionsgemisch wurde säulenchromatographisch (Petrolether (PE) : Ethylacetat (EE) = 40:1) aufgetrennt. Es ergaben sich 7.8 g (78 % d. Th.) an Benzoyltrimethylgermanium als gelbe Flüssigkeit. DC (Petrolether : Ethylacetat = 20:1): R_{f}=0.58.

UV-VIS: λₘₐₓ: 411,5 nm, ε = 1374 dm²/mol

¹H-NMR (200 MHz; CDCl₃): δ (ppm): 7.78-7.82 (m, 2H, Ar-H^{2,6}), 7.48-7.58 (m, 3H, Ar-H^{3,4,5}), 0.51 (s, 9H, -CH₃).

¹³C-NMR (200 MHz; CDCl₃): δ (ppm) : 234.39 (-C=O), 140.61 (Ar-C¹), 132. 90 (Ar-C⁴), 128.75 (Ar-C^{2,6}), 127.71 (Ar-C^{3,5}), -1.14 (-CH₃).

IR (cm⁻¹): 2979, 2916 , 1628 (C=O), 1582, 1448, 1310, 1239, 1207, 1172, 905, 827, 770, 732.

### Beispiel 2: Synthese von Diethylbis(2-phenyl-1,3-dithian-2-yl)germanium

In einem trockenen 50 ml Dreihalskolben wurden unter Argon 1.85 g (9.42 mmol) 2-Phenyl-1,3-dithian vorgelegt, und in 28 ml wasserfreiem THF gelöst. Bei 0 °C wurden 3.99 ml 2.36 M BuLi-Lösung in Hexan zugetropft und die Reaktionslösung 2 h bei 0 °C gerührt. 0.83 mg (3.93 mmol) Diethyldichlorgermanium gelöst in 8 ml wasserfreiem THF wurden langsam bei 0°C zum Reaktionsgemisch getropft und anschließend weitere 2 h bei O °C gerührt. Zur Vervollständigung der Reaktion wurde eine weitere Lösung von 2-Phenyl-2-lithium-1,3-dithian (2.36 mmol) wie zuvor beschrieben hergestellt und bei 0 °C zur Reaktionslösung getropft, welche anschließend 18 h bei 6°C gerührt wurde. Die Reaktion wurde durch Zugabe von 20 ml Wasser gequencht und das Rohprodukt mit Diethylether (3 x 30 ml) extrahiert. Die vereinigten organischen Phasen wurden mit Na₂SO₄ getrocknet und das Lösungsmittel am Rotationsverdampfer abgezogen. Der Rückstand wurde säulenchromatographisch aufgetrennt (Petrolether : Ethylacetat = 20:1). Es ergaben sich 1.42 g (70% d. Th.) an Diethylbis(2-phenyl-1,3-dithian-2-yl)germanium als farbloser Feststoff. DC (Petrolether : Ethylacetat = 20:1): R_{f}=0.51

Schmelzpunkt: 112-115 °C

¹H-NMR (200 MHz; CDCl₃): δ (ppm): 7.82-7.86 (m, 4H, Ar-H^{2,6}), 7.01-7.24 (m, 6H, Ar-H^{3,4,5}), 2.55 - 2.69 (m, 4H, S-CH₂-), 2.12 - 2.23 (m, 4H, S-CH₂-), 1.63 - 2.01 (m, 4H -CH₂-), 1.19 (m, 4H, Ge-CH2-), 1.02 (m, 6H -CH₃).

¹³C-NMR (200 MHz; CDCl₃): δ (ppm): 140.58 (Ar-C¹), 130.38 (Ar-C⁴), 128.18 (Ar-C^{2,6}), 125.53 (Ar-C^{3,5}), 51.90 (Ge-C-S), 25.88 (S-CH₂-), 25.16 (-CH₂-), 10.26 (Ge-CH₂-), 4.74 (-CH₃).

### Beispiel 3: Synthese von Bisbenzoyldiethylgermanium

In einem 25 ml Rundkolben wurden 1.12 g (2.24 mmol) Diethylbis(2-phenyl-1,3-dithian-2-yl)germanium vorgelegt, und in 15 ml wässrigem THF (THF:Wasser 4:1) gelöst. Nach der Zugabe von 3.53 g (35.01 mmol) CaCO₃ wurde die Suspension 5 min bei Raumtemperatur gerührt. Unter Lichtschutz wurden 6.83 g (26.93 mmol) Iod portionsweise zugegeben. Nach 3 h Rühren bei Raumtemperatur wurde das Reaktionsgemisch mit 15 ml Diethylether verdünnt, und überschüssiges Iod durch Zugabe von 20 ml einer gesättigten Na₂S₂O₄-Lösung unter starkem Rühren zersetzt. Die dabei entstandenen Salze wurden durch Filtration über Hyflo von der Reaktionslösung abgetrennt, und mit Diethylether (3 x 15 ml) gewaschen. Die vereinten organischen Phasen wurden mit Na₂SO₄ getrocknet, abfiltriert, und das Lösungsmittel am Rotationsverdampfer abgezogen. Der Rückstand wurde säulenchromatographisch (Petrolether : Ethylacetat 20:1) aufgetrennt. Es ergaben sich 0.46 g (60 % d. Th.) an Bisbenzoyldiethylgermanium als gelber Feststoff. DC (Petrolether : Ethylacetat = 20:1): R_{f}=0.42

UV-VIS: λₘₐₓ: 418,5 nm, ε = 4880 dm²/mol

¹H-NMR (200 MHz; CDCl₃): δ (ppm) : 7.70-7.75 (m, 2H, Ar-H^{2,6}), 7.37-7.50 (m, 3H, Ar-H^{3,4,5}), 1.50 (d, 4H, -CH₂), 1.11 (t, 6H, - CH₃).

¹³C-NMR (200 MHz; CDCl₃): δ (ppm): 230.22 (-C=O), 141.23 (Ar-C¹), 133.66 (Ar-C⁴), 129.06 (Ar-C^{2,6}), 128.720 (Ar-C^{3,5}), 9.11 (-CH₂), 6.61 (-CH₃).

IR (cm⁻¹): 2959, 2911 , 1622 (C=O), 1579, 1447, 1308, 1207, 1169, 1022, 892, 767, 688

Im Vergleich zu langwellig absorbierenden Norrish-Typ-I-Photoinitiatoren (= Photoinitiatoren, deren monomolekulare Photolyse direkt polymerisationsauslösende Radikale liefert), wie z.B. das kommerzielle Bisacylphosphinoxid Irgacure 819 (Bis(2,4,6-trimethylbenzoyl)-phenyl-phosphinoxid) mit dem langwelligsten Absorptionsmaxima bei 397 nm, liegt das Maximum des Norrish-Typ-I-Photoinitiators Benzoyltrimethylgermanium mit 411,5 nm bzw. das Maximum von Bisbenzoyldiethylgermanium mit 418.5 nm deutlicher bathochromer, was die Durchhärtungstiefe der Photopolyreaktionsprodukte signifikant verbessert. Einzig die Gruppe der spaltenden Titanocene haben ein Maximum bei etwa 480 nm, allerdings weisen diese bekannterweise (K. Dietliker; Photoinitiators for Free Radical, Cationic and Anionic Photopolymerization 2nd Ed. Sita Technology Ld, London UK p.228-239) keine ausreichenden Photobleachingeffekt auf, was zu orange gefärbten Polymeren führt.

Im Vergleich zum im Dentalbereich weit verbreitet verwendeten Norrish-Typ-II-Photoinitiator Campherchinon (λₘₐₓ: 468 nm), der zur effizienten Radikalbildung ein zusätzliches Reduktionsmittel benötigt, liegt das Absorptionsmaximum von Benzoyltrimethylgermanium deutlich kurzwelliger und zeigt ein sehr gutes Ausbleichen (Photobleaching) beim Bestrahlen.

### Beispiel 4: Herstellung eines Kompositzements unter Verwendung des Benzoyltrimethylgermaniums aus Beispiel 1

Entsprechend der nachfolgend aufgeführten Tabelle 1 wurden Kompositbefestigungszemente auf der Basis einer Methacrylatmischung und unter Einbeziehung entweder unterschiedlicher Konzentrationen des Benzoyltrimethylgermaniums aus Beispiel 1 (Zement A bis C) oder einer Mischung aus Campherchinon und p-N,N-Dimethylaminobenzoesäureethylester (Zement D, Vergleich) mittels eines Walzenstuhles (Modell "Exakt", Exakt Apparatebau, Norderstedt) hergestellt. Dabei enthielten die Zemente B bzw. D die gleiche molare Konzentration an Photoinitiator, d.h. an Benzoyltrimethylgermanium (Zement B) bzw. Campherchinon (Zement D) Von den Materialien wurden Prüfkörper präpariert, die 2 mal 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit, des Biege-E-Moduls und der Exothermzeit. Zusätzlich wurde die Gelbfärbung der nichtausgehärteten Pasten und sowie der ausgehärteten Zemente entsprechend der DIN-Norm 5033 "Farbmessung" unter Verwendung des L*a*b*-Farbmesssystems Minolta CR-300 anhand des b*-Wertes charakterisiert, wobei weiterhin ein b*-Wert von -2,7 für Zementpasten-Rezeptur ohne Initiatorkomponenten gemessen wurde. Die Ergebnisse sind in Tabelle 2 gezeigt.

**Tabelle 1: Zusammensetzung der Kompositzemente (Angaben in Gew.-%)**

| **Komponente** | **Zement A** | **Zement B** | **Zement C** | **Zement D²⁾** |
|---|---|---|---|---|
| Benzoyltrimethylgermanium | 0,10 | 0,32 | 0,50 | - |
| Campherchinon | - | - | - | 0,24 |
| p-N,N-Dimethylaminobenzoesäureethylester | - | - | - | 0,23 |
| UDMA¹⁾ | 32,11 | 31,89 | 31,71 | 31,80 |
| Triethylenglycoldimethacrylat | 7,81 | 7,81 | 7,81 | 7,81 |
| Aerosil OX-50 (Degussa) | 41,27 | 41,27 | 41,28 | 41,23 |
| Ytterbiumtrifluorid (Rhone-Poulenc) | 18,71 | 18,71 | 18,70 | 18,69 |

| | | | | |
|---|---|---|---|---|
| ¹⁾ Additionsprodukt aus 2 mol 2-Hydroxyethylmethacrylat und 1 mol 2,2,4-Trimethylhexamethylendiisocyanat ²⁾ Vergleich | | | | |

**Tabelle 2: Eigenschaften der Kompositzemente**

| **Komponente** | **Zement A** | **Zement B** | **Zement C** | **Zement D²⁾** |
|---|---|---|---|---|
| Exothermzeit (s) | 13 | 12 | 11 | 8 |
| Biegefestigkeit (MPa) nach 24 h WL¹⁾ | 102 | 116 | 129 | 118 |
| E-Modul (MPa) nach 24 h WL¹⁾ | 3230 | 5240 | 5560 | 5580 |
| b*-Wert Paste vor Aushärtung | 7,7 | 16,0 | 19,6 | 27,4 |
| b*-Wert Zement nach Aushärtung | -5,8 | -1,9 | 0,7 | 4,5 |

| | | | | |
|---|---|---|---|---|
| ¹⁾ WL = Wasserlagerung der Prüfkörper bei 37 °C ²⁾ Vergleich | | | | |

Aus Tabelle 2 ist ersichtlich, daß die auf Benzoyltrimethylgermanium basierende Zemente mit zunehmender Photoinitiatorkonzentration eine kürzere Exothermzeit und damit eine schnellere Aushärtung ergeben und im Vergleich zum Zement D (konventionelle Photoinitiatormischung auf der Basis einer Mischung aus Campherchinon und p-N,N-Dimethylaminobenzoesäureethylester) ab einer Konzentration von 0,32 Gew.-% Benzoyltrimethylgermanium (Zement B) Materialien mit vergleichbaren mechanischen Eigenschaften erhalten werden. Überraschenderweise wurde gefunden, daß die ausgehärteten Zemente auf der Basis von Benzoyltrimethylgermanium negative oder nur kleine positive b*-Werte und damit keine Gelbverfärbung zeigen, während sich für den ausgehärteten Zement auf der Basis von Campherchinon ein b*-Wert von 4,5 ergab, was einer deutlichen Gelbverfärbung entspricht.

### Beispiel 5: Herstellung eines Füllungskomposits unter Verwendung des Benzoyltrimethylgermaniums aus Bsp. 1

Entsprechend der nachfolgend aufgeführten Tabelle 3 wurde ein Füllungskomposit auf der Basis einer Methacrylatmischung und unter Einbeziehung entweder unterschiedlicher Konzentrationen des Benzoyltrimethylgermaniums aus Beispiel 1 (Komposit E) oder einer Mischung aus Campherchinon und p-N,N-Dimethylaminobenzoesäureethylester (Komposit F, Vergleich) mittels eines Kneters (Typ LPM 0.1 SP, Linden, Marienheide) hergestellt. Von den Materialien wurden analog Beispiel 4 Prüfkörper hergestellt und ausgehärtet. Nach der ISO-Norm ISO-4049 erfolgte die Bestimmung der Biegefestigkeit, des Biege-E-Moduls und des Polymeriationsschrumpfes. Die Ergebnisse sind in Tabelle 4 gezeigt.

**Tabelle 3: Zusammensetzung der Füllungskomposite (Angaben in Gew.-%)**

| **Komponente** | **Komposit E** | **Komposit F⁵⁾** |
|---|---|---|
| Monomerharz¹⁾ | 18,06 | 17,99 |
| Benzoyltrimethylgermanium | 0,08 | - |
| Campherchinon | - | 0,05 |
| p-N,N-Dimethylaminobenzoesäureethylester | - | 0,09 |
| Glasfüller GM27884 (Schott)²⁾ | 51,6 | 51,61 |
| Sphärosil (Tokoyama Soda)³⁾ | 14,37 | 14,36 |
| Ytterbiumtrifluorid (Rhone-Poulenc) | 14,89 | 14,89 |
| OX-50⁴⁾ | 0,2 | 0,2 |

| | | |
|---|---|---|
| ¹⁾ Mischung aus 42.4 Gew.-% Bis-GMA, 37,4 Gew.-% UDMA und 20,2 Gew.-% Triethylenglycoldimethacrylat ²⁾ Silanisierter Ba-Al-Borosilikatglasfüller mit einer mittleren Partikelgröße von 1,5 µm, ³⁾ SiO₂-ZrO₂-Mischoxid, mittl. Primärpartikelgröße: 250 nm ⁴⁾ Silanisiertes pyrogenes SiO₂ OX-50 (Degussa) ⁵⁾ Vergleich | | |

**Tabelle 4: Eigenschaften der Füllungskomposite**

| **Materialeigenschaft** | **Komposit E** | **Komposit F²⁾** |
|---|---|---|
| Exothermzeit (s) | 10 | 9 |
| Biegefestigkeit (MPa) nach 24 h WL¹⁾ | 150 | 168 |
| Biege-E-Modul (GPa) nach 24 h WL¹⁾ | 10540 | 12190 |

| | | |
|---|---|---|
| ¹⁾ WL = Wasserlagerung der Prüfkörper bei 37 °C ²⁾ Vergleich | | |

### Beispiel 6: Herstellung eines stark aciden Kompositzementes unter Verwendung des Benzoyltrimethylgermaniums aus Beispiel 1

Entsprechend der nachfolgend aufgeführten Tabelle 5 wurden Kompositbefestigungszemente auf der Basis einer Mischung von zwei Dimethacrylaten mit der aciden Phosphonsäure MA-154 (2-[2-Dihydroxyphosphoryl)-ethoxymethyl]-acrylsäureethylester und unter Einbeziehung entweder des Benzoyltrimethylgermaniums aus Beispiel 1 (Zement G) oder einer Mischung aus Campherchinon und p-N,N-Dimethylaminobenzoesäureethylester (Zement H, Vergleich) mittels eines Walzenstuhles (Modell "Exakt", Exakt Apparatebau, Norderstedt) hergestellt. Von den Materialien wurden analog Beispiel 2 Prüfkörper präpariert, ausgehärtet und die Biegefestigkeit der E-Moduls bestimmt (Tabelle 6).

Aus Tabelle 6 ist ersichtlich, daß der auf Benzoyltrimethylgermanium basierende Zement G im Vergleich zum Zement H (konventionelle Photoinitiatormischung einer Mischung aus Campherchinon und p-N,N-Dimethylaminobenzoesäureethylester) auch in Gegenwart von stark aciden Monomeren zu Materialien mit vergleichbaren mechanischen Eigenschaften führt.

**Tabelle 5: Zusammensetzung der aciden Kompositzemente (Angaben in Gew.-%)**

| **Komponente** | **Zement G** | **Zement H²⁾** |
|---|---|---|
| Benzoyltrimethylgermanium | 0,33 | - |
| Campherchinon | - | 0,24 |
| p-N,N-Dimethylaminobenzoesäureethylester | - | 0,23 |
| UDMA¹⁾ | 21,88 | 21,82 |
| Triethylenglycoldimethacrylat | 7,81 | 7,81 |
| Phosphonsäure MA-154 | 10,01 | 9,99 |
| Aerosil OX-50 (Degussa) | 41,27 | 41,22 |
| Ytterbiumtrifluorid (Rhone-Poulenc) | 18,70 | 18,69 |

| | | |
|---|---|---|
| ¹⁾ Additionsprodukt aus 2 mol 2-Hydroxyethylmethacrylat und 1 mol 2,2,4-Trimethylhexamethylendiisocyanat ²⁾ Vergleich | | |

**Tabelle 6: Eigenschaften der Kompositzemente**

| **Komponente** | **Zement G** | **Zement H²⁾** |
|---|---|---|
| Biegefestigkeit (MPa) nach 24 h WL¹⁾ | 118 | 120 |
| E-Modul (MPa) nach 24 h WL¹⁾ | 5380 | 5690 |

| | | |
|---|---|---|
| ¹⁾ WL = Wasserlagerung der Prüfkörper bei 37 °C ²⁾ Vergleich | | |

### Beispiel 7: Vergleich der Aktivität der Acylgermane mit bekannten Photoinitiatoren, die im sichtbaren Bereich absorbieren.

Die Aktivität der Photoinitiatoren wurde mittels Photo-DSC (Differential Scanning Calorimetry) Messungen an einem DSC-50 Gerät der Fa. Shimadzu gemessen, wobei die Proben wahlweise mit verschiedenen Dentallampen bestrahlt wurden (Astalis 3: Halogenlampe, Wellenlängenbereich 400-500 nm, Intensität 530 mW/cm²; Bluephase C8: LED, Wellenlängenbereich 430-490 nm, Intensität 1100 mW/cm²; Ivoclar Vivadent AG). Gekennzeichnet wird die Aktivität durch den Zeitpunkt des Peakmaximums (tₘₐₓ), die Polymerisationsrate (R_{P}), welche der Peakhöhe entspricht, und den Doppelbindungsumsatz (DBC). Die jeweiligen Photoinitiatoren wurden in einer Harzmischung aus D₃MA:UDMA:Bis-GMA = 1:1:1 gelöst, und anschließend in einem Aluminiumtiegel mittels DSC gemessen.

Die Tabellen 7 und 8 zeigen Photo-DSC-Daten von CQ (Campherquinon / Dimethylaminobenzoesäureethylester), Irg 819 (Bis(2,4,6-trimethylbenzoyl)phenylphosphinoxid), Benzoyltrimethylgermanium (Mono-AG) und Bisbenzoyldiethylgermanium (Bis-AG) bei einer Konzentration von 0,022 mmol PI (Photoinitiator) pro Gramm Harz.

Tabelle 9 zeigt Photo-DSC-Daten von Irg 819 (Bis(2,4,6-trimethylbenzoyl)phenylphosphinoxid), Benzoyltrimethylgermanium (Mono-AG) und Bisbenzoyldiethylgermanium (Bis-AG) bei unterschiedlichen PI Konzentrationen.

**Tabelle 7: Bestrahlung mit einer Astralis 3-Lampe (400-500 nm)**

| **PI** | **tₘₐₓ [s]** | **Rₚx10⁻³ [mol/(lxs)]** | **DBC [%]** |
|---|---|---|---|
| CQ | 14 | 54.9 | 59 |
| Irg 819 | 11 | 67.5 | 59 |
| Mono-AG | 16 | 51.0 | 56 |
| Bis-AG | 7.8 | 99.2 | 84 |

**Tabelle 8: Bestrahlung mit Bluephase C8-Lampe (430-490 nm)**

| **PI** | **tₘₐₓ [s]** | **Rₚx10⁻³ [mol/(lxs)]** | **DBC [%]** |
|---|---|---|---|
| CQ | 13 | 59.9 | 61 |
| Irg 819 | 17 | 43.5 | 45 |
| Mono-AG | 16 | 48.3 | 49 |
| Bis-AG | 7.8 | 98.5 | 72 |

**Tabelle 9: Aktivitätsvergleich unterschiedlicher Initiatoren**

| **Konzentration [mmol/g]** | **PI** | **tₘₐₓ [s]** | **Rₚx10⁻³ [mol/(lxs)]** | **DBC [%]** |
|---|---|---|---|---|
| 0.0055 | Irg 819 | 14 | 48.3 | 48 |
| | Mono-AG | 24 | 36.2 | 48 |
| | Bis-AG | 9 | 85.6 | 66 |
| 0.0014 | Irg 819 | 23 | 30.4 | 42 |
| | Mono-AG | 52 | 16.4 | 30 |
| | Bis-AG | 12 | 61.0 | 56 |

### Beispiel 8: Synthese von 1,1-Bis(2-phenyl-1,3-dithian-2-yl)-germinan

Unter Inertgasatmoshäre wurden 48.30 g (0.246 mol) 2-Phenyl-1,3-dithian in 300 ml absolutem Tetrahydrofuran gelöst und bei 0°C mit 98.4 ml (0.246 mol) n-Butyllithium (in Hexan ~ 2.5 moll⁻¹ ) versetzt. Man ließ 2 Std. bei 0°C rühren und tropfte eine Lösung von 24.3 g (0.114 mol) 1,1-Dichlorgerminan (Gehalt ~70-90%;) in 50 ml absolutem Tetrahydrofuran hinzu. Zur Vervollständigung der Reaktion wurde erst weitere 2 Std. bei 0°C gerührt und dann über Nacht bei 4°C stehen gelassen. Es wurde mit 30 ml Wasser gequencht (starke Aufhellung bis schwachgelbliche Lösung), die organische Phase mit 250 ml Ethylacetat verdünnt und mit je 2 x 50 ml Wasser gewaschen. Die vereinigten wässrigen Phasen reextrahierte man mit je 2 x 100 ml Ethylacetat. Man wusch die vereinigten organischen Phasen mit gesättigter Kochsalzlösung, trocknete über Natriumsulfat und engte ein. Nach Trocknen im Feinvakuum erhielt man 51 g eines hellgelblichen schmierigen Feststoffs, der 1 Std. mit 30 ml Ethylacetat verrührt wurde. Der Niederschlag wurde abgesaugt und bei 50 °C im Vakuum getrocknet. Man erhielt 27.35 g (45%) eines weissen Feststoffes vom Schmp.: 159-160°C.

¹H-NMR (400 MHz; CDCl₃): 1.19 - 1.23 (m, 2H, CH₂-C₂H₄-Ge), 1.36 - 1.42 (m, 4H, CH₂-CH₂-Ge), 1 .48 - 1.55 (m, 4H, CH₂-Ge), 1 .76 - 1.82 und 1.93 - 2.03 (m, 2 x 2H, CH₂CH₂S), 2.27 - 2.32 und 2.70 - 2.77 (m, 2 x 4H, CH₂S), 7.12 - 7.17 (m, 2 H, Ar-H⁴), 7.25 - 7.31 (m, 4 H, Ar-H^{2,6}), 7.90 - 7.93 (m, 4 H, Ar-H^{3,5})

¹³C-NMR (100 MHz; CDCl₃): 11.45 (CH₂-Ge), 25.4, 26.0, 26.2, 26.6, und 28.5 (CH₂CH₂), 51.2 (S-C-S), 128.2, 129.0 und 133.6 (Ar-C²⁻⁶), 140.7 (Ar-C¹)

### Beispiel 9: Synthese von 1,1-Bisbenzoyl-germinan

In einem trockenen 500 ml Rundkolben wurden unter Gelblicht (gesamte Reaktion inklusive Reinigung) 16.91 g (31.7 mmol) 1,1-Bis(2-phenyl-1,3-dithian-2-yl)-germinan in 300 ml Tetrahydrofuran und 50 ml Wasser gelöst. Nach der Zugabe von 16.68 g (0.167 mol) Calciumcarbonat wurde die Suspension 5 Min. bei Raumtemperatur gerührt und dann mit 32.18 g (0.127 mol) Iod versetzt, wobei das Reaktionsgemisch leicht aufschäumte und die Temperatur auf ca. 30 °C anstieg. Man ließ weiter rühren und gab nach 1 bzw. 2 Std. nochmals je 16.68 g (0.167 mol) Calciumcarbonat und (im Abstand von 5 Min.) 32.18 g (0.127 mol) Iod dazu. Wie beschrieben wurden nach 6 Std. sowie 24 Std. Rührzeit weitere je 15 g (0.15 mol) Calciumcarbonat und 30 g (0.118 mol) Iod zugegeben. Nach insgesamt 28 Std. wurde das rot-braune Reaktionsgemisch vorsichtig bis zum Farbumschlag nach gelb mit 280 ml gesättigter wässriger Natriumdithionit-Lösung versetzt (starkes Schäumen). Das Gemisch wurde über eine Glasfritte filtriert und der Filtrationsrückstand mit 6 x 100 ml Ethylacetat gewaschen. Die organische Phase wurde mit 2 x 100 ml Wasser gewaschen und die vereinigten Wasserphasen mit je 2 x 100 ml Ethylacetat reextrahiert. Die vereinigten organischen Phasen wurden über Natriumsulfat getrocknet, filtriert und am Rotationsverdampfer eingeengt. Der Rückstand wurde mit 100 ml Dichlormethan versetzt und über eine mit Kieselgel gefüllte Fritte filtriert (Kieselgel 60, 0.035-0.070 mm, 40 g, ∅ 50 mm; ca. 600 ml Dichlormethan). Das Eluat wurde am Rotationsverdampfer eingeengt und am Feinvakuum getrocknet. Man erhielt 10.95 g (31.0 mmol; 98%) eines wachsartigen gelben Rohprodukts, dass säulenchromatographisch (Kieselgel 60, 0.035-0.070 mm, *n*-Hexan/Ethylacetat 20:1; R_{f}=0.3) gereinigt wurde. Man erhielt 7.33 g (20.8 mmol; 66%) eines gelben Feststoffs vom Schmelzpunkt 85 - 88 °C.

¹H-NMR (400 MHz; CDCl₃) : 1 .41 - 1.47 (m, 2H, CH₂-C₂H₄-Ge), 1.60 - 1.63 (m, 4H, CH₂-CH₂-Ge), 1.78 - 1.84 (m, 4H, CH₂-Ge), 7.40 - 7.51 (m, 6 H, Ar-H^{3,4,5}), 7 . 7 5 - 7.78 m, 4 H, Ar-H^{2,6})

¹³C-NMR (100 MHz; CDCl₃): 14.4 (CH₂-Ge), 25.6 und 29.1 (CH₂CH₂), 128.2, 129.0 und 133.6 (Ar-C²⁻⁶), 140.7 (Ar-C¹), 229.0 (C=O)

Das Absoptionsmaximum des Norrish-Typ-I-Photoinitiators 1,1-Bisbenzoyl-germinan liegt bei 418,1 nm.

### Beispiel 10: Herstellung eines Kompositzements unter Verwendung des Bisbenzoyldiethylgermaniums aus Beispiel 3 bzw. von 1,1-Bisbenzoyl-germinan aus Beispiel 9

Entsprechend der nachfolgend aufgeführten Tabelle 10 wurden analog Beispiel 4 Kompositbefestigungszemente auf der Basis einer Methacrylatmischung und unter Einbeziehung entweder zweier unterschiedlicher Konzentrationen des Bisbenzoyldiethylgermaniums aus Beispiel 3 (Zement A bis B) oder von 1,1-Bisbenzoylgerminan aus Beispiel 9 (Zemente C und D) hergestellt. Dabei enthielten die Zemente A und C bzw. B und D die gleiche molare Konzentration an Photoinitiator. Von den Materialien wurden analog Beispiel 6 Prüfkörper präpariert, ausgehärtet und die Biegefestigkeit und der Biege-E-Modul bestimmt. Die Ergebnisse sind in Tabelle 11 gezeigt.

**Tabelle 10: Zusammensetzung der Kompositzemente (Angaben in Gew.-%)**

| **Komponente** | **Zement A** | **Zement B** | **Zement C** | **Zement D** |
|---|---|---|---|---|
| Bisbenzoyldiethylgermanium | 0,16 | 0,49 | - | - |
| 1,1-Bisbenzoyl-germinan | - | - | 0,21 | 0,64 |
| UDMA¹⁾ | 32,06 | 31,72 | 32,01 | 31,56 |
| Triethylenglycoldimethacrylat | 7,80 | 7,82 | 7,81 | 7,81 |
| Aerosil OX-50 (Degussa) | 41,27 | 41,27 | 41,26 | 41,27 |
| Ytterbiumtrifluorid (Rhone-Poulenc) | 18,71 | 18,70 | 18,71 | 18,72 |

| | | | | |
|---|---|---|---|---|
| ¹⁾ Additionsprodukt aus 2 mol 2-Hydroxyethylmethacrylat und 1 mol 2,2,4-Trimethylhexamethylendiisocyanat | | | | |

**Tabelle 11: Eigenschaften der Kompositzemente**

| **Komponente** | **Zement A** | **Zement B** | **Zement C** | **Zement D** |
|---|---|---|---|---|
| Biegefestigkeit (MPa) nach 24 h WL¹⁾ | 119 | 143 | 124 | 133 |
| E-Modul (MPa) nach 24 h WL¹⁾ | 5615 | 6345 | 5439 | 6101 |

| | | | | |
|---|---|---|---|---|
| ¹⁾ WL = Wasserlagerung der Prüfkörper bei 37 °C | | | | |

### Beispiel 11: Herstellung eines kationisch polymerisierbaren Komposites unter Verwendung von Bisbenzoyldiethylgermaniums aus Beispiel 3

Entsprechend der nachfolgend aufgeführten Tabelle 12 wurde analog Beispiel 5 ein Komposit auf der Basis des kommerziellen cycloaliphatischen Oligo(dimethylsiloxan)-Diepoxides Silbione UV Polymer 30 und einer Mischung des kationischen Photoinitiators Rhodorsil 2074 (Isopropylphenyl-methylphenyl-iodoniumtetrakis(pentafluorophenylborat, Rhodia) und von Bisbenzoyldiethylgermaniums aus Beispiel 3 hergestellt. Von den Materialien wurden analog Beispiel 5 Prüfkörper präpariert ausgehärtet und die Biegefestigkeit und der Biege-E-Modul bestimmt. Die Ergebnisse sind in Tabelle 13 gezeigt.

**Tabelle 12: Zusammensetzung des kationischen Füllungskomposites (Angaben in Gew.-%)**

| **Komponente** | **Komposit** |
|---|---|
| Monomerharz¹⁾ | 25,4 |
| Glasfüller G018-163 (Schott)²⁾ | 63,9 |
| Ytterbiumtrifluorid (Rhone-Poulenc) | 10,4 |
| OX-50³⁾ | 0,3 |

| | |
|---|---|
| ¹⁾ Mischung aus 96,8 Gew.-% Silbione UV Polymer 30, 3,0 Gew.-% Rhodorsil 2074 und 0,2 Gew.-% Bisbenzoyldiethylgermanium ²⁾ Silanisierter Sr-Al-Borosilikatglasfüller mit einer mittleren Partikelgröße von 1,0 µm, ³⁾ Silanisiertes pyrogenes SiO₂ OX-50 (Degussa) | |

**Tabelle 13: Eigenschaften des kationischen Komposites**

| | **Kationisches Komposit** |
|---|---|
| Biegefestigkeit (MPa) nach 24 h WL¹⁾ | 90 |
| E-Modul (MPa) nach 24 h WL¹⁾ | 8700 |

| | |
|---|---|
| ¹⁾ WL = Wasserlagerung der Prüfkörper bei 37 °C | |

### Beispiel 12: Herstellung einer Beschichtung unter Verwendung von Bisbenzoyldiethylgermaniums aus Beispiel 3

Entsprechend der nachfolgend aufgeführten Tabelle 14 wurden ein Beschichtungsmaterialien auf der Basis einer Mischung aus Methylmethacrylat (MMA), Pentaerythritoltetraacrylat (SR-295) Methacrylatmischung und des Triacrylates V-546 unter Verwendung von Bisbenzoyldiethylgermaniums aus Beispiel 3 (Beschichtung A) oder einer Mischung aus Campferchinon (CC) und 4-(N,N-Dimethylamino)benzoesäurediethylester (EMBO) (Vergleich Beschichtung B) hergestellt.

**Tabelle 14: Zusammensetzung von zwei Beschichtungsmaterialien (Angaben in Gew.-%)**

| **Komponente** | **Beschichtung A** | **Beschichtung B** |
|---|---|---|
| MMA | 27,8 | 28,1 |
| SR-295 | 20,833 | 20,0 |
| V-546¹⁾ | 51,133 | 50, 0 |
| Bisbenzoyldiethylgermanium | 0.034 | - |
| CC | - | 0,35 |
| EMBO | - | 0,55 |

| | | |
|---|---|---|
| ¹⁾ HPA-Addukt von 3 mol Hydroxypropylacrylat an 1 mol des asymmetrischen Trimers von Hexamethylendiisocyanat | | |

Die Beschichtungsmaterialien wurden jeweils mit einem Microbrush auf einen Kompositprüfkörper (Systemp. C&B plus, Ivoclar Vivadent AG) aufgetragen und 20 s mit einer Halogenlampe Astralis 7 (Ivoclar Vivadent AG) oder LED Bluephase (Ivoclar Vivadent AG) bestrahlt. Mit der Beschichtung A ergab sich eine farblos nichtklebrige Oberfläche, während mit der Vergleichsbeschichtung die Oberfläche leicht gelblich und klebrig war.

## Patentansprüche

1. Zusammensetzung mit mindestens einem polymerisierbaren Bindemittel und einem Polymerisationsinitiator, **dadurch gekennzeichnet, daß** sie mindestens eine Acylgermanium-Verbindung gemäß der allgemeinen Formel (I) enthält, 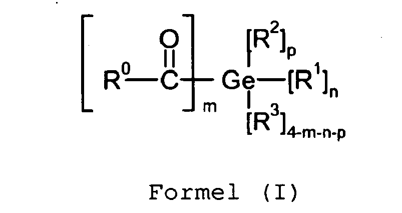 in der
R⁰ C₁₋₁₈-Alkyl, C₂₋₁₈-Alkenyl, wobei diese Reste unsubstituiert oder einfach oder mehrfach durch Halogen substituiert sein können, -OR¹⁰, -OCO-R¹⁰,-OCO-Hal, -COO-R¹⁰, -CH=CH-CO-OR¹⁰, -N(R¹¹)-CO-R¹⁰,-N(R¹¹)-CO-Hal, -C(C₁₋₄-Alkyl)=C(C₁₋₄-Alkyl)-CO-OR¹⁰, -CO-NR¹¹R¹², -CH=CH-Phenyl, -C(C₁₋₄-Alkyl)=C(C₁₋₄-Alkyl)-Phenyl, C₃₋₁₂-Cycloalkyl, C₂₋₁₈-Alkenyl, Phenyl-C₁₋₄-Alkyl, Phenyl, Naphthyl, Antryl, Biphenyl, ein gesättigter oder ungesättigter 5-oder 6-gliedriger O-, S- oder N-haltiger heterocyclischer Ring, wobei alle genannten Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder 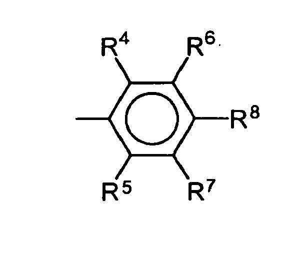 ist, wobei
R¹⁰ H, C₁₋₁₈-Alkyl, C₁₋₁₈-Alkyl, das durch ein oder mehrere Sauerstoffatome unterbrochen ist, C₂₋₁₈-Alkenyl, C₂₋₁₈-Alkenyl, das durch ein oder mehrere Sauerstoffatome unterbrochen ist, C₃₋₁₂-Cycloalkyl, Tetrahydropyran-2-yl, Phenyl-C₁₋₂₀-Alkylen, Phenyl-C₁₋₂₀-Alkenylen, C₁₋₆-Alkyl, das unsubstituiert oder durch Halogen, Cyclohexyl, Cyclopentyl, Tetrahydrofuranyl, Furanyl oder Isopropyl-4-methyl-cyclohexyl substituiert sein kann, Phenyl, Naphthyl oder Biphenyl ist, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können,
R¹¹, R¹² unabhängig voneinander H, C₁₋₁₈-Alkyl, C₁₋₁₈-Alkyl, das durch ein oder mehrere Sauerstoffatome unterbrochen ist, C₂₋₁₈-Alkenyl, C₂₋₁₈-Alkenyl, das durch ein oder mehrere Sauerstoffatome unterbrochen ist, C₃₋₁₂-Cycloalkyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder R¹¹ und R¹² bilden zusammen einen 5- oder 6-gliedrigen O-, S- oder N-haltigen heterocyclischen Ring, der seinerseits mit einem aliphatischen oder aromatischen Ring anelliert sein kann,
R¹, R² unabhängig voneinander 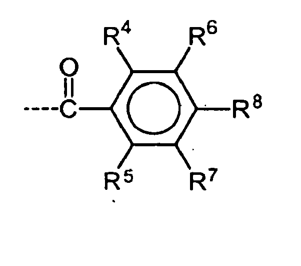 oder H sind, oder eine der für R³ angegebenen Bedeutungen haben; wobei
R⁴, R⁵ unabhängig voneinander jeweils H, Halogen, ein verzweigter oder linearer C₁₋₆-Alkyl- oder -O-C₁₋₆-Alkylrest sind die durch ein oder mehrere Sauerstoffatome unterbrochen sein können;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy-Rest sind, der durch ein oder mehrere O, S oder -NR'- unterbrochen und durch eine oder mehrere polymerisationsfähige Gruppen und/oder Reste R⁹ substituiert sein kann, wobei R' H, Halogen, ein verzweigter, cyclischer oder vorzugsweise linearer C₁₋₂₀-Alkyl-, -Alkenyl-, -Alkyloxy- oder -Alkenoxy-Rest ist;
R³ ein verzweigter oder vorzugsweise linearer C₁₋₁₈-Alkyl-Rest oder C₂₋₁₈-Alkenyl-Rest ist, wobei diese Reste unsubstituiert oder einfach oder mehrfach durch einen Rest substituiert sein können, der aus der folgenden Gruppe ausgewählt ist: Halogen, CN, 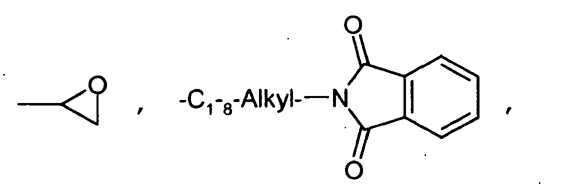 -OR¹⁰, -SR¹⁰, -OCO-R¹⁰, -COO-R¹⁰, -CH=CH-CO-OR¹⁰,-C(C₁₋₄-Alkyl)=C(C₁₋₄-Alkyl)-CO-OR¹⁰, -CO-R¹³, -CO-CH=CH-CO-C₁₋₆-Alkyl, -CO-CH=CH-CO-Phenyl, -CO-CH=CH-COO-C₁₋₁₈-Alkyl, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹²,-SO₂-R¹⁰, -SO₂-OR¹⁰, -SO₂-NR¹¹R¹², -PO(OC₁₋₈-Alkyl)₂, -SiR¹⁴R¹⁵R¹⁶, -CH=CH-Phenyl, -C(C₁₋₄-Alkyl)=C(C₁₋₄-Alkyl)-Phenyl, Phenyl-C₁₋₄-Alkyl, Phenyl, Naphtyl, Biphenyl, C₅₋₁₂-Cycloalkyl, ein gesättigter oder ungesättigter 5- oder 6-gliedriger O-, S- oder N-haltiger heterocyclischer Ring, Benzophenonyl, Thisanthonyl, wobei
R¹³ C₁₋₁₈-Alkyl, C₂₋₁₈-Alkenyl, das durch ein oder mehrere O-Atome unterbrochen ist, C₃₋₁₂-Cycloalkyl, Phenyl-C₁₋₄-Alkyl, Phenyl, Naphtyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈-Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei
R¹⁷, R¹⁸, R¹⁹ unabhängig voneinander jeweils H, C₁₋₈-Alkyl, C₂₋₈-Alkenyl, C₇₋₉-Phenylalkyl, -O-C₁₋₈-Alkyl oder Phenyl sind, und
wobei R¹⁰, R¹¹ und R¹² wie oben definiert sind;
oder
R³ ist ein verzweigter oder vorzugsweise linearer C₂₋₁₈-Alkyl-Rest oder ein C₂₋₁₈-Alkylen-Rest, der einfach oder mehrfach durch -O-, -NH-, -NR¹¹-, -S-unterbrochen ist, wobei die Reste unsubstituiert oder einfach oder mehrfach durch einen Rest substituiert sein können, der aus der folgenden Gruppe gewählt ist:
Halogen, CN, -OR¹⁰, -SR¹⁰, -OCO-R¹⁰, -COO-R¹⁰, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, Phenyl-C₁₋₄-Alkyl, Phenyl, C₅₋₁₂-Cycloalkyl;
wobei R¹⁰, R¹¹, R¹², R¹⁴, R¹⁵ und R¹⁶ wie oben definiert sind;
oder
R³ ist ein verzweigter oder vorzugsweise linearer C₂₋₁₈-Alkyl-Rest oder ein C₂₋₁₈-Alkylen-Rest, der einfach oder mehrfach durch -CO-, -COO-, -OCO-,-OCOO-, -CO-N(R¹²)-, -N(R¹²)-CO-, -N(R¹²)-CO-N(R¹²)-, -N(R¹²)-COO-, -COO-C₁₋₆-Alkylen, -COS-C₁₋₁₈-Alkylen,-SO₂-, -SO₂-O-, -SO₂-N(R¹²)-, -(CH₃)₂Si[OSi(CH₃)₂]_{q}-, mit q = 1 bis 6; Phenyl-C₁₋₄-Alkylen, Phenylen, Naphthylen, Biphenylen, C₅₋₁₂-Cycloakylen oder einen 5- oder 6-gliedrigen O-, S- oder N-haltigen heterocyclischen Ring unterbrochen ist;
wobei R¹² wie oben definiert ist;
oder
R³ ist Trimethylsilyl, Hal-(CH₃)₂Si-[OSi(CH₃)₂]ᵣ-, (CH₃)₃Si-[OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
R³ ist Phenyl-C₁₋₂₀-Alkyl, Phenyl, Naphthyl oder Biphenyl, C₅₋₁₂-Cycloalkyl oder ein gesättigter oder ungesättigter 5- oder 6-gliedriger O-, S- oder N-haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈-Alkylthioreste und/oder -NR¹¹R¹² substituiert sein können,
wobei R¹¹ und R¹² wie oben definiert sind und
m 1, 2 oder 3 ist,
n 0 oder 1 ist,
p 0 oder 1 ist;
oder vorzugsweise
R³ ist Halogen, OH, ein aromatischer C₆₋₃₀-Rest, der durch einen verzweigten, cyclischen oder vorzugsweise linearen C₁₋₂₀-Alkyl-, -Alkenyl-,-Alkoxy- oder -Alkenoxy-Rest substituiert sein kann, wobei die genannten Reste durch ein oder mehrere O, S oder N-Atome unterbrochen und/oder durch eine oder mehrere polymerisationsfähige Gruppen und/oder Reste R⁹ substituiert sein können, oder ein verzweigter, cyclischer oder vorzugsweise linearer C₁₋₂₀-Alkyl-, -Alkenyl-, -Alkoxy- oder -Alkenoxy-Rest ist, der ein- oder mehrfach durch O, S oder-NR²⁰- unterbrochen und durch eine oder mehrere polymerisationsfähige Gruppen und/oder Reste R⁹ substituiert sein kann, wobei
R⁹ -OH, -CₓF₂ₓ₊₁ mit x = 1 bis 20, -[Si(CH₃)₂]_{y}-CH₃ mit y = 1 bis 20 ist, und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist;
wobei jeweils zwei der Reste R⁰, R¹, R² oder R³ unter Ausbildung eines 5- bis 8-gliedrigen Rings miteinander verbunden sein können, wobei der oder die Ringe mit einem oder mehreren aliphatischen oder aromatischen Ringen anelliert, unsubstituiert oder ein- oder mehrfach substituiert sein und weitere Heteroatome enthalten können.

2. Zusammensetzung nach Anspruch 1, in der
R⁰ C₁₋₁₈-Alkyl, C₂₋₁₈-Alkenyl, wobei diese Reste unsubstituiert oder einfach oder mehrfach durch Halogen substituiert sein können, -OR¹⁰, -OCO-R¹⁰,-OCO-Hal, -COO-R¹⁰, -CH=CH-CO-OR¹⁰, -N(R¹¹)-CO-R¹⁰,-N (R¹¹) -CO-Hal, -C(C₁₋₄-Alkyl)=C(C₁₋₄-Alkyl)-CO-OR¹⁰, -CO-NR¹¹R¹², -CH=CH-Phenyl, -C(C₁₋₄-Alkyl)=C(C₁₋₄-Alkyl)-Phenyl, C₃₋₁₂-Cycloalkyl, C₂₋₁₈-Alkenyl, Phenyl-C₁₋₄-Alkyl, Phenyl, Naphthyl, Antryl, Biphenyl, ein 5- oder 6-gliedriger O-, S- oder N-haltiger heterocyclischer Ring, wobei alle genannten Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder 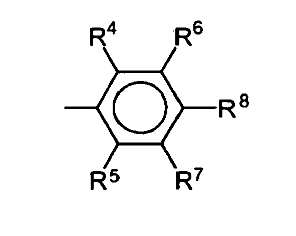 ist, wobei
R¹⁰ H, C₁₋₁₈-Alkyl, C₂₋₁₈-Alkenyl, C₂₋₁₈-Alkenyl, das durch ein oder mehrere Sauerstoffatome unterbrochen ist, C₃₋₁₂-Cycloalkyl, Tetrahydropyran-2-yl, Phenyl-C₁₋₄-Alkylen, Phenyl-C₁₋₄-Alkenylen, C₁₋₆-Alkyl, das unsubstituiert oder durch Halogen, Cyclohexyl, Cyclopentyl, Tetrahydrofuranyl, Furanyl oder Isopropyl-4-methyl-cyclohexyl substituiert sein kann, Phenyl, Naphthyl oder Biphenyl ist, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können,
R¹¹, R¹² unabhängig voneinander H, C₁₋₁₈-Alkyl, C₂₋₁₈-Alkenyl, C₂₋₁₈-Alkenyl, das durch ein oder mehrere Sauerstoffatome unterbrochen ist, C₃₋₁₂-Cycloalkyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder R¹¹ und R¹² bilden zusammen einen 5-oder 6-gliedrigen O-, S- oder N-haltigen heterocyclischen Ring, der seinerseits mit einem aliphatischen oder aromatischen Ring anelliert sein kann,
R¹, R² unabhängig voneinander H oder 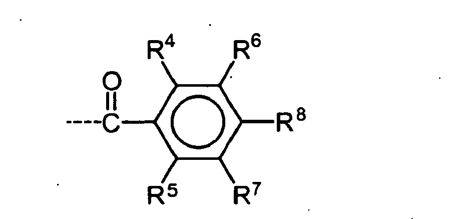 sind, oder eine der für R³ angegebenen Bedeutungen haben; wobei
R⁴, R⁵ unabhängig voneinander jeweils H, Halogen, ein verzweigter oder linearer C₁₋₆-Alkyl- oder -O-C₁₋₆-Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy-Rest sind, der durch ein oder mehrere O, S oder -NR²⁰- unterbrochen und durch eine oder mehrere polymerisationsfähige Gruppen und/oder Reste R⁹ substituiert sein kann, wobei
R⁹ -OH, -CₓF₂ₓ₊₁ mit x = 1 bis 20, Si(CH₃)₂]_{y}-CH₃ mit y = 1 bis 20 ist und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist;
R³ ein verzweigter oder vorzugsweise linearer C₁₋₁₈-Alkyl-Rest oder C₂₋₁₈-Alkenyl-Rest ist, wobei diese Reste unsubstituiert oder einfach oder mehrfach durch einen Rest substituiert sein können, der aus der folgenden Gruppe ausgewählt ist: Halogen, CN, 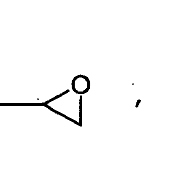 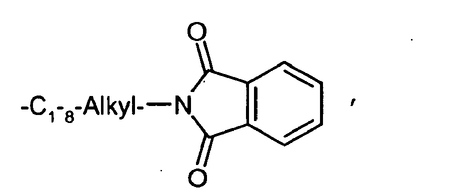 -OR¹⁰, -SR¹⁰, -OCO-R¹⁰, -COO-R¹⁰, -CH=CH-CO-OR¹⁰, -C(C₁₋₄--Alkyl)=C(C₁₋₄-Alkyl)-CO-OR¹⁰, -CO-R¹³, -CO-CH=CH-CO-C₁₋₆-Alkyl, -CO-CH=CH-CO-Phenyl, -CO-CH=CH-COO-C₁₋₁₈-Alkyl, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹²,-SO₂-R¹⁰, -SO₂-OR¹⁰, -SO₂-NR¹¹R¹², -PO(OC₁₋₈-Alkyl)₂, -SiR¹⁴R¹⁵R¹⁶, -CH=CH-Phenyl, -C(C₁₋₄-Alkyl)=C(C₁₋₄-Alkyl)-Phenyl, Phenyl-C₁₋₄-Alkyl, Phenyl, Naphtyl, Biphenyl, C₅₋₁₂-Cycloalkyl, ein 5- oder 6-gliedriger O-, S- oder N-haltiger heterocyclischer Ring, Benzophenonyl, Thisanthonyl, wobei
R¹³ C₁₋₁₈-Alkyl, C₂₋₁₈-Alkenyl, das durch ein oder mehrere O-Atome unterbrochen ist, C₃₋₁₂-Cycloalkyl, Phenyl-C₁₋₄-Alkyl, Phenyl, Naphtyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈-Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei
R¹⁷, R¹⁸, R¹⁹ unabhängig voneinander jeweils H, C₁₋₈-Alkyl, C₂₋₈-Alkenyl, C₇₋₉-Phenylalkyl, -O-C₁₋₈-Alkyl oder Phenyl sind, und
wobei R¹⁰, R¹¹ und R¹² wie oben definiert sind;
oder
R³ ist ein verzweigter oder vorzugsweise linearer C₂₋₁₈-Alkyl-Rest oder ein C₂₋₁₈-Alkylen-Rest, der einfach oder mehrfach durch -O-, -NH-, -NR¹¹-, -S-unterbrochen ist, wobei die Reste unsubstituiert oder einfach oder mehrfach durch einen Rest substituiert sein können, der aus der folgenden Gruppe gewählt ist:
Halogen, CN, -OR¹⁰, -SR¹⁰, -OCO-R¹⁰, -COO-R¹⁰, -NR¹¹R¹², -N(R¹¹)-CO-R¹⁰_{,} -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR^{10,} -SO₂-NR¹¹R¹², -PO(OC₁₋₈-Alkyl)₂, -SiR¹⁴R¹⁵R¹⁶, Phenyl-C₁₋₄-Alkyl, Phenyl, C₅₋₁₂-Cycloalkyl;
wobei R¹⁰, R¹¹ und R¹² wie oben definiert sind;
oder
R³ ist ein verzweigter oder vorzugsweise linearer C₂₋₁₈-Alkyl-Rest oder ein C₂₋₁₈-Alkylen-Rest, der einfach oder mehrfach durch -CO-, -COO-, -OCO-, -OCOO-, -CO-N(R¹¹)-, -N(R¹¹)-CO-, -N(R¹¹)-CO-N(R¹¹)-, -N (R¹¹) -COO-, -COO-C₁₋₆-Alkylen, -COS-C₁₋₁₈-Alkylen, -SO₂-, -SO₂-O-, -SO₂-N(R¹¹)-, -(CH₃)₂Si[OSi(CH₃)₂]_{q}-, mit q = 1 bis 6; Phenyl-C₁₋₄-Alkylen, Phenylen, Naphthylen, Biphenylen, C₅₋₁₂-Cycloalkylen oder einen 5- oder 6-gliedrigen O-, S- oder N-haltigen heterocyclischen Ring unterbrochen ist;
wobei R¹¹ wie oben definiert ist;
oder
R³ ist Trimethylsilyl, Hal-(CH₃)₂Si-[OSi(CH₃)₂]ᵣ-, (CH₃)₃Si-[OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
R³ ist Phenyl-C₁₋₄-Alkyl, Phenyl, Naphthyl oder Biphenyl, C₅₋₁₂-Cycloalkyl oder ein 5- oder 6-gliedriger O-, S- oder N-haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈-Alkylthioreste und/oder -NR¹¹R¹² substituiert sein können,
wobei R¹¹ und R¹² wie oben definiert sind,
m 1, 2 oder 3 ist,
n 0 oder 1 ist,
p 0 oder 1 ist;
oder
R³ ist Halogen, OH, ein aromatischer C₆₋₃₀-Rest, der durch einen verzweigten, cyclischen oder vorzugsweise linearen C₁₋₂₀-Alkyl-, -Alkenyl-, -Alkoxy- oder -Alkenoxy-Rest substituiert sein kann, wobei die genannten Reste ein- oder mehrfach durch O, S oder -NR²⁰- unterbrochen und/oder durch eine oder mehrere polymerisationsfähige Gruppen und/oder Reste R⁹ substituiert sein können, oder ein verzweigter, cyclischer oder vorzugsweise linearer C₁₋₂₀-Alkyl-, -Alkenyl-, -Alkoxy- oder -Alkenoxy-Rest ist, der ein- oder mehrfach durch O, S oder -NR²⁰- unterbrochen und durch eine oder mehrere polymerisationsfähige Gruppen und/oder Reste R⁹ substituiert sein kann, wobei R⁹ und R²⁰ wie oben definiert sind.

3. Zusammensetzung mit mindestens einem polymerisierbaren Bindemittel und einem Polymerisationsinitiator, die mindestens eine Acylgermanium-Verbindung gemäß der allgemeinen Formel (II) enthält, 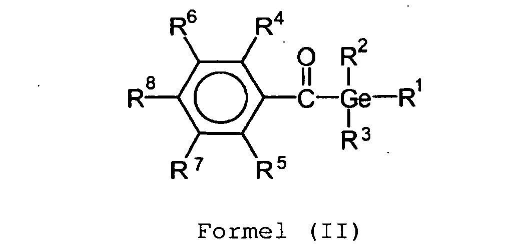 in der
R¹, R² unabhängig voneinander 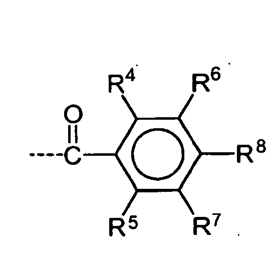 oder H sind, oder eine der für R³ angegebenen Bedeutungen haben;
R³ Halogen, OH, ein aromatischer C₆₋₃₀-Rest, der durch einen verzweigten, cyclischen oder vorzugsweise linearen C₁₋₂₀-Alkyl-, -Alkenyl-, -Alkoxy- oder -Alkenoxy-Rest substituiert sein kann, wobei die genannten Reste ein- oder mehrfach durch O, S oder -NR²⁰- unterbrochen und/oder durch eine oder mehrere polymerisationsfähige Gruppen und/oder Reste R⁹ substituiert sein können, oder ein verzweigter, cyclischer oder vorzugsweise linearer C₁₋₂₀-Alkyl-, -Alkenyl-, -Alkoxy- oder -Alkenoxy-Rest ist, der ein- oder mehrfach durch O, S oder-NR²⁰- unterbrochen und durch eine oder mehrere polymerisationsfähige Gruppen und/oder Reste R⁹ substituiert sein kann;
R⁴, R⁵ unabhängig voneinander jeweils H, Halogen, ein linearer oder verzweigter C₁₋₆-Alkyl- oder -O-C₁₋₆-Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy-Rest sind, der ein- oder mehrfach durch O, S oder -NR²⁰- unterbrochen und durch eine oder mehrere polymerisationsfähige Gruppen und/oder Reste R⁹ substituiert sein kann;
R⁹ -OH, -CₓF₂ₓ₊₁ mit x = 1 bis 20, -[Si(CH₃)₂]_{y}-CH₃ mit y = 1 bis 20 ist; und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist.

4. Zusammensetzung mit mindestens einem polymerisierbaren Bindemittel und einem Polymerisationsinitiator, die mindestens eine Acylgermanium-Verbindung gemäß der allgemeinen Formel (III) enthält, 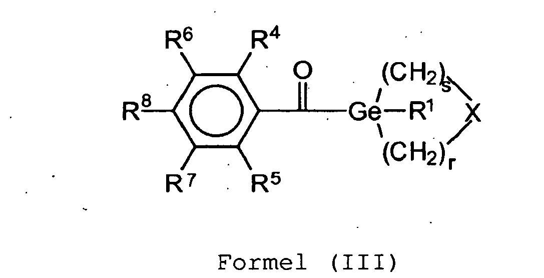 in der
R¹ 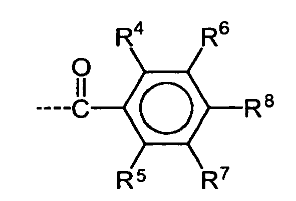 oder H ist, oder eine der für R³ angegebenen Bedeutungen hat;
r, s unabhängig voneinander eine ganze Zahl von 0 bis 6 sind, wobei r und s so gewählt sind, daß die Summe der Ringatome einschließlich des Germaniumatoms 5 bis 8 beträgt, und
X N-R²¹, O, S oder entfällt bedeutet, wobei R²¹ H oder C₁₋₁₀-Alkyl ist, wobei der Germanium enthaltende Ring mit einem oder mehreren aliphatischen oder aromatischen Ringen anelliert und unsubstituiert oder ein- oder mehrfach substituiert sein kann, wodurch die Anzahl der Wasserstoffatome des Rings entsprechend reduziert wird.

5. Zusammensetzung nach einem der vorhergehenden Ansprüche, bei der die polymerisationsfähigen Gruppen aus Vinyl, Styryl, (Meth)acrylat, (Meth)acrylamid oder N-Alkylacrylamid ausgewählt sind.

6. Zusammensetzung nach einem der vorhergehenden Ansprüche, bei der die Reste R², R³, R⁶, R⁷ und R⁸ jeweils mit 1 bis 3 polymerisierbaren Gruppe substituiert sind.

7. Zusammensetzung nach einem der vorhergehenden Ansprüche, die bezogen auf die Gesamtmasse der Zusammensetzung 0,001 bis 5 Gew.-% des Acylgermans der Formel (I) enthält.

8. Zusammensetzung nach einem der vorhergehenden Ansprüche, die als polymerisierbares Bindemittel mindestens ein radikalisch polymerisierbare Monomer und/oder Prepolymer enthält.

9. Zusammensetzung nach Anspruch 8, die als Bindemittel ein mono- oder multifunktionelles (Meth)acrylat oder eine Mischung davon enthält.

10. Zusammensetzung nach Anspruch 8 oder 9, die mindestens ein radikalisch ringöffnend polymerisierbares Monomer enthält.

11. Zusammensetzung nach einem der vorhergehenden Ansprüche, die als Bindemittel eine Mischung von mono- und/oder multifunktionellen Mercaptoverbindungen und di- und/oder multifunktionellen ungesättigten Monomeren enthält.

12. Zusammensetzung nach einem der vorhergehenden Ansprüche, die mindestens einen weiteren Initiator für die radikalische Polymerisation enthält.

13. Zusammensetzung nach einem der vorhergehenden Ansprüche, die mindestens einen weiteren Initiator für die kationische Polymerisation enthält.

14. Zusammensetzung nach einem der vorhergehenden Ansprüche, die zusätzlich Füllstoff enthält.

15. Zusammensetzung nach einem der vorhergehenden Ansprüche, die zusätzlich mindestens ein Additiv enthält, das aus Stabilisatoren, UV-Absorbern, Gleitmitteln, Netzmitteln, Dispergiermitteln, Haftvermittlern, Mattierungs- und Glanzmitteln, Verlaufs- und Filmbildehilfsmitteln, Hautverhinderungsmitteln, Lichtschutzmitteln, Korrosionsschutzmitteln, flammhemmenden Mitteln, Antioxidantien, optischen Aufheller, Fließverbesserern, Verdickern und Antischaummitteln ausgewählt ist.

16. Zusammensetzung nach Anspruch 1, die
0,001 bis 5 Gew.-% Acylgermanium-Verbindung gemäß Formel (I),
5 bis 99,9 Gew.-% polymerisierbares Bindemittel,
0 bis 90 Gew.-% Füllstoff enthält, jeweils bezogen auf die Gesamtmasse der Zusammensetzung.

17. Zusammensetzung nach Anspruch 16, die 0 bis 50 Gew.-% weiteres Additiv enthält.

18. System zur Herstellung von Formkörpern, daß eine einer Zusammensetzung gemäß einem der Ansprüche 1 bis 17 und einer LED-Lichtquelle umfaßt.

19. System nach Anspruch 18, bei dem die LED-Lichtquelle eine Wellenlänge im Bereich von 400 bis 550 nm hat und das Acylgermanium-Verbindung eine Aktivierungswellenlänge im Bereich von 400 bis 550 nm aufweist.

20. Verwendung eines Acylgermans gemäß Formel (I) als Initiator für die radikalische Polymerisation.

21. Verwendung eines Acylgermans gemäß Formel (I) zur Verhinderung der Ausbildung einer Inhibierungsschicht bei der radikalischen Polymerisation.

22. Verwendung eines Acylgermans gemäß Formel (I) zur Herstellung von Adhäsiven, Beschichtungen, Zementen, Kompositen, Formteilen oder Dentalwerkstoffen.

23. Verfahren zur Herstellung eines Formkörpers, bei dem man eine Zusammensetzung gemäß einem der Ansprüche 1 bis 16 zu einem Körper mit der gewünschten Form formt und anschließend ganz oder teilweise härtet.

24. Verfahren nach Anspruch 23 zur Herstellung von dentalen Kronen, Brücken, Inlays oder künstlichen Zähnen.

## Claims

1. A composition with at least one polymerisable binder and a polymerisation initiator comprising at least one acylgermane according to the general Formula (I), 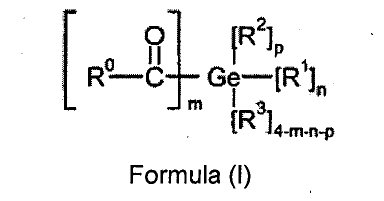 in which
R⁰ is C₁₋₁₈ alkyl, C₂₋₁₈ alkenyl, wherein these groups can be unsubstituted or mono or poly substituted by halogen, -OR¹⁰,-OCO-R¹⁰, -OCO-hal, -COO-R¹⁰, -CH-CH-CO-OR¹⁰, -N(R¹¹)-CO-R¹⁰, -N(R¹¹)-CO-hal, -C(C₁₋₄ alkyl)=C(C₁₋₄ alkyl)-CO-OR¹⁰, -CO-NR¹¹R¹², -CH=CH-phenyl, -C(C₁₋₄ alkyl)=C(C₁₋₄ alkyl)phenyl, C₃₋₁₂ cycloalkyl, C₂₋₁₈ alkenyl, phenyl-C₁₋₄ alkyl, phenyl, naphthyl, antryl, biphenyl, a saturated or unsaturated 5 or 6-membered O, S or N-containing heterocyclic ring, wherein all the cited ring systems can be unsubstituted or substituted by 1 to 5 halogen atoms, C₁₋₈ alkyl, C₁₋₈ alkoxy and/or C₁₋₈ alkylthio groups, or is 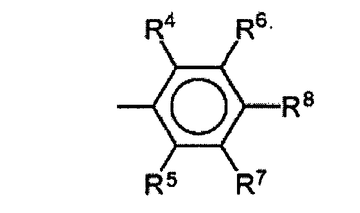
wherein
R¹⁰ is H, C₁₋₁₈ alkyl, C₁₋₁₈ alkyl interrupted by one or more oxygen atoms, C₂₋₁₈ alkenyl, C₂₋₁₈ alkenyl interrupted by one or more oxygen atoms, C₃₋₁₂ cycloalkyl, tetrahydropyran-2-yl, phenyl C₁₋₂₀ alkylene, C₁₋₂₀ alkenylene, C₁₋₆ alkyl that is unsubstituted or can be substituted by halogen, cyclohexyl, cyclopentyl, tetrahydrofuranyl, furanyl or isopropyl-4-methyl-cyclohexyl, phenyl, naphthyl or biphenyl, wherein these ring systems can be unsubstituted or substituted by 1 to 5 halogen atoms, C₁₋₈ alkyl, C₁₋₈ alkoxy and/or C₁₋₈ alkylthio groups,
R¹¹, R¹² independently of each other are H, C₁₋₁₈ alkyl, C₁₋₁₈ alkyl interrupted by one or more oxygen atoms, C₂₋₁₈ alkenyl, C₂₋₁₈ alkenyl interrupted by one or more oxygen atoms, C₃₋₁₂ cycloalkyl, phenyl-C₁₋₄ alkyl, phenyl, naphthyl or pyridyl, wherein these ring systems can be unsubstituted or substituted by 1 to 5 halogen atoms, C₁₋₈ alkyl, C₁₋₈ alkoxy and/or C₁₋₈ alkylthio groups, or R¹¹ and R¹² together form a 5 or 6-membered O, S or N-containing heterocyclic ring that itself can be anellated with an aliphatic or aromatic ring,
R¹, R² independently of each other are H or 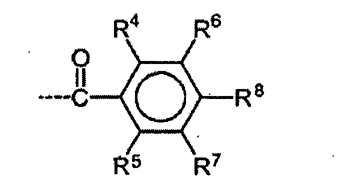
or have one of the meanings given for R³; wherein
R⁴, R⁵ independently of each other are in each case H, halogen, a branched or linear C₁₋₆ alkyl or -O-C₁₋₆ alkyl group;
R⁶, R⁷, R⁸ independently of each other are in each case H, halogen, a branched, cyclic or preferably linear C₁₋₂₀ alkyl, -alkenyl, -alkyloxy or -alkenoxy group, which can be interrupted by one or more O, S or -NR'- and can be substituted by one or more polymerisable groups and/or groups R⁹, wherein R' is H, halogen, a branched, cyclic or preferably linear C₁₋₂₀ alkyl, -alkenyl, -alkyloxy oralkenoxy group;
R³ is a branched or preferably linear C₁₋₁₈ alkyl group or C₂₋₁₈ alkenyl group, wherein these groups can be unsubstituted or mono or poly substituted by a group that is selected from the following groups:
halogen, CN, 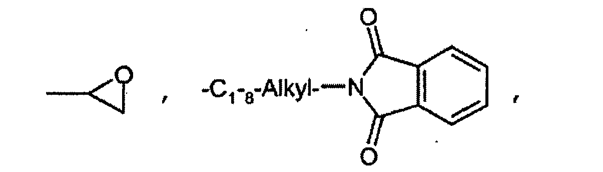 -OR¹⁰, -SR¹⁰, -OCO-R¹⁰, -COOR¹⁰, -CH=CH-CO-OR¹⁰, -C(C₁₋₄ alkyl)=C(C₁₋₄ alkyl)-CO-OR¹⁰, -CO-R¹³, -CO-CH=CH-CO-C₁₋₆ alkyl, -CO-CH=CH-CO-phenyl, -CO-CH=CH-COO-C₁₋₁₈ alkyl,NR¹¹R¹², -N(R¹¹)-CO-R¹⁰, -N(R¹¹)-COO-R¹⁰, -N(R¹¹)-CO-NR¹¹R¹², -N(R¹¹)-CO-hal, -CO-NR¹¹R¹², SO₂-R¹⁰, -SO₂-OR¹⁰, -SO₂-NR¹¹R¹², -PO(OC₁₋₈ alkyl)₂, -SiR¹⁴R¹⁵R¹⁶, CH=CH-phenyl, -C(C₁₋₄ alkyl)=C(C₁₋₄ alkyl)phenyl, phenyl-C₁₋₄ alkyl, phenyl, naphthyl, biphenyl, C₅₋₁₂ cycloalkyl, a saturated or unsaturated 5 or 6-membered O, S or N-containing heterocyclic ring, benzophenonyl, thisanthonyl, wherein
R¹³ is C₁₋₁₈ alkyl, C₂₋₁₈ alkenyl that is interrupted by one or more O atoms, C₃₋₁₂ cycloalkyl, phenyl-C₁₋₄ alkyl, phenyl, naphthyl or biphenyl, wherein the cited ring systems can be unsubstituted or substituted by 1 to 5 C₁₋₈ alkyl, C₁₋₈ alkoxy, C₁₋₈ alkylthio groups and/or halogen atoms;
R¹⁴, R¹⁵, R¹⁶ independently of each other are in each case H, C₁₋₈ alkyl, C₂₋₈ alkenyl, C₇₋₉ phenylalkyl, -O-C₁₋₈ alkyl, phenyl or -O-SiR¹⁷R¹⁸R¹⁹, wherein
R¹⁷, R¹⁸, R¹⁹ independently of each other are in each case H, C₁₋₈ alkyl, C₂₋₈ alkenyl, C₇₋₉ phenylalkyl, -O-C₁₋₈ alkyl or phenyl, and
wherein R¹⁰, R¹¹ and R¹² are as defined above;
or
R³ is a branched or preferably linear C₂₋₁₈ alkyl group or a C₂₋₁₈ alkylene group that is interrupted once or more by -O-, -NH-,-NR¹¹-, -S-, wherein the groups can be mono or polysubstituted by a group that is selected from the following group:
halogen, CN, -OR¹⁰, -SR¹⁰, -OCO-R¹⁰, -COOR¹⁰, -NR¹¹R¹²,-N(R¹¹)-CO-R¹⁰, -N(R¹¹)-COO-R¹⁰, -N(R¹¹)-CO-NR¹¹R¹², -N(R¹¹)-CO-hal, -CO-NR¹¹R¹², SO₂-R¹⁰, -SO₂-OR¹⁰, -SO₂-NR¹¹R¹², -PO(OC₁₋₈ alkyl)₂, -SiR¹⁴R¹⁵R¹⁶, phenyl-C₁₋₄ alkyl, phenyl, C₅₋₁₂ cycloalkyl;
wherein R¹⁰, R¹¹, R¹², R¹⁴, R¹⁵ and R¹⁶ are defined as above;
or
R³ is a branched or preferably linear C₂₋₁₈ alkyl group or a C₂₋₁₈ alkylene group that is interrupted once or more by -CO-, -COO-,-OCO-, -OCOO-, -CO-N(R¹²)-, -N(R¹²)-CO-, -N(R¹²)-CO-N(R¹²)-,-N(R¹²)-COO-, -COO-C₁₋₆ alkylene, -COS-C₁₋₁₈ alkylene, -SO₂-,-SO₂-O-, -SO₂-N(R¹²)-, -(CH₃)₂Si[OSi(CH₃)₂]_{q}-, with q = 1 to 6; phenyl-C₁₋₄ alkylene, phenylene, naphthylene, biphenylene, C₅₋₁₂ cycloalkylene or a 5 or 6-membered O, S or N-containing heterocyclic ring;
wherein R¹² is as defined above;
or
R³ is trimethylsilyl, hal-(CH₃)₂Si-[OSi(CH₃)₂]ᵣ-, (CH₃)₃Si-[OSi(CH₃)₂]ᵣ-with r = 1 to 6, -COOH, -COO-R¹⁰, -CO-NR¹¹R¹², -CO-vinyl, -CO-phenyl; wherein the phenyl group can be unsubstituted or be substituted by -CH₃, -OCH₃ and/or -Cl;
wherein R¹⁰, R¹¹ and R¹² are as defined above;
R³ is phenyl-C₁₋₂₀ alkyl, phenyl, naphthyl or biphenyl, C₅₋₁₂ cycloalkyl or a saturated or unsaturated 5 or 6-membered O, S or N-containing heterocyclic ring, wherein these ring systems can be unsubstituted or substituted by 1 to 5 halogen atoms, C₁₋₈ alkyl, C₁₋₈ alkoxy, C₁₋₈ alkylthio groups and/or -NR¹¹R¹²,
wherein R¹¹ and R¹² are defined as above and
m is 1, 2 or 3,
n is 0 or 1,
p is 0 or 1;
or preferably
R³ is halogen, OH, an aromatic C₆₋₃₀ group that can be substituted by a branched, cyclic or preferably linear C₁₋₂₀ alkyl, -alkenyl, -alkoxy or -alkenoxy group, wherein the cited groups can be interrupted by one or more O, S or N atoms and/or by one or more polymerisable groups and/or R⁹ groups, or is a branched, cyclic or preferably linear C₁₋₂₀ alkyl, -alkenyl, -alkoxy or -alkenoxy group, which can be interrupted once or more by O, S or -NR²⁰- and substituted by one or more polymerisable groups and/or R⁹ groups, wherein
R⁹ is -OH, CₓF₂ₓ₊₁ with x = 1 to 20, -[Si(CH₃)₂]_{y}-CH₃ with y = 1 to 20, and
R²⁰ is H, halogen, a branched, cyclic or preferably linear C₁₋₂₀ alkyl,-alkenyl, -alkoxy or -alkenoxy group;
wherein each two of the groups R⁰, R¹, R² or R³ can be linked together forming a 5 to 8 membered ring, wherein the ring(s) can be anellated with one or more aliphatic or aromatic rings, unsubstituted or mono or poly substituted and can comprise additional heteroatoms.

2. The composition of claim 1, in which
R⁰ is C₁₋₁₈ alkyl, C₂₋₁₈ alkenyl, wherein these groups can be unsubstituted or mono or poly substituted by halogen, -OR¹⁰,-OCO-R¹⁰, -OCO-hal, -COO-R¹⁰, -CH-CH-CO-OR¹⁰, -N(R¹¹)-CO-R¹⁰, -N(R¹¹)-CO-hal, -C(C₁₋₄ alkyl)=C(C₁₋₄ alkyl)-CO-OR¹⁰, -CO-NR¹¹R¹², -CH=CH-phenyl, -C(C₁₋₄ alkyl)=C(C₁₋₄ alkyl)phenyl, C₃₋₁₂ cycloalkyl, C₂₋₁₈ alkenyl, phenyl-C₁₋₄ alkyl, phenyl, naphthyl, antryl, biphenyl, a 5 or 6-membered O, S or N-containing heterocyclic ring, wherein all the cited ring systems can be unsubstituted or substituted by 1 to 5 halogen atoms, C₁₋₈ alkyl, C₁₋₈ alkoxy and/or C₁₋₈ alkylthio groups, or is 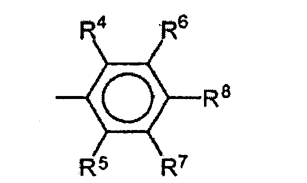 wherein
R¹⁰ is H, C₁₋₁₈ alkyl, C₂₋₁₈ alkenyl, C₂₋₁₈ alkenyl interrupted by one or more oxygen atoms, C₃₋₁₂ cycloalkyl, tetrahydropyran-2-yl, phenyl C₁₋₄ alkylene, phenyl-C₁₋₄ alkenylene, C₁₋₆ alkyl that is unsubstituted or can be substituted by halogen, cyclohexyl, cyclopentyl, tetrahydrofuranyl, furanyl or isopropyl-4-methyl-cyclohexyl, phenyl, naphthyl or biphenyl, wherein these ring systems can be unsubstituted or substituted by 1 to 5 halogen atoms, C₁₋₈ alkyl, C₁₋₈ alkoxy and/or C₁₋₈ alkylthio groups,
R¹¹, R¹² independently of each other are H, C₁₋₁₈ alkyl, C₂₋₁₈ alkenyl, C₂₋₁₈ alkenyl interrupted by one or more oxygen atoms, C₃₋₁₂ cycloalkyl, phenyl-C₁₋₄ alkyl, phenyl, naphthyl or pyridyl, wherein these ring systems can be unsubstituted or substituted by 1 to 5 halogen atoms, C₁₋₈ alkyl, C₁₋₈ alkoxy and/or C₁₋₈ alkylthio groups, or R¹¹ and R¹² together form a 5 or 6-membered O, S or N-containing heterocyclic ring that itself can be anellated with an aliphatic or aromatic ring,
R¹, R² independently of each other are H or 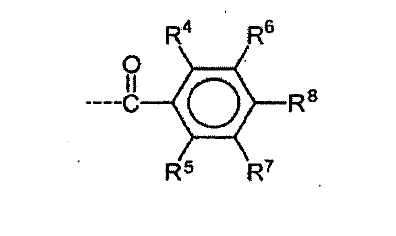
R⁴, R⁵ or have one of the meanings given for R³; wherein independently of each other are in each case H, halogen, a branched or linear C₁₋₆ alkyl or -O-C₁₋₆ alkyl group;
R⁶, R⁷, R⁸ independently of each other are in each case H, halogen, a branched, cyclic or preferably linear C₁₋₂₀ alkyl, -alkenyl, -alkyloxy or -alkenoxy group, which can be interrupted by one or more O, S or -NR²⁰- and can be substituted by one or more polymerisable groups and/or groups R⁹, wherein
R⁹ is -OH, -CₓF₂ₓ₊₁ with x = 1 to 20, -[Si(CH₃)₂]_{y}-CH₃ with y = 1 to 20 and
R²⁰ is H, halogen, a branched, cyclic or preferably linear C₁₋₂₀ alkyl,-alkenyl, -alkyloxy or -alkenoxy group;
R³ is a branched or preferably linear C₁₋₁₈ alkyl group or C₂₋₁₈ alkenyl group, wherein these groups can be unsubstituted or mono or poly substituted by a group that is selected from the following groups:
halogen, CN, 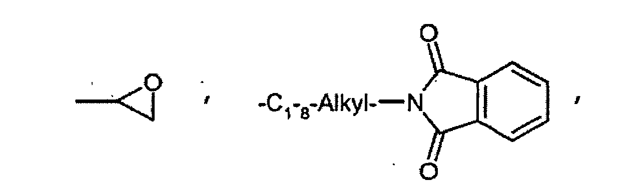 -OR¹⁰, -SR¹⁰, -OCO-R¹⁰, -COOR¹⁰, -CH=CH-CO-OR¹⁰, -C(C₁₋₄ alkyl)=C(C₁₋₄ alkyl)-CO-OR¹⁰, -CO-R¹³, -CO-CH=CH-CO-C₁₋₆ alkyl, -CO-CH=CH-CO-phenyl, -CO-CH=CH-COO-C₁₋₁₈ alkyl,-NR¹¹R¹², -N(R¹¹)-CO-R¹⁰, -N(R¹¹)-COO-R¹⁰, -N(R¹¹)-CO-NR¹¹R¹², -N(R¹¹)-CO-hal, -CO-NR¹¹R¹², SO₂-R¹⁰, -SO₂-OR¹⁰, -SO₂-NR¹¹R¹², -PO(OC₁₋₈ alkyl)₂, -SiR¹⁴R¹⁵R¹⁶, CH=CH-phenyl, -C(C₁₋₄ alkyl)=C(C₁₋₄ alkyl)phenyl, phenyl-C₁₋₄ alkyl, phenyl, naphthyl, biphenyl, C₅₋₁₂ cycloalkyl, a saturated or unsaturated 5 or 6-membered O, S or N-containing heterocyclic ring, benzophenonyl, thisanthonyl, wherein
R¹³ is C₁₋₁₈ alkyl, C₂₋₁₈ alkenyl that is interrupted by one or more O atoms, C₃₋₁₂ cycloalkyl, phenyl-C₁₋₄ alkyl, phenyl, naphthyl or biphenyl, wherein the cited ring systems can be unsubstituted or substituted by 1 to 5 C₁₋₈ alkyl, C₁₋₈ alkoxy, C₁₋₈ alkylthio groups and/or halogen atoms;
R¹⁴, R¹⁵, R¹⁶ independently of each other are in each case H, C₁₋₈ alkyl, C₂₋₈ alkenyl, C₇₋₉ phenylalkyl, -O-C₁₋₈ alkyl, phenyl or -O-SiR¹⁷R¹⁸R¹⁹ wherein
R¹⁷, R¹⁸, R¹⁹ independently of each other are in each case H, C₁₋₈ alkyl, C₂₋₈ alkenyl, C₇₋₉ phenylalkyl, -O-C₁₋₈ alkyl or phenyl, and
wherein R¹⁰, R¹¹ and R¹² are as defined above;
or
R³ is a branched or preferably linear C₂₋₁₈ alkyl group or a C₂₋₁₈ alkylene group that is interrupted once or more by -O-, -NH-,-NR¹¹-, -S-, wherein the groups can be mono or polysubstituted by a group that is selected from the following group:
halogen, CN, -OR¹⁰, -SR¹⁰, -OCO-R¹⁰, -COOR¹⁰, -NR¹¹R¹², -N(R¹¹)-CO-R¹⁰, -N(R¹¹)-COO-R¹⁰, -N(R¹¹)-CO-NR¹¹R¹², -N(R¹¹)-CO-hal, -CO-NR¹¹R¹², SO₂-R¹⁰, -SO₂-OR¹⁰, -SO₂-NR¹¹R¹²,-PO(OC₁₋₈ alkyl)₂, -SiR¹⁴R¹⁵R¹⁶, phenyl-C₁₋₄ alkyl, phenyl, C₅₋₁₂ cycloalkyl;
wherein R¹⁰, R¹¹, R¹², R¹⁴, R¹⁵ and R¹⁶ are defined as above;
or
R³ is a branched or preferably linear C₂₋₁₈ alkyl group or a C₂₋₁₈ alkylene group that is interrupted once or more by -CO-, -COO-,-OCO-, -OCOO-, -CO-N(R¹²)-, -N(R¹²)-CO-, -N(R¹²)-CO-N(R¹²)-,-N(R¹²)-COO-, -COO-C₁₋₆ alkylene, -COS-C₁₋₁₈ alkylene, -SO₂-,-SO₂-O-, -SO₂-N(R¹²)-, -(CH₃)₂Si[OSi(CH₃)₂]_{q}-, with q = 1 to 6; phenyl-C₁₋₄ alkylene, phenylene, naphthylene, biphenylene, C₅₋₁₂ cycloalkylene or a 5 or 6-membered O, S or N-containing heterocyclic ring;
wherein R¹¹ is as defined above;
or
R³ is trimethylsilyl, hal-(CH₃)₂Si-[OSi(CH₃)₂]ᵣ-, (CH₃)₃Si-[OSi(CH₃)₂]ᵣ-with r = 1 to 6, -COOH, -COO-R¹⁰, -CO-NR¹¹R¹², -CO-vinyl, -CO-phenyl; wherein the phenyl group can be unsubstituted or be substituted by -CH₃, -OCH₃ and/or -Cl;
wherein R¹⁰, R¹¹ and R¹² are as defined above;
or
R³ is phenyl-C₁₋₄ alkyl, phenyl, naphthyl or biphenyl, C₅₋₁₂ cycloalkyl or a saturated or unsaturated 5 or 6-membered O, S or N-containing heterocyclic ring, wherein these ring systems can be unsubstituted or substituted by 1 to 5 halogen atoms, C₁₋₈ alkyl, C₁₋₈ alkoxy, C₁₋₈ alkylthio groups and/or -NR¹¹R¹²,
wherein R¹¹ and R¹² are defined as above and
m is 1, 2 or 3,
n is 0 or 1,
p is 0 or 1;
or
R³ is halogen, OH, an aromatic C₆₋₃₀ group that can be substituted by a branched, cyclic or preferably linear C₁₋₂₀ alkyl, -alkenyl, -alkoxy or -alkenoxy group, wherein the cited groups can be interrupted by one or more O, S or N atoms and/or by one or more polymerisable groups and/or R⁹ groups, or is a branched, cyclic or preferably linear C₁₋₂₀ alkyl, -alkenyl, -alkoxy or -alkenoxy group, which can be interrupted once or more by O, S or -NR²⁰- and substituted by one or more polymerisable groups and/or R⁹ groups, wherein R⁹ and R²⁰ are defined as above.

3. A composition with at least one polymerisable binder and at least one polymerisation initiator, the composition comprising at least one acylgermanium compound according to the general Formula (II), 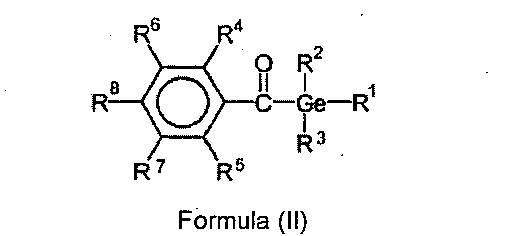 in which
R¹, R² independently of each other are 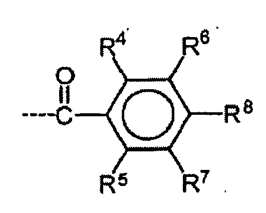 or H or have one of the meanings given for R³;
R³ is halogen, OH, an aromatic C₆₋₃₀ group that can be substituted by a branched, cyclic or linear C₁₋₂₀ alkyl, -alkenyl, -alkoxy or-alkenoxy radical, wherein the named groups can be interrupted one or more times by O, S or -NR²⁰- and/or can be substituted by one or more polymerisable groups and/or radicals R⁹, or is a branched, cyclic or preferably linear C₁₋₂₀ alkyl, -alkenyl, -alkoxy or -alkenoxy radical which can be interrupted one or more times by O, S or -NR²⁰- and can be substituted by one or more polymerizable groups and/or groups R⁹;
R⁴, R⁵ independently of each other are H, halogen, a linear or branched C₁₋₆ alkyl or -O-C₁₋₆ alkyl group;
R⁶, R⁷, R⁸ independently of each other are H, halogen, a branched, cyclic or linear C₁₋₂₀ alkyl, -alkenyl, -alkyloxy or -alkenoxy radical which is interrupted one or more times by O, S or -NR²⁰- and can be substituted by one or more polymerizable groups and/or radicals R⁹;
R⁹ is -OH, -CₓF₂ₓ₊₁ with x = 1 to 20, -[Si(CH₃)₂]_{y}-CH₃ with y = 1 to 20; and
R²⁰ is H, halogen, a branched, cyclic or linear C₁₋₂₀ alkyl, -alkenyl,-alkyloxy or -alkenoxy radical.

4. A composition with at least one polymerisable binder and at least one polymerisation initiator, the composition comprising at least an acylgermanium compound according to the general Formula (III): 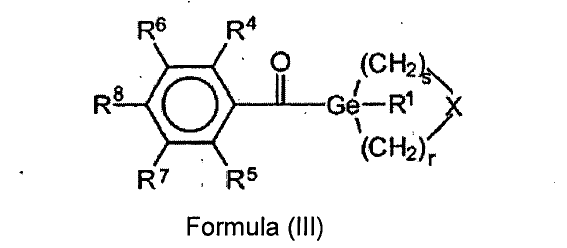 in which
R¹ is 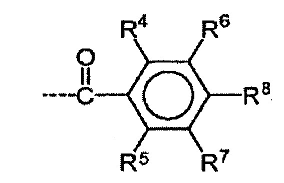 or H, or has one of the meanings given for R³;
r, s independently of each other are an integer from 0 to 6,
wherein r and s are chosen such that the sum of the ring atoms including the germanium atom is 5 to 8, and
X is N-R²¹, O, S or is absent, wherein R²¹ is H or C₁₋₁₀ alkyl, and wherein the germanium-containing ring can be anellated with one or more aliphatic or aromatic rings and can be unsubstituted or substituted once or more whereby the number of hydrogen atoms of the ring is correspondingly reduced.

5. Composition according to one of the previous claims, wherein the polymerisable groups are selected from vinyl, styryl, (meth)acrylate, (meth)acrylamide or N-alkylacrylamide.

6. Composition according to one of the previous claims, wherein the groups R², R³, R⁶, R⁷ and R⁸ are each substituted with 1 to 3 polymerisable groups.

7. Composition according to one of the previous claims, comprising 0.001 to 5 weight % of the acylgermane of Formula (I).

8. Composition according to one of the previous claims wherein the polymerisable binder comprises at least one radically polymerisable monomer and/or prepolymer.

9. Composition according to claim 8, wherein the binder comprises a mono- or multifunctional (meth)acrylate, or a mixture thereof.

10. Composition according to claim 8 or 9, comprising at least one radically ring-opening polymerisable monomer.

11. Composition according to one of the previous claims, wherein the binder comprises a mixture of mono- and/or multifunctional mercapto compounds and di- and/or multifunctional unsaturated monomers.

12. Composition according to one of the previous claims, comprising at least one further initiator for radical polymerisation.

13. Composition according to one of the previous claims, comprising at least one further initiator for cationic polymerisation.

14. Composition according to one of the previous claims, further comprising a filler.

15. Composition according to one of the previous claims, further comprising at least one additive which is selected from stabilizers, UV absorbers, slip additives, wetting agents, dispersants, adhesion promoters, matting and brightening agents, levelling agents and film-forming auxiliaries, antiskinning agents, light-protection agents, corrosion-protection agents, flame retardants, antioxidants, optical brighteners, flow improvers, thickeners and anti-foaming agents.

16. Composition according to claim 1, comprising:
0.001 to 5 weight % acylgermanium compound according to Formula (I),
5 to 99.9 weight % polymerisable binder, and
0 to 90 weight % filler, each based on the total weight of the composition.

17. Composition according to claim 16, comprising 0 to 50 weight % of a further additive.

18. System for the preparation of moulded articles, the system comprising a composition according to one of claims 1 to 17 and an LED light source.

19. System according to claim 18, wherein the LED light source has a wavelength in the range from 400 to 550 nm and the acylgermanium compound has an activation wavelength in the range from 400 to 550 nm.

20. Use of an acylgermane according to Formula (I) as an initiator for radical polymerisation.

21. Use of an acylgermane according to Formula (I) for preventing the formation of an inhibiting layer during the radical polymerisation.

22. Use of an acylgermane according to Formula (I) for manufacturing adhesives, coatings, cements, composites, mouldings or dental materials.

23. Process for manufacturing a moulded article, in which a composition according to one of claims 1 to 16 is moulded to an article of the desired shape and is subsequently partially or totally cured.

24. Process according to claim 23 for manufacturing dental crowns, bridges, inlays or synthetic teeth.

## Revendications

1. Composition comprenant au moins un liant polymérisable et un amorceur de polymérisation, **caractérisée en ce qu'**elle contient au moins un composé de type acyl-germanium répondant à la formule générale (I) : 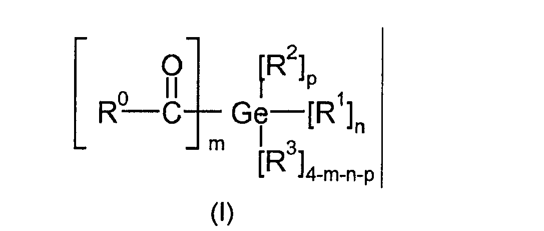 dans laquelle
- R⁰ représente un groupe alkyle en C₁₋₁₈ ou alcényle en C₂₋₁₈, ces groupes pouvant être dépourvus de substituant ou porter un ou plusieurs atome(s) d'halogène comme substituant(s), un groupe -CH=CH-phényle, -C(alkyle en C₁₋₄)=C(alkyle en C₁₋₄)-phényle, cycloalkyle en C₃₋₁₂, alcényle en C₂₋₁₈, phényl-(alkyle en C₁₋₄), phényle, naphtyle, anthryle ou biphényle, un groupe symbolisé par -OR¹⁰, -OCO-R¹⁰, -OCO-Hal, -COO-R¹⁰, -CH=CH-CO-OR¹⁰, -N(R¹¹)-CO-R¹⁰, -N(R¹¹)-CO-Hal, -C(alkyle en C₁₋₄)=C(alkyle en C₁₋₄)-CO-OR¹⁰ ou -CO-NR¹¹R¹², un groupe hétérocyclique de 5 ou 6 chaînons, saturé ou insaturé, à hétéroatome d'oxygène, de soufre ou d'azote, chacun des systèmes cycliques indiqués pouvant être dépourvu de substituant ou porter comme substituant(s) 1 à 5 atome(s) d'halogène, groupe(s) alkyle en C₁₋₈, groupe(s) alcoxy en C₁₋₈ et/ou groupe(s) alkylsulfanyle en C₁₋₈, ou un groupe de formule 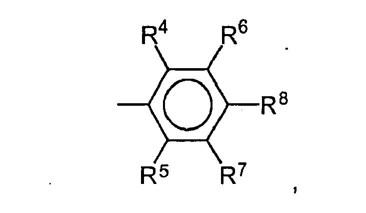 où
R¹⁰ représente un atome d'hydrogène, un groupe alkyle en C₁₋₁₈, alkyle en C₁₋₁₈ dont la chaîne est interrompue par un ou plusieurs atome(s) d'oxygène, alcényle en C₂₋₁₈, alcényle en C₂₋₁₈ dont la chaîne est interrompue par un ou plusieurs atome(s) d'oxygène, cycloalkyle en C₃₋₁₂, tétrahydropyran-2-yle, phényl-(alcanediyle en C₁₋₂₀), phényl-(alcènediylé en C₁₋₂₀), alkyle en C₁₋₆ qui peut être dépourvu de substituant ou porter un substituant halogène, cyclohexyle, cyclopentyle, tétrahydrofuranyle, furanyle ou isopropyl-4-méthylcyclohexyle, phényle, naphtyle ou biphényle, ces systèmes cycliques pouvant être dépourvus de substituant ou porter comme substituant(s) 1 à 5 atome(s) d'halogène, groupe(s) alkyle en C₁₋₈, groupe(s) alcoxy en C₁₋₈ et/ou groupe(s) alkylsulfanyle en C₁₋₈,
et R¹¹ et R¹² représentent chacun, indépendamment l'un de l'autre, un atome d'hydrogène, un groupe alkyle en C₁₋₁₈, alkyle en C₁₋₁₈ dont la chaîne est interrompue par un ou plusieurs atome(s) d'oxygène, alcényle en C₂₋₁₈, alcényle en C₂₋₁₈ dont la chaîne est interrompue par un ou plusieurs atome(s) d'oxygène, cycloalkyle en C₃₋₁₂, phényl-(alkyle en C₁₋₄), phényle, naphtyle ou pyridinyle, ces systèmes cycliques pouvant être dépourvus de substituant ou porter comme substituant(s) 1 à 5 atome(s) d'halogène, groupe(s) alkyle en C₁₋₈, groupe(s) alcoxy en C₁₋₈ et/ou groupe(s) alkylsulfanyle en C₁₋₈, ou bien R¹¹ et R¹² forment ensemble un groupe hétérocyclique de 5 ou 6 chaînons à hétéroatome d'oxygène, de soufre ou d'azote, qui peut lui-même être condensé avec un cycle aliphatique ou aromatique,
- R¹ et R² représentent chacun, indépendamment l'un de l'autre, un atome d'hydrogène ou un groupe de formule 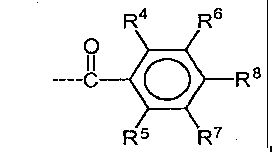 ou bien répondent à l'une des définitions indiquées pour le symbole R³ ;
où
R⁴ et R⁵ représentent chacun, indépendamment l'un de l'autre, un atome d'hydrogène ou d'halogène, ou un groupe alkyle en C₁₋₆ ou -O-alkyle en C₁₋₆ dont la chaîne, linéaire ou ramifiée, peut être interrompue par un ou plusieurs atome(s) d'oxygène ;
et R⁶, R⁷ et R⁸ représentent chacun, indépendamment les uns des autres, un atome d'hydrogène ou d'halogène, ou un groupe alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène ou de soufre ou groupe(s) de formule -NR'-, et peut porter comme substituant(s) un ou plusieurs groupe(s) polymérisable(s) et/ou groupe(s) symbolisé(s) par R⁹, le symbole R' désignant un atome d'hydrogène ou d'halogène, ou un groupe alkyle en C₁₋₂₀, alcényle en C₂₋₂₀, alcoxy en C₁₋₂₀ ou alcényloxy en C₂₋₂₀ à chaîne ramifiée, cyclique ou, de préférence, linéaire ;
- R³ représente un groupe alkyle en C₁₋₁₈ ou alcényle en C₂₋₁₈ à chaîne ramifiée ou, de préférence, linéaire, ces groupes pouvant être dépourvus de substituant ou porter un ou plusieurs substituant(s) choisi(s) parmi les atomes d'halogène, les groupes cyano, -CO-CH=CH-CO-(alkyle en C₁₋₆), -CO-CH=CH-COO-(alkyle en C₁₋₁₈), -CH=CH-phényle, -C(alkyle en C₁-₄)=C(alkyle en C₁₋₄)-phényle, phényl-(alkyle en C₁₋₄), -CO-CH=CH-CO-phényle, phényle, naphtyle, biphényle, benzophénonyle, thisanthonyle, cycloalkyle en C₅₋₁₂ et -PO(O-alkyle en C₁₋₈)₂, les groupes symbolisés par -OR¹⁰, -SR¹⁰, -OCO-R¹⁰, -COO-R¹⁰, -CH=CH-CO-OR¹⁰, -C(alkyle en C₁₋₄)=C(alkyle en C₁₋₄)-CO-OR¹⁰, -CO-R¹³, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹² et -SiR¹⁴R¹⁵R¹⁶, les groupes hétérocycliques de 5 ou 6 chaînons, saturés ou insaturés, à hétéroatome d'oxygène, de soufre ou d'azote, et les groupes de formules 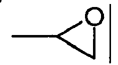 et 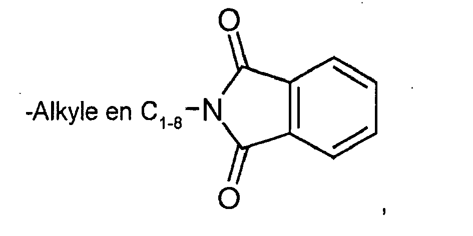 où
R¹³ représente un groupe alkyle en C₁₋₁₈, alcényle en C₂₋₁₈ dont la chaîne est interrompue par un ou plusieurs atome(s) d'oxygène, cycloalkyle en C₃₋₁₂, phényl-(alkyle en C₁₋₄), phényle, naphtyle ou biphényle, chacun des systèmes cycliques indiqués pouvant être dépourvu de substituant ou porter comme substituant(s) 1 à 5 groupe(s) alkyle en C₁₋₈, groupe(s) alcoxy en C₁₋₈, groupe(s) alkylsulfanyle en C₁₋₈ et/ou atome(s) d'halogène ;
et R¹⁴, R¹⁵ et R¹⁶ représentent chacun, indépendamment les uns des autres, un atome d'hydrogène, un groupe alkyle en C₁₋₈, alcényle en C₂₋₈, alkylphényle en C₇₋₉, -O-alkyle en C₁₋₈ ou phényle, ou un groupe symbolisé par -O-SiR¹⁷R¹⁸R¹⁹,
où
R¹⁷, R¹⁸ et R¹⁹ représentent chacun, indépendamment les uns des autres, un atome d'hydrogène ou un groupe alkyle en C₁₋₈, alcényle en C₂₋₈, alkylphényle en C₇₋₉, -O-alkyle en C₁₋₈ ou phényle,
les symboles R¹⁰, R¹¹ et R¹² répondant aux définitions déjà indiquées ;
ou bien
- R³ représente un groupe alkyle en C₂₋₁₈ à chaîne ramifiée ou, de préférence, linéaire, ou un groupe alcanediyle en C₂₋₁₈, dont la chaîne est interrompue par un ou plusieurs atome(s) d'oxygène ou de soufre ou groupe(s) de formule -NH-ou -NR¹¹-, ces groupes pouvant être dépourvus de substituant ou porter un ou plusieurs substituant(s) choisi(s) parmi les atomes d'halogène, les groupes cyano, phényl-(alkyle en C₁₋₄), phényle, cycloalkyle en C₅₋₁₂ et -PO(O-alkyle en C₁₋₈)₂, et les groupes symbolisés par -OR¹⁰, -SR¹⁰, -OCO-R¹⁰, -COO-R¹⁰, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹² et -SiR¹⁴R¹⁵R¹⁶;
les symboles R¹⁰, R¹¹, R¹², R¹⁴, R¹⁵ et R¹⁶ répondant aux définitions déjà indiquées ;
ou bien
- R³ représente un groupe alkyle en C₂₋₁₈ à chaîne ramifiée ou, de préférence, linéaire, ou un groupe alcanediyle en C₂₋₁₈, dont la chaîne est interrompue par un ou plusieurs groupe(s) phényl-(alcanediyle en C₁₋₄), phénylène, naphtylène, biphénylène ou cycloalcanediyle en C₅₋₁₂, groupe(s) de formule -CO-, -COO-, -OCO-, -OCOO-, -COO-(alcanediyle en C₁₋₆), -COS-(alcanediyle en C₁₋₁₈), -SO₂-, -SO₂-O- ou -(CH₃)₂Si[OSi(CH₃)₂]_{q}- où l'indice q vaut de 1 à 6, groupe(s) symbolisé(s) par -CO-N(R¹²)-, -N(R¹²)-CO-, -N(R¹²)-CO-N(R¹²)-, -N(R¹²)-COO-ou -SO₂-N(R¹²)-, ou groupe(s) hétérocyclique(s) de 5 ou 6 chaînons à hétéroatome d'oxygène, de soufre ou d'azote ;
le symbole R¹² répondant à la définition déjà indiquée ;
ou bien
- R³ représente un groupe de formule -COOH, Hal-(CH₃)₂Si-[OSi(CH₃)₂]ᵣ- ou (CH₃)₃Si-[OSi(CH₃)₂]ᵣ- où l'indice r vaut de 1 à 6, un groupe symbolisé par -COO-R¹⁰ ou -CO-NR¹¹R¹², ou un groupe triméthylsilyle, -CO-vinyle ou -CO-phényle, le groupe phényle pouvant être dépourvu de substituant ou porter un substituant méthyle, méthoxy et/ou atome de chlore ;
les symboles R¹⁰, R¹¹ et R¹² répondant aux définitions déjà indiquées ;
ou bien
- R³ représente un groupe phényle-(alkyle en C₁₋₂₀), phényle, naphtyle, biphényle ou cycloalkyle en C₅₋₁₂ ou un groupe hétérocyclique de 5 ou 6 chaînons, saturé ou insaturé, à hétéroatome d'oxygène, de soufre ou d'azote, ces systèmes cycliques pouvant être dépourvus de substituant ou porter comme substituant(s) 1 à 5 atome(s) d'halogène, groupe(s) alkyle en C₁₋₈, groupe(s) alcoxy en C₁₋₈, groupe(s) alkylsulfanyle en C₁₋₈ et/ou groupe(s) de formule -NR¹¹R¹² ;
les symboles R¹¹ et R¹² répondant aux définitions déjà indiquées ;
et
- l'indice m vaut 1, 2 ou 3,
- l'indice n vaut 0 ou 1,
- l'indice p vaut 0 ou 1 ;
ou bien, de préférence,
- R³ représente un atome d'halogène, un groupe hydroxyle, un groupe aromatique en C₆₋₃₀ pouvant porter un substituant alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène, de soufre ou d'azote, et/ou peut porter comme substituant(s) un ou plusieurs groupe(s) polymérisable(s) et/ou groupe(s) symbolisé(s) par R⁹, ou R³ représente un groupe alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène ou de soufre ou groupe(s) symbolisé(s) par -NR²⁰-, et peut porter comme substituant(s) un ou plusieurs groupe(s) polymérisable(s) et/ou groupe(s) symbolisé(s) par R⁹,
où
le symbole R⁹ représente un groupe hydroxyle, ou un groupe symbolisé par -CₓF₂ₓ₊₁ où l'indice x vaut de 1 à 20, ou par -[Si(CH₃)₂]_{y}-CH₃ où l'indice y vaut de 1 à 20,
et le symbole R²⁰ représente un atome d'hydrogène ou d'halogène ou un groupe alkyle en C₁₋₂₀, alcényle en C₂₋₂₀, alcoxy en C₁₋₂₀ ou alcényloxy en C₂₋₂₀ à chaîne, ramifiée, cyclique ou, de préférence, linéaire ;
étant entendu que les groupes symbolisés par R⁰, R¹, R² et R³ peuvent être reliés ensemble deux à deux et former un cycle de 5 à 8 chaînons, et que le ou les cycle(s) peut ou peuvent être condensé(s) avec un ou plusieurs cycle(s) aliphatique(s) ou aromatique(s), être dépourvu(s) de substituant ou porter un ou plusieurs substituant(s), et comporter d'autres hétéroatomes.

2. Composition conforme à la revendication 1, dans laquelle
- R⁰ représente un groupe alkyle en C₁₋₁₈ ou alcényle en C₂₋₁₈, ces groupes pouvant être dépourvus de substituant ou porter un ou plusieurs atome(s) d'halogène comme substituant(s), un groupe -CH=CH-phényle, -C(alkyle en C₁₋₄)=C(alkyle en C₁₋₄)-phényle, cycloalkyle en C₃₋₁₂, alcényle en C₂₋₁₈, phényl-(alkyle en C₁₋₄), phényle, naphtyle, anthryle ou biphényle, un groupe symbolisé par -OR¹⁰, -OCO-R¹⁰, -OCO-Hal, -COO-R¹⁰, -CH=CH-CO-OR¹⁰, -N(R¹¹)-CO-R¹⁰, -N(R¹¹)-CO-Hal, -C(alkyle en C₁₋₄)=C(alkyle en C₁₋₄)-CO-OR¹⁰ ou -CO-NR¹¹R¹², un groupe hétérocyclique de 5 ou 6 chaînons à hétéroatome d'oxygène, de soufre ou d'azote, chacun des systèmes cycliques indiqués pouvant être dépourvu de substituant ou porter comme substituant(s) 1 à 5 atome(s) d'halogène, groupe(s) alkyle en C₁₋₈, groupe(s) alcoxy en C₁₋₈ et/ou groupe(s) alkylsulfanyle en C₁₋₈, ou un groupe de formule 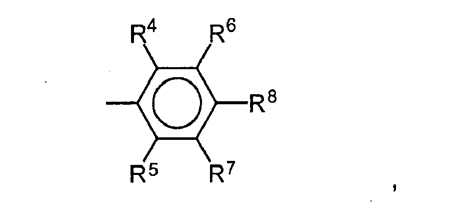 où
R¹⁰ représente un atome d'hydrogène, un groupe alkyle en C₁₋₁₈, alcényle en C₂₋₁₈, alcényle en C₂₋₁₈ dont la chaîne est interrompue par un ou plusieurs atome(s) d'oxygène, cycloalkyle en C₃₋₁₂, tétrahydropyran-2-yle, phényl-(alcanediyle en C₁₋₄), phényl-(alcènediyle en C₁₋₄), alkyle en C₁₋₆ qui peut être dépourvu de substituant ou porter un substituant halogène, cyclohexyle, cyclopentyle, tétrahydrofuranyle, furanyle ou isopropyl-4-méthylcyclohexyle, phényle, naphtyle ou biphényle, ces systèmes cycliques pouvant être dépourvus de substituant ou porter comme substituant(s) 1 à 5 atome(s) d'halogène, groupe(s) alkyle en C₁₋₈, groupe(s) alcoxy en C₁₋₈ et/ou groupe(s) alkylsulfanyle en C₁₋₈,
et R¹¹ et R¹² représentent chacun, indépendamment l'un de l'autre, un atome d'hydrogène, un groupe alkyle en C₁₋₁₈, alcényle en C₂₋₁₈, alcényle en C₂₋₁₈ dont la chaîne est interrompue par un ou plusieurs atome(s) d'oxygène, cycloalkyle en C₃₋₁₂, phényl-(alkyle en C₁₋₄), phényle, naphtyle ou pyridinyle, ces systèmes cycliques pouvant être dépourvus de substituant ou porter comme substituant(s) 1 à 5 atome(s) d'halogène, groupe(s) alkyle en C₁₋₈, groupe(s) alcoxy en C₁₋₈ et/ou groupe(s) alkylsulfanyle en C₁₋₈, ou bien R¹¹ et R¹² forment ensemble un groupe hétérocyclique de 5 ou 6 chaînons à hétéroatome d'oxygène, de soufre ou d'azote, qui peut lui-même être condensé avec un cycle aliphatique ou aromatique,
- R¹ et R² représentent chacun, indépendamment l'un de l'autre, un atome d'hydrogène ou un groupe de formule 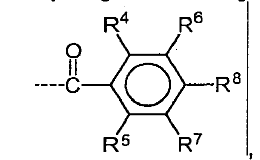 ou bien répondent à l'une des définitions indiquées pour le symbole R³ ;
où
R⁴ et R⁵ représentent chacun, indépendamment l'un de l'autre, un atome d'hydrogène ou d'halogène, ou un groupe alkyle en C₁₋₆ ou -O-alkyle en C₁₋₆ à chaîne linéaire ou ramifiée ;
et R⁶, R⁷ et R⁸ représentent chacun, indépendamment les uns des autres, un atome d'hydrogène ou d'halogène, ou un groupe alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène ou de soufre ou groupe(s) de formule -NR²⁰-, et peut porter comme substituant(s) un ou plusieurs groupe(s) polymérisable(s) et/ou groupe(s) symbolisé(s) par R⁹ ;
où
le symbole R⁹ représente un groupe hydroxyle, ou un groupe symbolisé par -CₓF₂ₓ₊₁ où l'indice x vaut de 1 à 20, ou par -[Si(CH₃)₂]_{y}-CH₃ où l'indice y vaut de 1 à 20,
et le symbole R²⁰ désigne un atome d'hydrogène ou d'halogène, ou un groupe alkyle en C₁₋₂₀, alcényle en C₂₋₂₀, alcoxy en C₁₋₂₀ ou alcényloxy en C₂₋₂₀ à chaîne ramifiée, cyclique ou, de préférence, linéaire ;
- R³ représente un groupe alkyle en C₁₋₁₈ ou alcényle en C₂₋₁₈ à chaîne ramifiée ou, de préférence, linéaire, ces groupes pouvant être dépourvus de substituant ou porter un ou plusieurs substituant(s) choisi(s) parmi les atomes d'halogène, les groupes cyano, -CO-CH=CH-CO-(alkyle en C₁₋₆), -CO-CH=CH-COO-(alkyle en C₁₋₁₈), -CH=CH-phényle, -C(alkyle en C₁₋₄)=C(alkyle en C₁₋₄)-phényle, phényl-(alkyle en C₁₋₄), -CO-CH=CH-CO-phényle, phényle, naphtyle, biphényle, benzophénonyle, thisanthonyle, cycloalkyle en C₅₋₁₂ et -PO(O-alkyle en C₁₋₈)₂, les groupes symbolisés par -OR¹⁰, -SR¹⁰, -OCO-R¹⁰, -COO-R¹⁰, -CH=CH-CO-OR¹⁰, -C(alkyle en C₁₋₄)=C(alkyle en C₁₋₄)-CO-OR¹⁰, -CO-R¹³, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹² et -SiR¹⁴R¹⁵R¹⁶, les groupes hétérocycliques de 5 ou 6 chaînons à hétéroatome d'oxygène, de soufre ou d'azote, et les groupes de formules 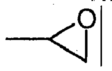 et 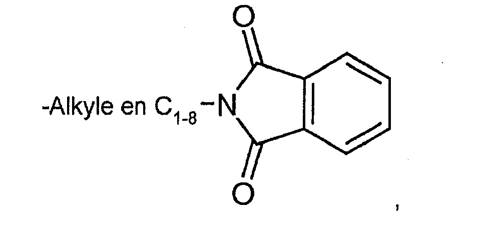 où
R¹³ représente un groupe alkyle en C₁₋₁₈, alcényle en C₂₋₁₈ dont la chaîne est interrompue par un ou plusieurs atome(s) d'oxygène, cycloalkyle en C₃₋₁₂, phényl-(alkyle en C₁₋₄), phényle, naphtyle ou biphényle, chacun des systèmes cycliques indiqués pouvant être dépourvu de substituant ou porter comme substituant(s) 1 à 5 groupe(s) alkyle en C₁₋₈, groupe(s) alcoxy en C₁₋₈, groupe(s) alkylsulfanyle en C₁₋₈ et/ou atome(s) d'halogène ;
et R¹⁴, R¹⁵ et R¹⁶ représentent chacun, indépendamment les uns des autres, un atome d'hydrogène, un groupe alkyle en C₁₋₈, alcényle en C₂₋₈, alkylphényle en C₇₋₉, -O-alkyle en C₁₋₈ ou phényle, ou un groupe symbolisé par -O-SiR¹⁷R¹⁸R¹⁹,
où
R¹⁷, R¹⁸ et R¹⁹ représentent chacun, indépendamment les uns des autres, un atome d'hydrogène ou un groupe alkyle en C₁₋₈, alcényle en C₂₋₈, alkylphényle en C₇₋₉, -O-alkyle en C₁₋₈ ou phényle,
les symboles R¹⁰, R¹¹ et R¹² répondant aux définitions déjà indiquées ;
ou bien
- R³ représente un groupe alkyle en C₂₋₁₈ à chaîne ramifiée ou, de préférence, linéaire, ou un groupe alcanediyle en C₂₋₁₈, dont la chaîne est interrompue par un ou plusieurs atome(s) d'oxygène ou de soufre ou groupe(s) de formule -NH-ou -NR¹¹-, ces groupes pouvant être dépourvus de substituant ou porter un ou plusieurs substituant(s) choisi(s) parmi les atomes d'halogène, les groupes cyano, phényl-(alkyle en C₁₋₄), phényle, cycloalkyle en C₅₋₁₂ et -PO(O-alkyle en C₁₋₈)₂, et les groupes symbolisés par -OR¹⁰, -SR¹⁰, -OCO-R¹⁰, -COO-R¹⁰, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹² et -SiR¹⁴R¹⁵R¹⁶ ;
les symboles R¹⁰, R¹¹ et R¹² répondant aux définitions déjà indiquées ;
ou bien
- R³ représente un groupe alkyle en C₂₋₁₈ à chaîne ramifiée ou, de préférence, linéaire, ou un groupe alcanediyle en C₂₋₁₈, dont la chaîne est interrompue par un ou plusieurs groupe(s) phényl-(alcanediyle en C₁₋₄), phénylène, naphtylène, biphénylène ou cycloalcanediyle en C₅₋₁₂, groupe(s) de formule -CO-, -COO-, -OCO-, -OCOO-, -COO-(alcanediyle en C₁₋₆), -COS-(alcanediyle en C₁₋₁₈), -SO₂-, -SO₂-O- ou -(CH₃)₂Si[OSi(CH₃)₂]_{q}- où l'indice q vaut de 1 à 6, groupe(s) symbolisé(s) par -CO-N(R¹¹)-, -N(R¹¹)-CO-, -N(R¹¹)-CO-N(R¹¹)-, -N(R¹¹)-COO-ou -SO₂-N(R¹¹)-, ou groupe(s) hétérocyclique(s) de 5 ou 6 chaînons à hétéroatome d'oxygène, de soufre ou d'azote ;
le symbole R¹¹ répondant à la définition déjà indiquée ;
ou bien
- R³ représente un groupe de formule -COOH, Hal-(CH₃)₂Si-[OSi(CH₃)₂]ᵣ- ou (CH₃)₃Si-[OSi(CH₃)₂]ᵣ- où l'indice r vaut de 1 à 6, un groupe symbolisé par -COO-R¹⁰ ou -CO-NR¹¹R¹², ou un groupe triméthylsilyle, -CO-vinyle ou -CO-phényle, le groupe phényle pouvant être dépourvu de substituant ou porter un substituant méthyle, méthoxy et/ou atome de chlore ;
les symboles R¹⁰, R¹¹ et R¹² répondant aux définitions déjà indiquées ;
ou bien
- R³ représente un groupe phényle-(alkyle en C₁₋₄), phényle, naphtyle, biphényle ou cycloalkyle en C₅₋₁₂, ou un groupe hétérocyclique de 5 ou 6 chaînons à hétéroatome d'oxygène, de soufre ou d'azote, ces systèmes cycliques pouvant être dépourvus de substituant ou porter comme substituant(s) 1 à 5 atome(s) d'halogène, groupe(s) alkyle en C₁₋₈, groupe(s) alcoxy en C₁₋₈, groupe(s) alkylsulfanyle en C₁₋₈ et/ou groupe(s) de formule -NR¹¹R¹² ;
les symboles R¹¹ et R¹² répondant aux définitions déjà indiquées ; et
- l'indice m vaut 1, 2 ou 3,
- l'indice n vaut 0 ou 1,
- l'indice p vaut 0 ou 1 ;
ou bien
- R³ représente un atome d'halogène, un groupe hydroxyle, un groupe aromatique en C₆₋₃₀ pouvant porter un substituant alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène ou de soufre ou groupe(s) symbolisé(s) par -NR²⁰-, et/ou peut porter comme substituant(s) un ou plusieurs groupe(s) polymérisable(s) et/ou groupe(s) symbolisé(s) par R⁹, ou R³ représente un groupe alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence; linéaire, peut être interrompue par un ou plusieurs atome(s) d'oxygène ou de soufre ou groupe(s) symbolisés par -NR²⁰-, et peut porter comme substituant(s) un ou plusieurs groupe(s) polymérisable(s) et/ou groupe(s) symbolisé(s) par R⁹, les symboles R⁹ et R²⁰ répondant aux définitions déjà indiquées.

3. Composition comprenant au moins un liant polymérisable et un amorceur de polymérisation, qui contient au moins un composé de type acyl-germanium répondant à la formule générale (II) : 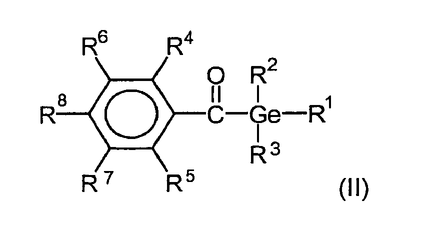 dans laquelle
- R¹ et R² représentent chacun, indépendamment l'un de l'autre, un atome d'hydrogène ou un groupe de formule 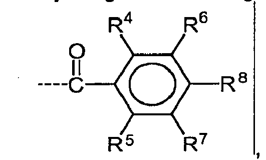
ou bien répondent à l'une des définitions indiquées pour le symbole R³ ;
- R³ représente un atome d'halogène, un groupe hydroxyle, un groupe aromatique en C₆₋₃₀ pouvant porter un substituant alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène ou de soufre ou groupe(s) symbolisé(s) par -NR²⁰-, et/ou peut porter comme substituant(s) un ou plusieurs groupe(s) polymérisable(s) et/ou groupe(s) symbolisé(s) par R⁹, ou R³ représente un groupe alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène ou de soufre ou groupe(s) symbolisés par -NR²⁰-, et peut porter comme substituant(s) un ou plusieurs groupe(s) polymérisable(s) et/ou groupe(s) symbolisé(s) par R⁹ ;
- R⁴ et R⁵ représentent chacun, indépendamment l'un de l'autre, un atome d'hydrogène ou d'halogène, ou un groupe alkyle en C₁₋₆ ou -O-alkyle en C₁₋₆ à chaîne linéaire ou ramifiée ;
- R⁶, R⁷ et R⁸ représentent chacun, indépendamment les uns des autres, un atome d'hydrogène ou d'halogène, ou un groupe alkyle en C₁₋₂₀, alcényle en C₂₋₂₀, alcoxy en C₁₋₂₀ ou alcényloxy en C₂₋₂₀ dont la chaîne, ramifiée, cyclique ou, de préférence, linéaire, peut être interrompue par un ou plusieurs atome(s) d'oxygène ou de soufre ou groupe(s) de formule -NR²⁰-, et peut porter comme substituant(s) un ou plusieurs groupe(s) polymérisable(s) et/ou groupe(s) symbolisé(s) par R⁹ ;
- R⁹ représente un groupe hydroxyle, ou un groupe symbolisé par -CₓF₂ₓ₊₁ où l'indice x vaut de 1 à 20, ou par -[Si(CH₃)₂]_{y}-CH₃ où l'indice y vaut de 1 à 20 ;
- et R²⁰ désigne un atome d'hydrogène ou d'halogène, ou un groupe alkyle en C₁₋₂₀, alcényle en C₂₋₂₀, alcoxy en C₁₋₂₀ ou alcényloxy en C₂₋₂₀ à chaîne ramifiée, cyclique ou, de préférence, linéaire.

4. Composition comprenant au moins un liant polymérisable et un amorceur de polymérisation, qui contient au moins un composé de type acyl-germanium répondant à la formule générale (III) : 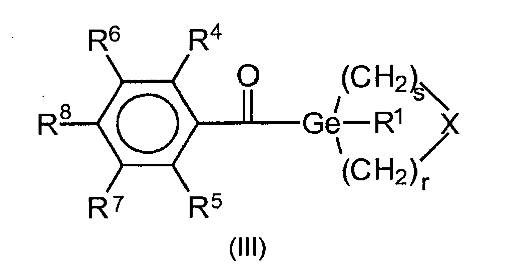 dans laquelle
- R¹ représente un atome d'hydrogène ou un groupe de formule 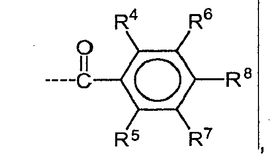 ou bien répond à l'une des définitions indiquées pour le symbole R³ ;
- les indices r et s désignent, indépendamment l'un de l'autre, un nombre entier de 0 à 6, et ont une valeur telle que le nombre total des atomes du cycle, incluant l'atome de germanium, soit de 5 à 8 ;
- et le symbole X est absent ou représente un atome d'oxygène ou de soufre, ou un groupe symbolisé par N-R²¹, le symbole R²¹ désignant un atome d'hydrogène ou un groupe alkyle en C₁₋₁₀,
étant entendu que le cycle comportant l'atome de germanium peut être condensé avec un ou plusieurs cycle(s) aliphatique(s) ou aromatique(s), et peut être dépourvu de substituant ou porter un ou plusieurs substituant(s), auquel cas le nombre des atomes d'hydrogène du cycle est réduit en conséquence.

5. Composition conforme à l'une des revendications précédentes, pour laquelle les groupes polymérisables sont choisis parmi les groupes vinyle, styryle, (méth)acrylate, (méth)acrylamide et N-alkylacrylamide.

6. Composition conforme à l'une des revendications précédentes, pour laquelle les groupes symbolisés par R², R³, R⁶, R⁷ et R⁸ portent chacun 1 à 3 groupe(s) polymérisable(s) comme substituant(s).

7. Composition conforme à l'une des revendications précédentes, dans laquelle la proportion du dérivé d'acyl-germanium de formule (I), rapportée au poids total de la composition, représente 0,001 à 5 % en poids.

8. Composition conforme à l'une des revendications précédentes, dans laquelle le liant polymérisable est constitué par au moins un monomère et/ou un prépolymère polymérisable(s) par voie radicalaire.

9. Composition conforme à la revendication 8, dans laquelle le liant est un mono-(méth)acrylate ou un poly-(méth)acrylate ou un mélange de tels composés.

10. Composition conforme à la revendication 8 ou 9, qui contient au moins un monomère polymérisable par voie radicalaire et ouverture de cycle.

11. Composition conforme à l'une des revendications précédentes, dans laquelle le liant est constitué par un mélange de composés de type(s) mono-mercapto et/ou polymercapto, et de monomères di-insaturés et/ou poly-insaturés.

12. Composition conforme à l'une des revendications précédentes, qui contient au moins un amorceur supplémentaire pour la polymérisation radicalaire.

13. Composition conforme à l'une des revendications précédentes, qui contient au moins un amorceur supplémentaire pour la polymérisation cationique.

14. Composition conforme à l'une des revendications précédentes, qui contient en outre une charge.

15. Composition conforme à l'une des revendications précédentes, qui contient en outre au moins un additif choisi parmi les agents stabilisants, agents absorbant le rayonnement UV, lubrifiants, agents mouillants, dispersants, promoteurs d'adhésion, agents de matage et agents brillanteurs, adjuvants d'écoulement et adjuvants filmogènes, agents anti-peau, agents photo-protecteurs, agents anticorrosion, agents ignifuges, antioxydants, agents de blanchiment optique, plastifiants, épaississants et agents anti-mousse.

16. Composition conforme à la revendication 1, qui contient :
- 0,001 à 5 % en poids de composé de type acyl-germanium répondant à la formule (I),
- 5 à 99,9 % en poids de liant polymérisable,
- 0 à 90 % en poids de charge,
chacune de ces proportions étant rapportée au poids total de la composition.

17. Composition conforme à la revendication 16, qui contient 0 à 50 % en poids d'additif supplémentaire.

18. Système de préparation de corps moulés, qui comprend une composition conforme à l'une des revendications 1 à 17 et une source lumineuse à diode électroluminescente.

19. Système conforme à la revendication 18, pour lequel la source lumineuse à diode électroluminescente possède une longueur d'onde valant de 400 à 550 nm et le composé de type acyl-germanium présente une longueur d'onde d'activation valant de 400 à 550 nm.

20. Emploi d'un dérivé d'acyl-germanium répondant à la formule (I) comme amorceur pour la polymérisation radicalaire.

21. Emploi d'un dérivé d'acyl-germanium répondant à la formule (I) pour empêcher la formation d'une couche d'inhibition lors de la polymérisation radicalaire.

22. Emploi d'un dérivé d'acyl-germanium répondant à la formule (I) pour préparer des adhésifs, des revêtements, des ciments, des produits composites, des pièces moulées ou des substances dentaires.

23. Procédé de fabrication d'un corps moulé, pour lequel on moule une composition conforme à l'une des revendications 1 à 16 en un corps présentant la forme voulue, et l'on fait ensuite durcir partiellement ou complètement le corps ainsi obtenu.

24. Procédé conforme à la revendication 23 pour la fabrication de couronnes dentaires, de bridges, d'inlays ou de dents artificielles.
